# EUROPEAN PATENT APPLICATION

(11) **EP 4 368 619 A1**
(43) Date of publication of application: **15.05.2024**
(21) Application number: 22837954.1
(22) Date of filing: 06.07.2022
(51) Int. Cl.: C07D 403/04, C07D 209/82, H10K 99/00, H10K 50/00

(54) **COMPOUND FOR ORGANIC OPTOELECTRONIC DEVICE, ORGANIC OPTOELECTRONIC DEVICE, AND DISPLAY DEVICE**

(30) Priority: 06.07.2021 KR 20210088619; 05.07.2022 KR 20220082645
(71) Applicant: Samsung SDI Co., Ltd., Yongin-si, Gyeonggi-do 17084 (KR)
(72) Inventor: LIM, Youngmook, Suwon-si, Gyeonggi-do 16678 (KR); KIM, Jonghoon, Suwon-si, Gyeonggi-do 16678 (KR); KIM, Hyung Sun, Suwon-si, Gyeonggi-do 16678 (KR); LEE, Mijin, Suwon-si, Gyeonggi-do 16678 (KR); JANG, Jinseok, Suwon-si, Gyeonggi-do 16678 (KR); JUNG, Ho Kuk, Suwon-si, Gyeonggi-do 16678 (KR); JO, Youngkyoung, Suwon-si, Gyeonggi-do 16678 (KR)
(74) Representative: Michalski Hüttermann & Partner Patentanwälte mbB
(86) International application number: PCT/KR2022/009714
(87) International publication number: WO 2023/282602

(57) **Abstract**

Disclosed are a compound for an organic optoelectronic device represented by Chemical Formula 1, an organic optoelectronic device including the same, and a display device.

Details for Chemical Formula 1 are the same as described in the specification.

## Description

### [Technical Field]

A compound for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is a light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

### [Disclosure]

### [Technical Problem]

An embodiment provides a compound for an organic optoelectronic device capable of realizing an organic optoelectronic device having low driving, high efficiency, and long life-span.

An embodiment provides an organic optoelectronic device including the compound.

Another embodiment provides a display device including the organic optoelectronic device.

### [Technical Solution]

According to an embodiment, a compound for an organic optoelectronic device represented by Chemical Formula 1 is provided.

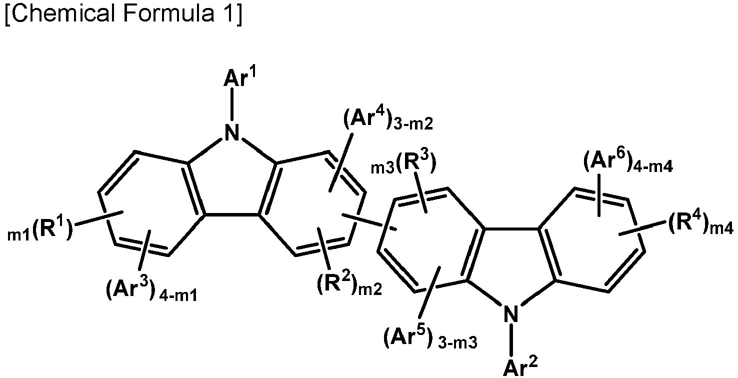

In Chemical Formula 1,
R¹ to R⁴ are each independently hydrogen, deuterium, or a substituted or unsubstituted C6 to C30 aryl group,
Ar³ to Ar⁶ are each independently hydrogen, or a substituted or unsubstituted C6 to C30 aryl group,
m1 and m4 are each independently one of integers of 1 to 4,
m2 and m3 are each independently one of integers of 1 to 3,
at least one of R¹ to R⁴ is deuterium,
Ar¹ and Ar² are each independently one selected from the substituents listed in Group I and Group II, and
at least one of Ar¹ and Ar² is one selected from the substituents listed in Group II ; 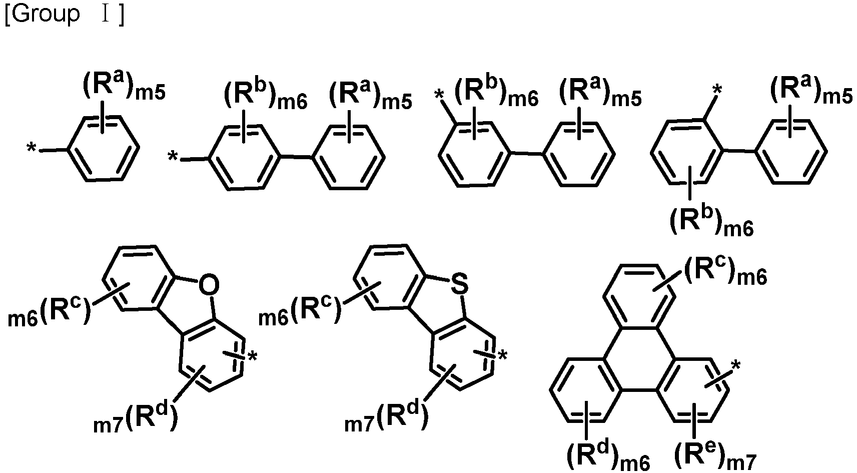 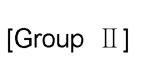 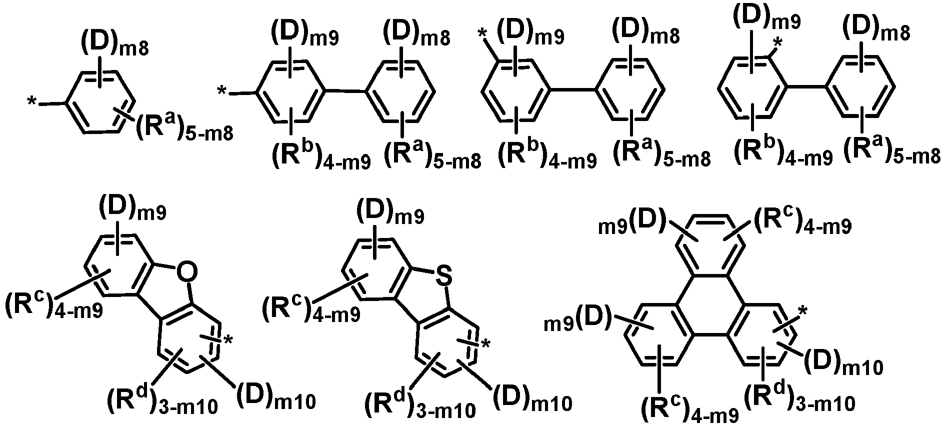
wherein, in Group I and Group II,
R^{a} and R^{b} are each independently hydrogen, deuterium, a cyano group, or a substituted or unsubstituted C1 to C10 alkyl group,
R^{c} to R^{e} are each independently hydrogen, deuterium, a cyano group, a substituted or unsubstituted C1 to C10 alkyl group, or a substituted or unsubstituted C6 to C20 aryl group,
D is deuterium,
m5 and m8 are each independently one of integers of 1 to 5,
m6 and m9 are each independently one of integers of 1 to 4, and
m7 and m10 are each independently one of integers of 1 to 3.

According to another embodiment, an organic optoelectronic device includes an anode and a cathode facing each other, and at least one organic layer between the anode and the cathode, wherein the organic layer includes the compound for an organic optoelectronic device.

According to another embodiment, a display device including the organic optoelectronic device is provided.

### [Advantageous Effects]

An organic optoelectronic device having low driving, high efficiency, and a long life-span may be realized.

### [Description of the Drawings]

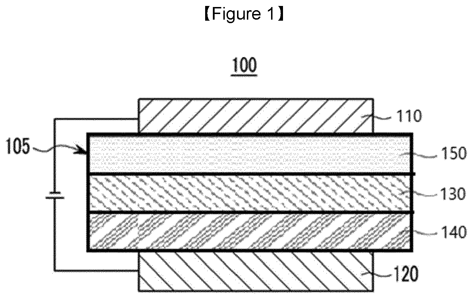

FIG. 1 is a cross-sectional view illustrating an organic light emitting diode according to an embodiment.

### <Description of Symbols>

100: organic light emitting diode
105: organic layer
110: cathode
120: anode
130: light emitting layer
140: hole transport region
150: electron transport region

### [Mode for Invention]

Hereinafter, embodiments of the present invention are described in detail. However, these embodiments are exemplary, the present invention is not limited thereto and the present invention is defined by the scope of claims.

In the present specification, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In specific example of the present invention, the "substituted" refers to replacement of at least one hydrogen of a substituent or a compound by deuterium, a C1 to C20 alkyl group, a C6 to C30 aryl group, or a cyano group. In specific example of the present invention, the "substituted" refers to replacement of at least one hydrogen of a substituent or a compound by deuterium, a C1 to C5 alkyl group, a C6 to C18 aryl group, or a cyano group. In specific example of the present invention, the "substituted" refers to replacement of at least one hydrogen of a substituent or a compound by deuterium, a cyano group, a methyl group, an ethyl group, a propyl group, a butyl group, a phenyl group, a biphenyl group, a terphenyl group, or a naphthyl group.

In this specification, "unsubstituted" means that a hydrogen atom remains as a hydrogen atom without being substituted with another substituent.

In this specification, "hydrogen substitution (-H)" may include "deuterium substitution (-D) or tritium substitution (-T)."

In the present specification, when a definition is not otherwise provided, "hetero" refers to one including one to three heteroatoms selected from N, O, S, P, and Si, and remaining carbons in one functional group.

In the present specification, "an aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quarterphenyl group, and the like, and two or more hydrocarbon aromatic moieties are fused directly or indirectly to provide a nonaromatic fused ring, for example a fluorenyl group.

The aryl group may include a monocyclic, polycyclic, or fused ring polycyclic (i.e., rings sharing adjacent pairs of carbon atoms) functional group.

In the present specification, "a heterocyclic group" is a generic concept of a heteroaryl group, and may include at least one heteroatom selected from N, O, S, P, and Si instead of carbon (C) in a cyclic compound such as an aryl group, a cycloalkyl group, a fused ring thereof, or a combination thereof. When the heterocyclic group is a fused ring, the entire ring or each ring of the heterocyclic group may include one or more heteroatoms.

For example, "a heteroaryl group" refers to an aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylene group, a substituted or unsubstituted perylenyl group, a substituted or unsubstituted fluorenyl group, a substituted or unsubstituted indenyl group, a substituted or unsubstituted furanyl group, or a combination thereof, but is not limited thereto.

More specifically, the substituted or unsubstituted C2 to C30 heterocyclic group may be a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an embodiment is described.

The compound for an organic optoelectronic device according to an embodiment is represented by Chemical Formula 1.

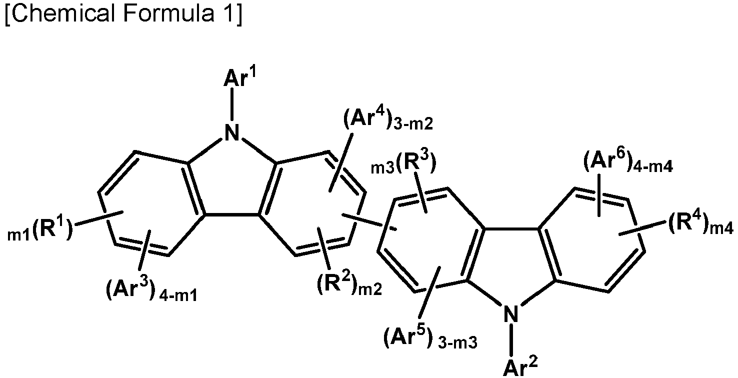

In Chemical Formula 1,
R¹ to R⁴ are each independently hydrogen, deuterium, or a substituted or unsubstituted C6 to C30 aryl group,
Ar³ to Ar⁶ are each independently hydrogen, or a substituted or unsubstituted C6 to C30 aryl group,
m1 and m4 are each independently one of integers of 1 to 4,
m2 and m3 are each independently one of integers of 1 to 3,
at least one of R¹ to R⁴ is deuterium,
Ar¹ and Ar² are each independently one selected from the substituents listed in Group I and Group II, and
at least one of Ar¹ and Ar² is one selected from the substituents listed in Group II ; 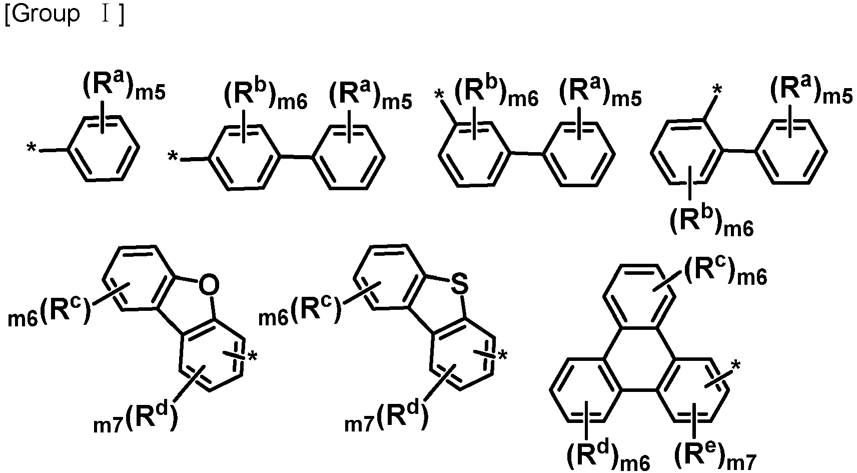 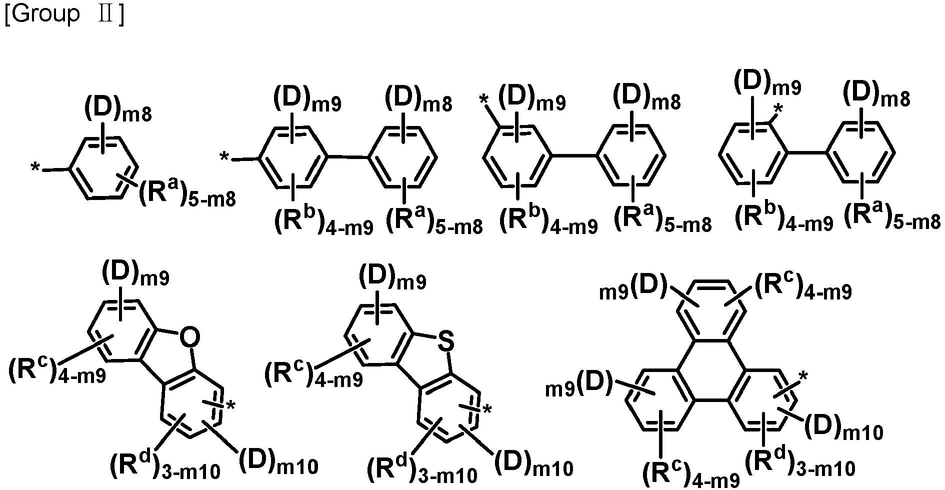
wherein, in Group I and Group II,
R^{a} and R^{b} are each independently hydrogen, deuterium, a cyano group, or a substituted or unsubstituted C1 to C10 alkyl group,
R^{c} to R^{e} are each independently hydrogen, deuterium, a cyano group, a substituted or unsubstituted C1 to C10 alkyl group, or a substituted or unsubstituted C6 to C20 aryl group,
D is deuterium,
m5 and m8 are each independently one of integers of 1 to 5,
m6 and m9 are each independently one of integers of 1 to 4, and
m7 and m10 are each independently one of integers of 1 to 3.

The compound represented by Chemical Formula 1 has biscarbazole as a basic skeleton, a benzene moiety constituting carbazole is substituted with at least one deuterium, and at least one of Ar¹ and Ar², which are the 9th (N-direction) substituents of carbazole, is substituted with deuterium.

As the benzene moiety constituting carbazole and the 9th (N-direction) substituent of carbazole are substituted with deuterium at the same time, a zero-point energy and vibrational energy of the compound may be further lowered. As a result, a ground state energy is further lowered, and an intermolecular interaction is weakened, so that the thin film formed therefrom may be made in an amorphous state, so that heat resistance is further improved and life-span is improved. That is, when this is applied, an organic light emitting diode with low driving, high efficiency, and particularly long life-span may be implemented.

Chemical Formula 1 may be represented by any one of Chemical Formula 1-1 to Chemical Formula 1-10 according to the linking position of carbazole.

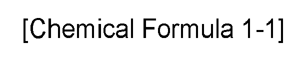

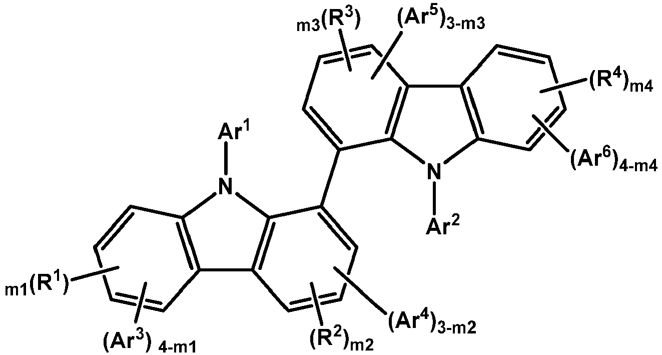

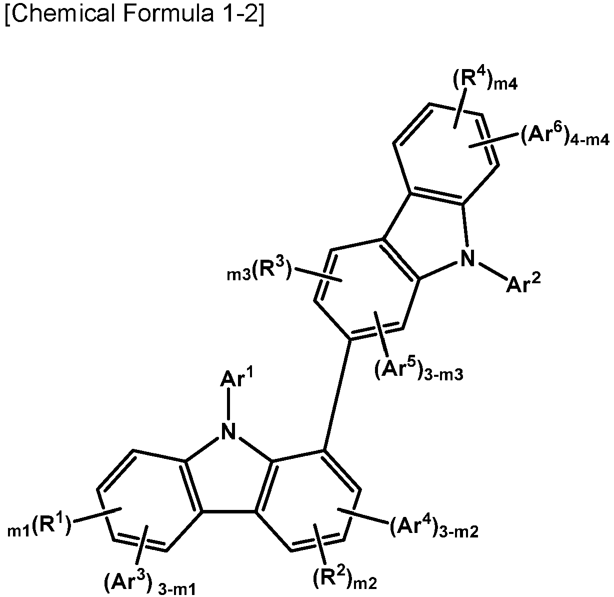

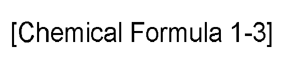

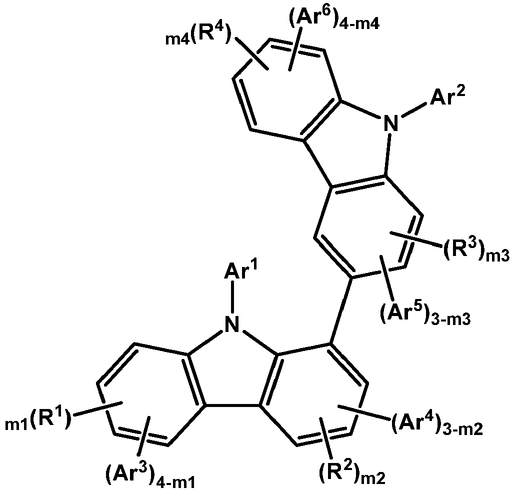

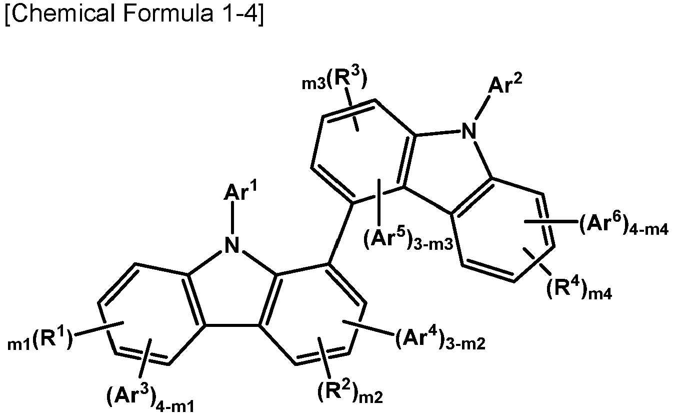

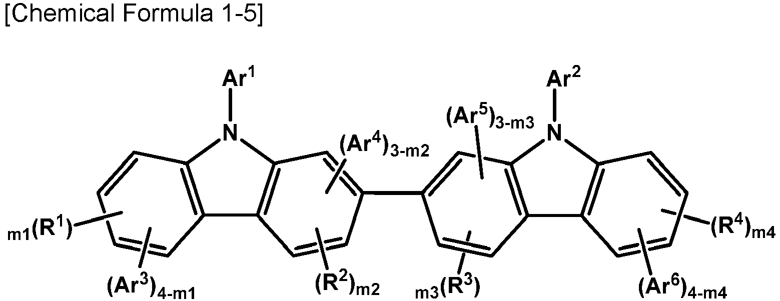

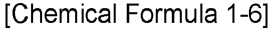

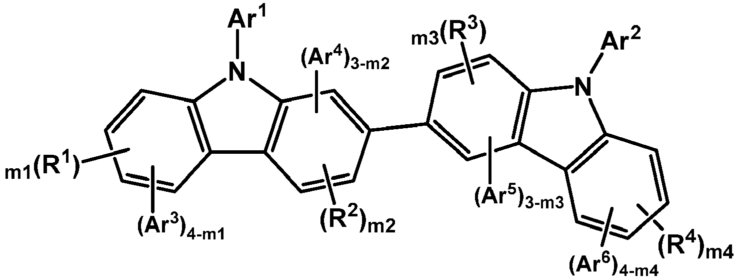

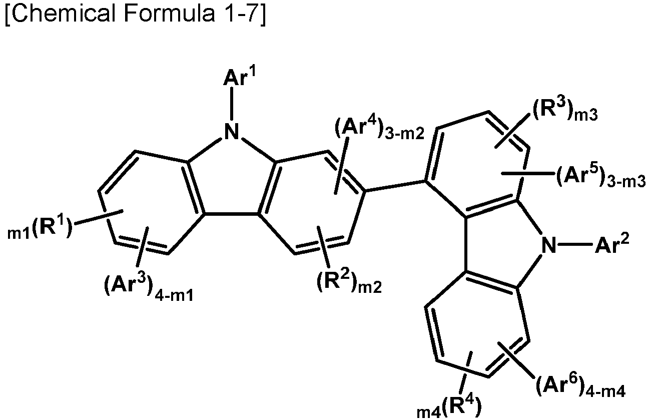

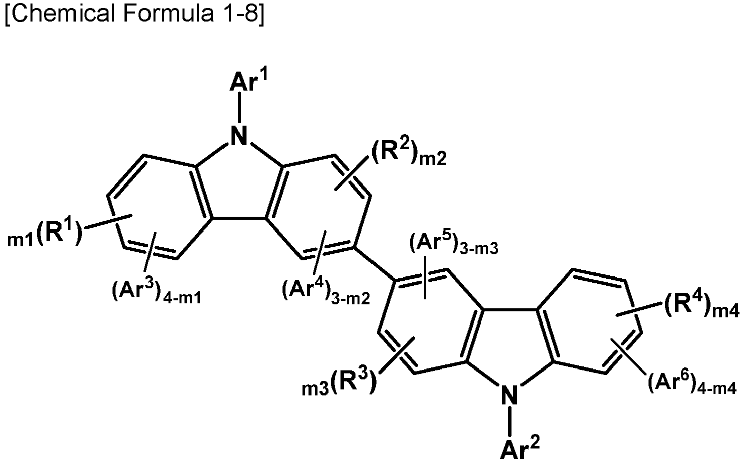

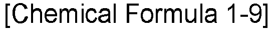

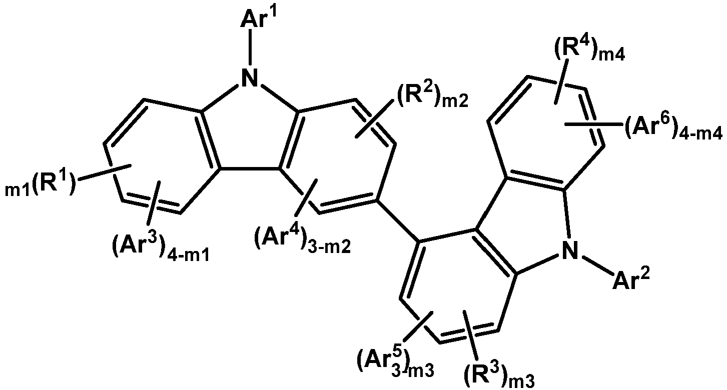

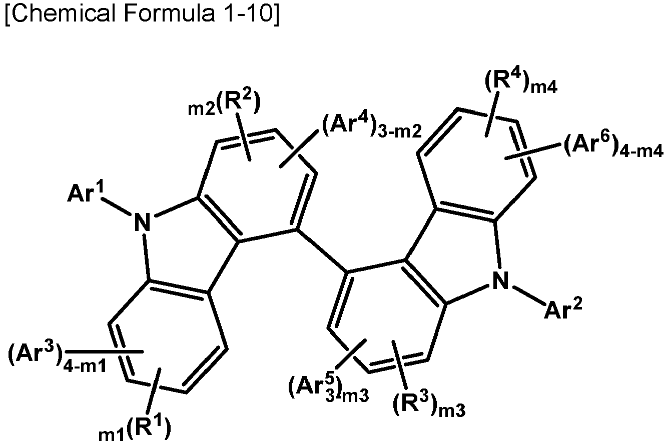

In Chemical Formula 1-1 to Chemical Formula 1-10,
definitions of Ar¹ to Ar⁶, R¹ to R⁴, and m¹ to m⁴ are the same as described above.
If R¹ is 2 or more, each R¹ may be the same or different from each other.
If R² is 2 or more, each R² may be the same or different from each other.
If R³ is 2 or more, each R³ may be the same or different from each other.
If R⁴ is 2 or more, each R⁴ may be the same or different from each other.
If Ar³ is 2 or more, each Ar³ may be the same or different from each other.
If Ar⁴ is 2 or more, each Ar⁴ may be the same or different from each other.
If Ar⁵ is 2 or more, each Ar⁵ may be the same or different from each other.
If Ar⁶ is 2 or more, each Ar⁶ may be the same or different from each other.

For example, at least two of R¹ to R⁴ may be deuterium.

For example, each of R¹ to R⁴ may be deuterium, m1 and m4 may each be an integer of 4, and m2 and m3 may each be an integer of 3.

For example, each of R¹ and R² may be deuterium, m1 may be one of integers from 1 to 4, m2 may be one of integers from 1 to 3, and each of R³ and R⁴ may be hydrogen.

For example, R³ and R⁴ may each be deuterium, m3 may be one of integers from 1 to 3, m4 may be one of integers from 1 to 4, and R¹ and R² may each be hydrogen.

For example, R¹ and R⁴ may each be deuterium, m1 and m4 may each be an integer from 1 to 4, and R² and R³ may each be hydrogen.

For example, R¹ to R³ may each be deuterium, m2 and m3 may each be an integer of 1 to 3, m1 may be an integer of 1 to 4, and R⁴ may be deuterium or a C6 to C30 aryl group that is substituted or unsubstituted with deuterium.

For example, Chemical Formula 1 may be represented by any one of Chemical Formula 1a to Chemical Formula 1e according to the substitution position of deuterium substituted in R¹ to R⁴.

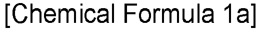

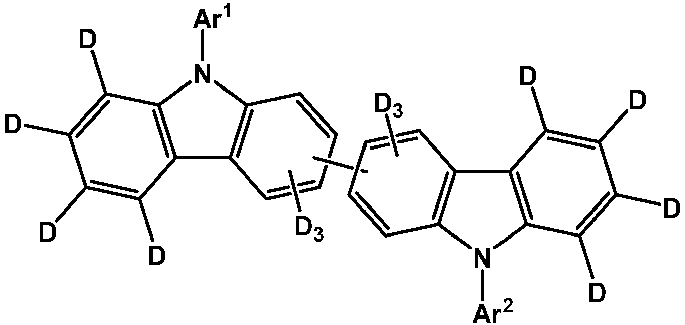

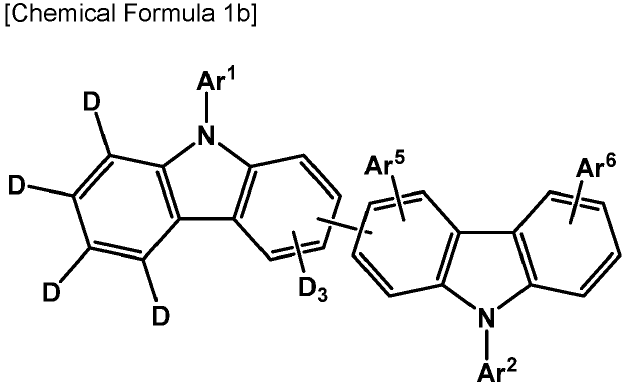

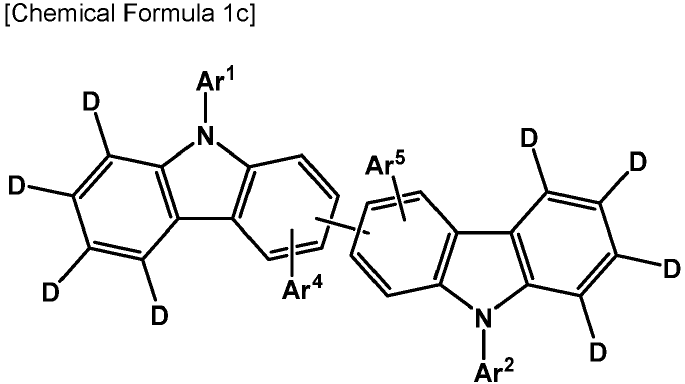

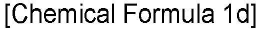

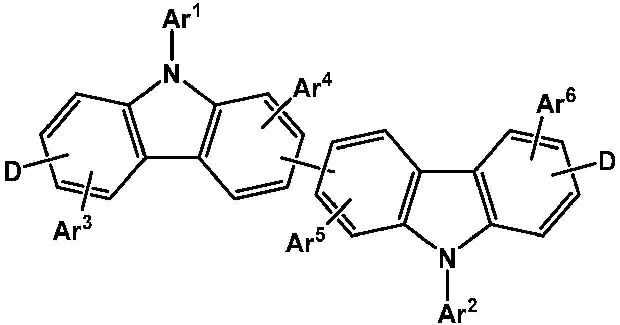

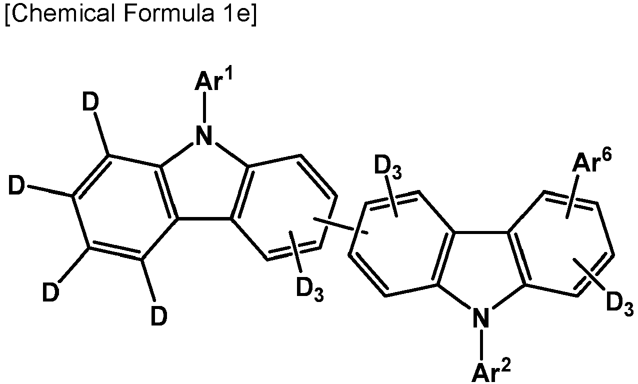

In Chemical Formula 1a to Chemical Formula 1e, definitions of Ar¹ to Ar⁶ are the same as described above.
Ar³ to Ar⁶ are each independently hydrogen or a C6 to C30 aryl group unsubstituted or substituted with deuterium, and
D₃ means substitution with three deuterium atoms.

As a specific example, Ar³ to Ar⁶ may each independently be hydrogen or a C6 to C20 aryl group unsubstituted or substituted with at least one deuterium.

For example, Ar³ to Ar⁶ may each independently be hydrogen or a phenyl group that is substituted or unsubstituted deuterium, a biphenyl group that is substituted or unsubstituted deuterium, a terphenyl group that is substituted or unsubstituted deuterium, a naphthyl group that is substituted or unsubstituted deuterium, a phenanthrenyl group that is substituted or unsubstituted deuterium, an anthracenyl group that is substituted or unsubstituted deuterium, a triphenylene group that is substituted or unsubstituted deuterium, or a fluorenyl group that is substituted or unsubstituted deuterium.

For example, Ar¹ and Ar² in Chemical Formula 1 may each independently be one selected from the substituents listed in Group I -1 and Group II -1, and
at least one of Ar¹ and Ar² may be one selected from the substituents listed in Group II -1 .

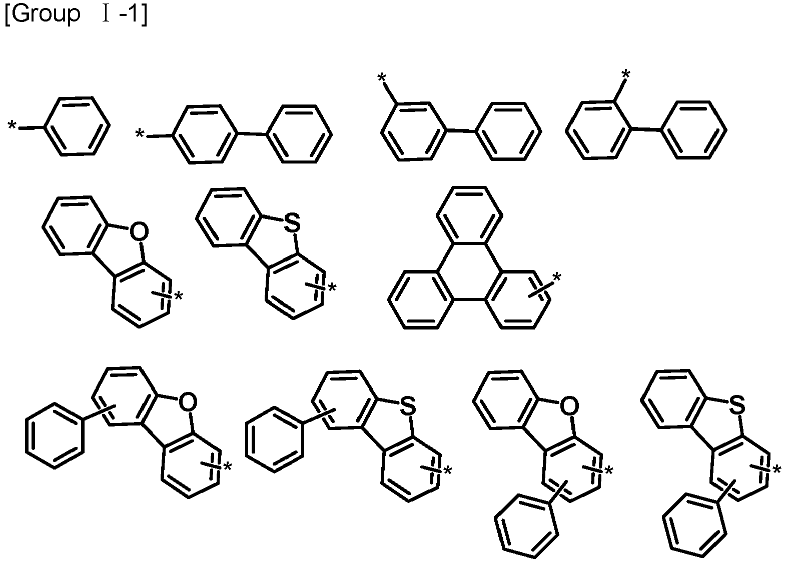

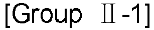

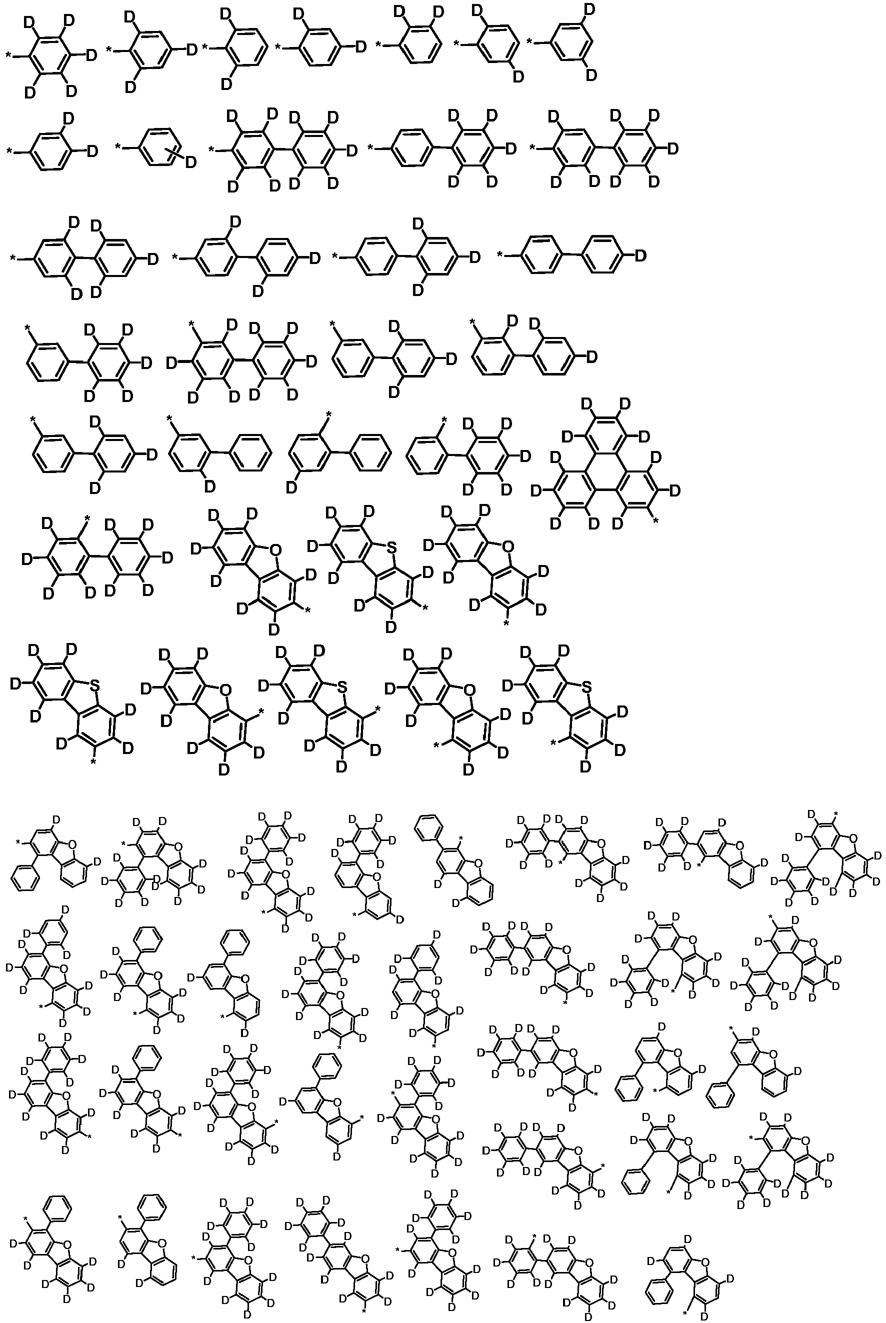

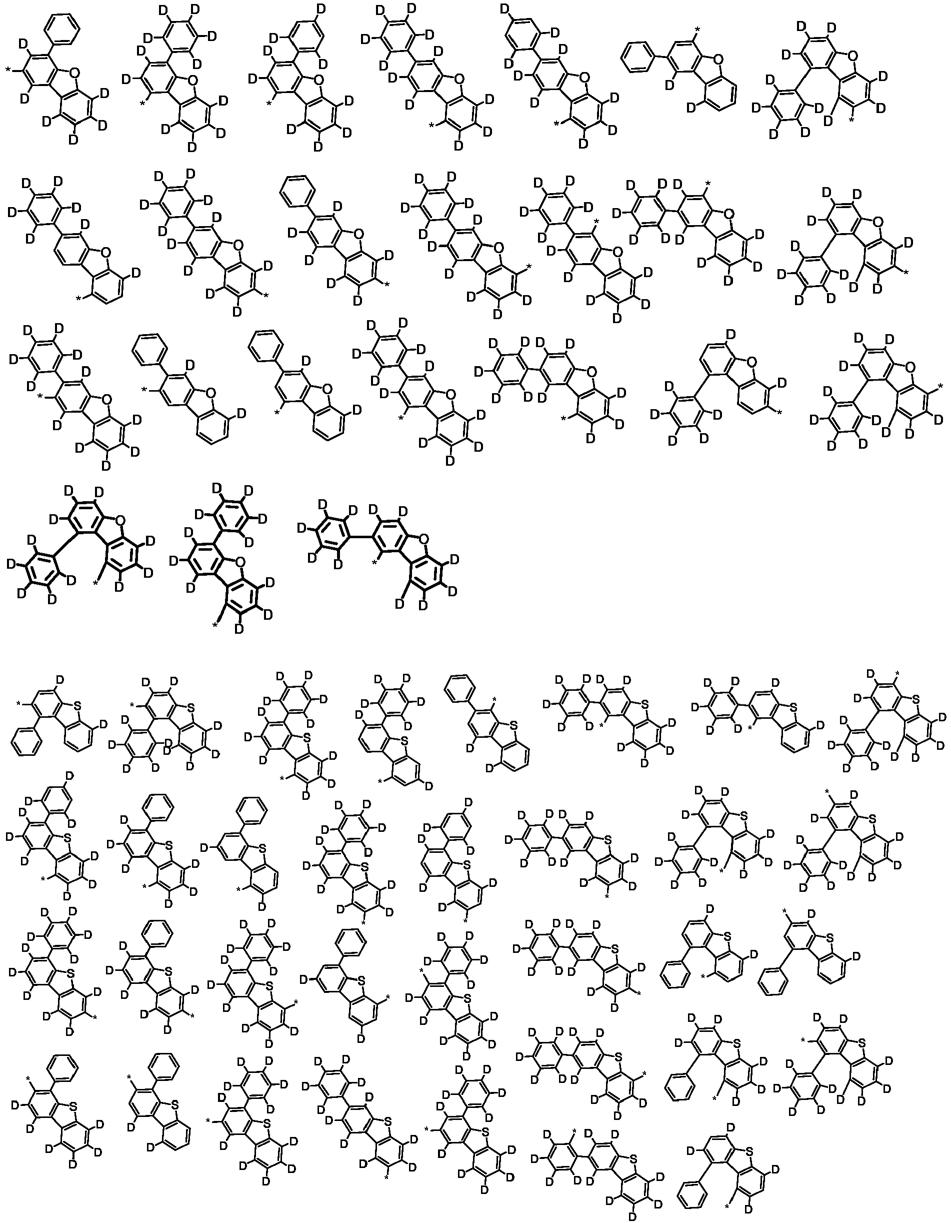

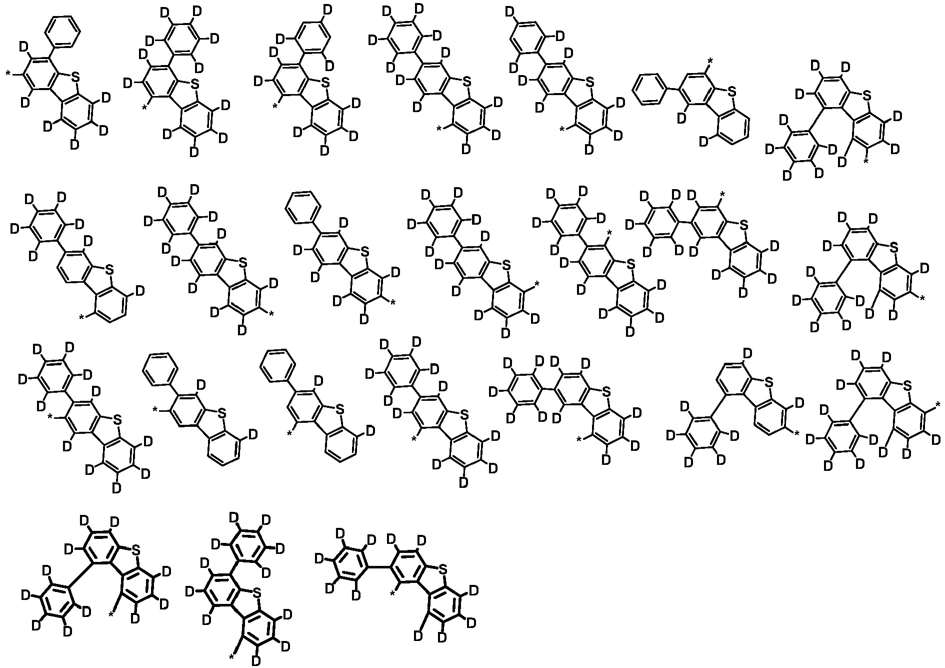

In Group I -1 and Group II -1, * is a linking point.

For example, Chemical Formula 1 may be represented by Chemical Formula 1-8a or Chemical Formula 1-8e.

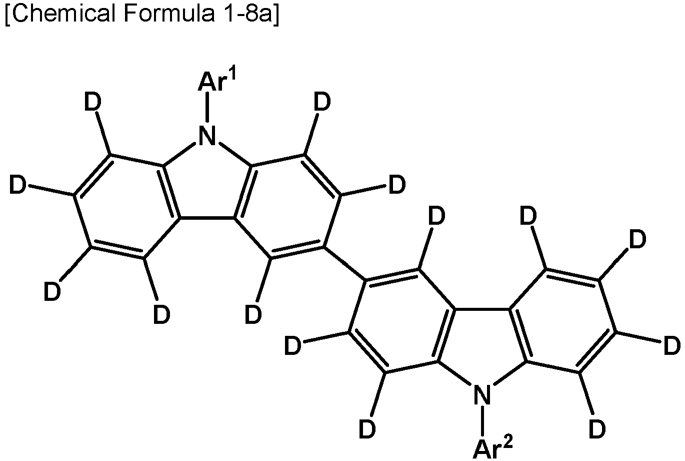

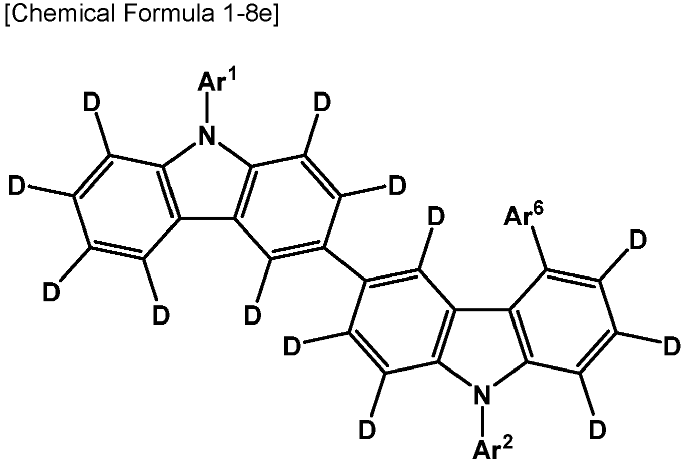

In Chemical Formula 1-8a and Chemical Formula 1-8e,
Ar¹ and Ar² are the same as described above, Ar⁶ is a C6 to C30 aryl group that is substituted or unsubstituted with deuterium.

For example, Ar⁶ may be a phenyl group that is substituted or unsubstituted deuterium, a biphenyl group that is substituted or unsubstituted deuterium, a terphenyl group that is substituted or unsubstituted deuterium, a naphthyl group that is substituted or unsubstituted deuterium, a phenanthrenyl group that is substituted or unsubstituted deuterium, an anthracenyl group that is substituted or unsubstituted deuterium, a triphenylene group that is substituted or unsubstituted deuterium, or a fluorenyl group that is substituted or unsubstituted deuterium.

For example, the compound for an organic optoelectronic device represented by Chemical Formula 1 may be one selected from compounds listed in Group 1, but is not limited thereto.

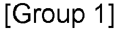

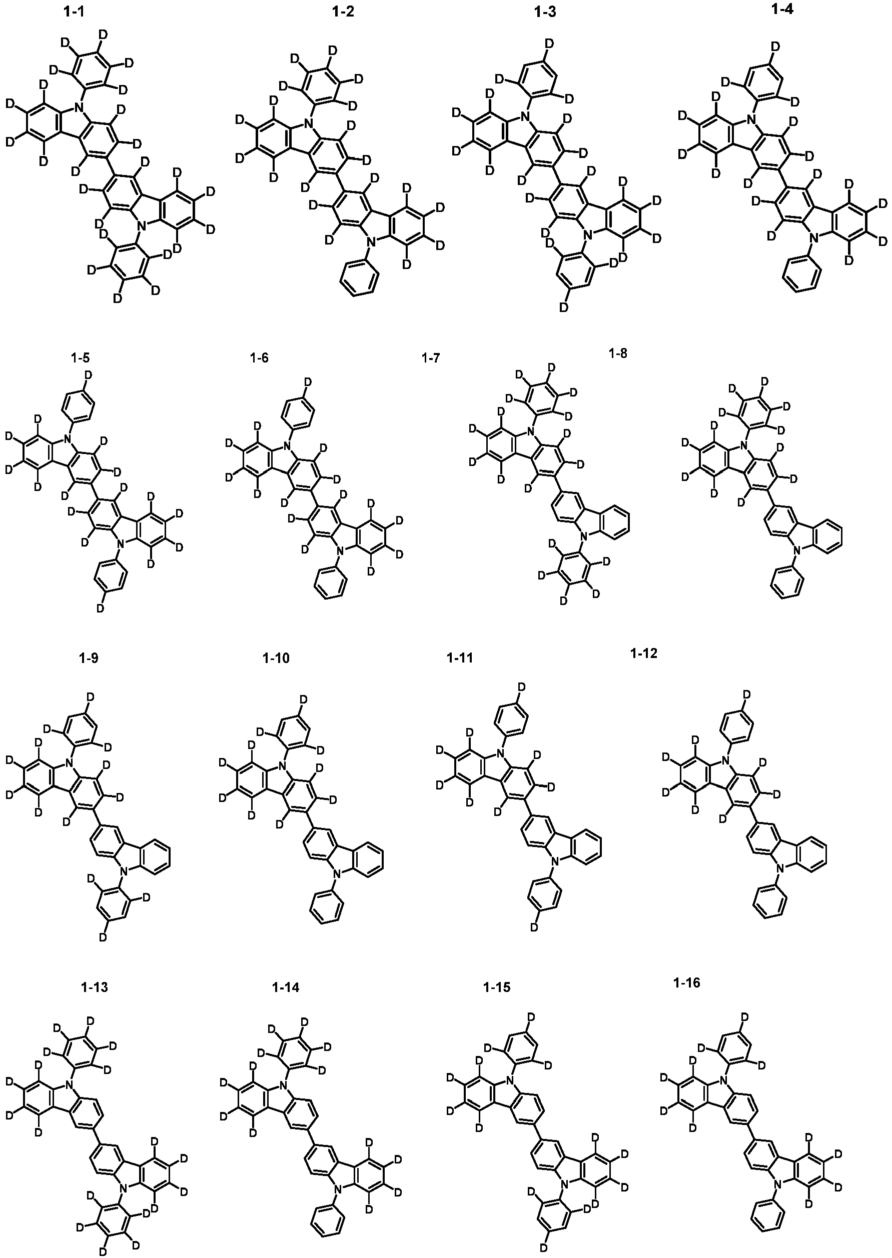

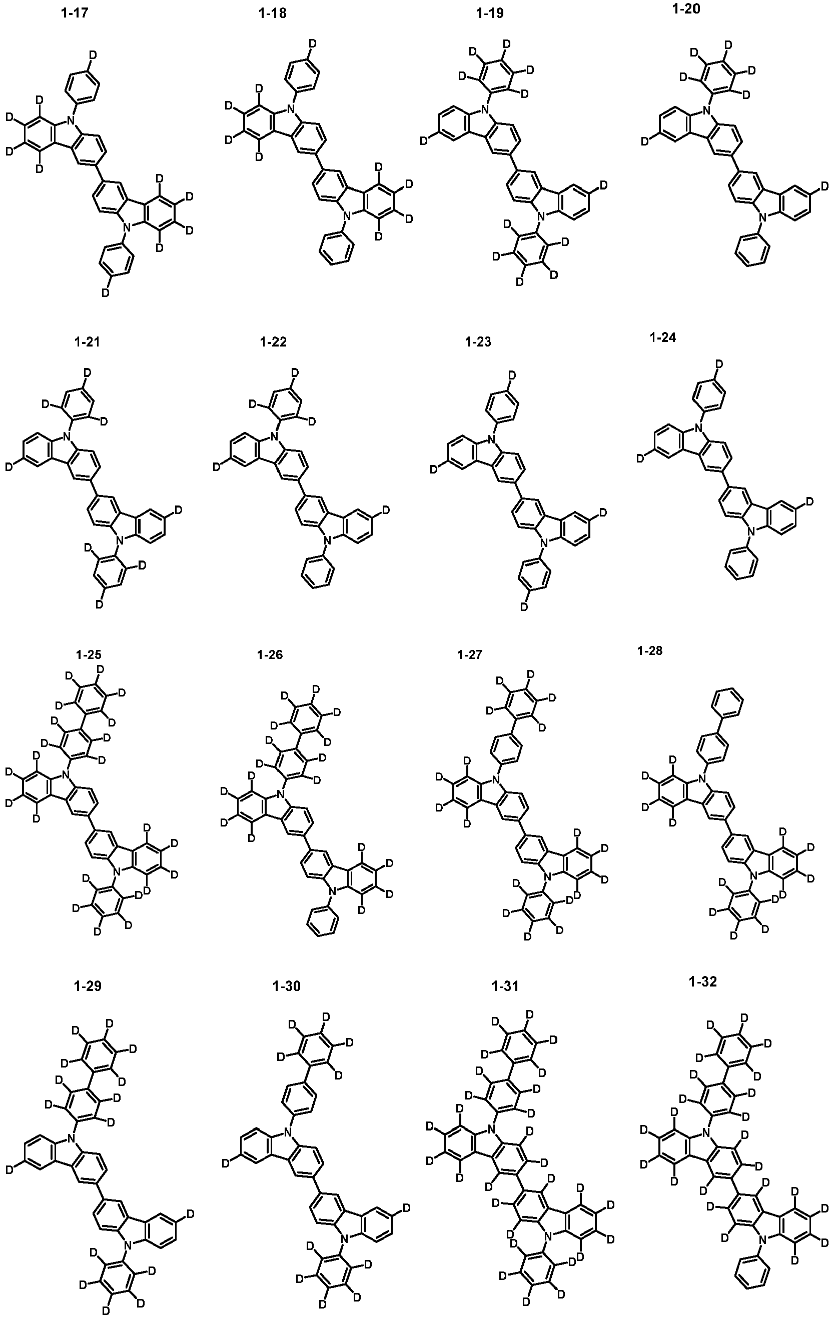

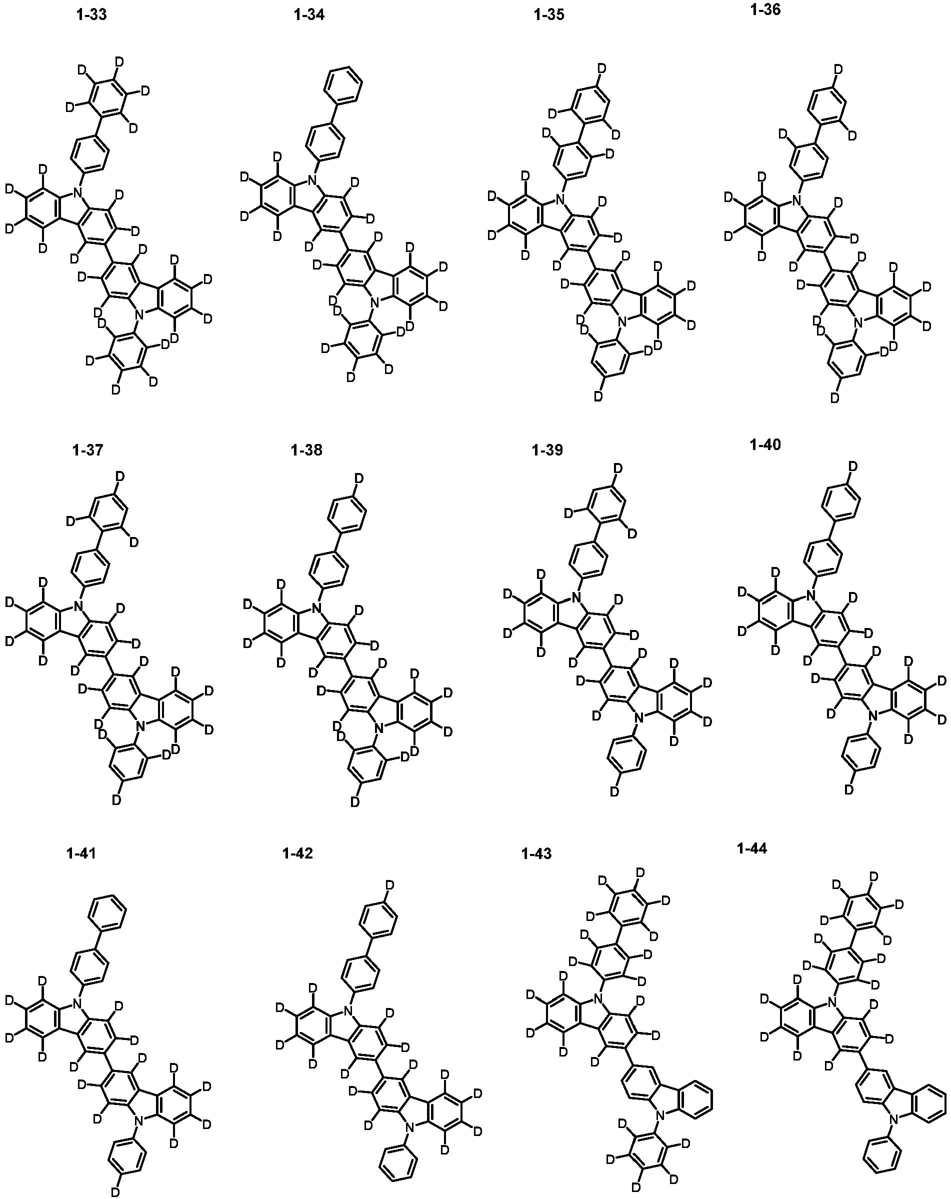

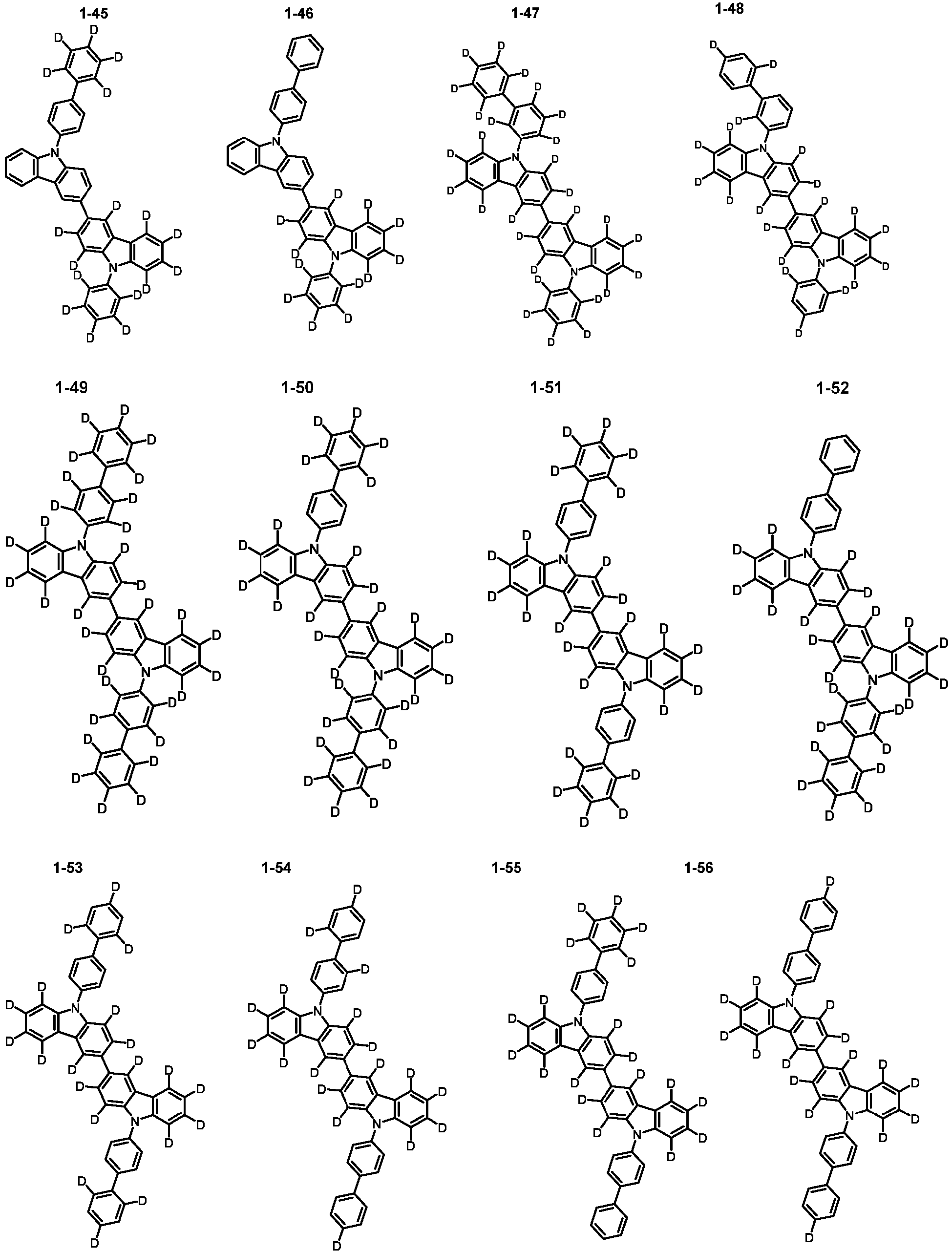

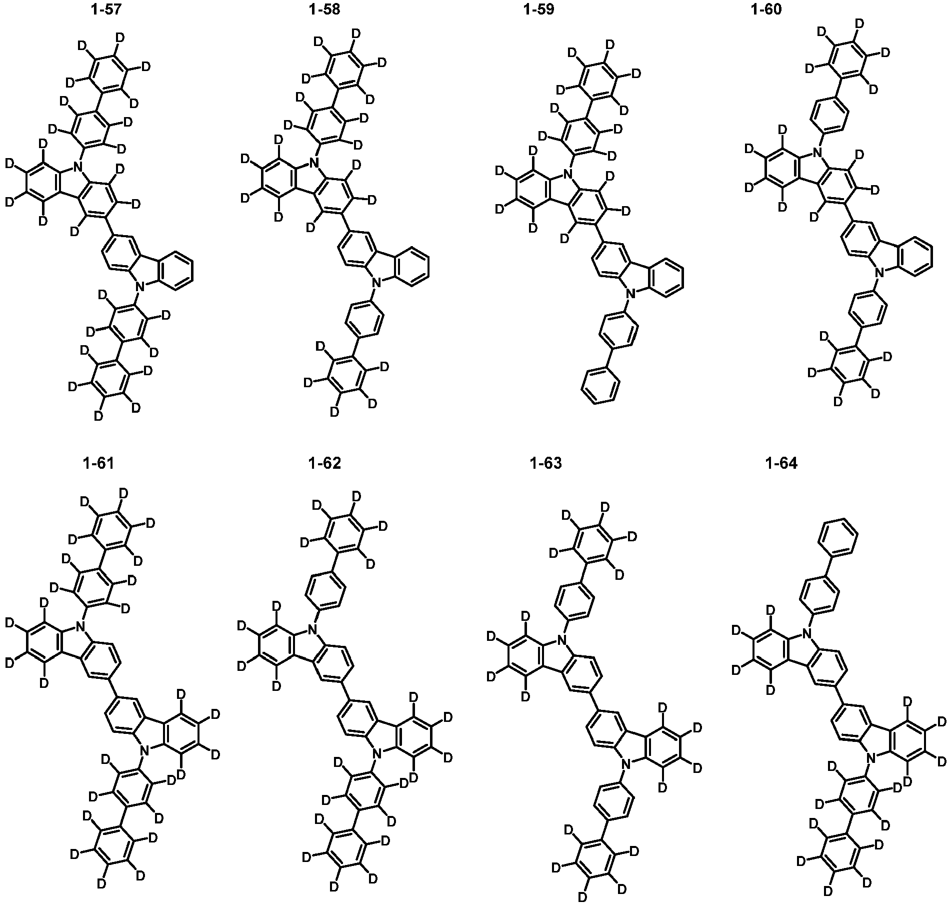

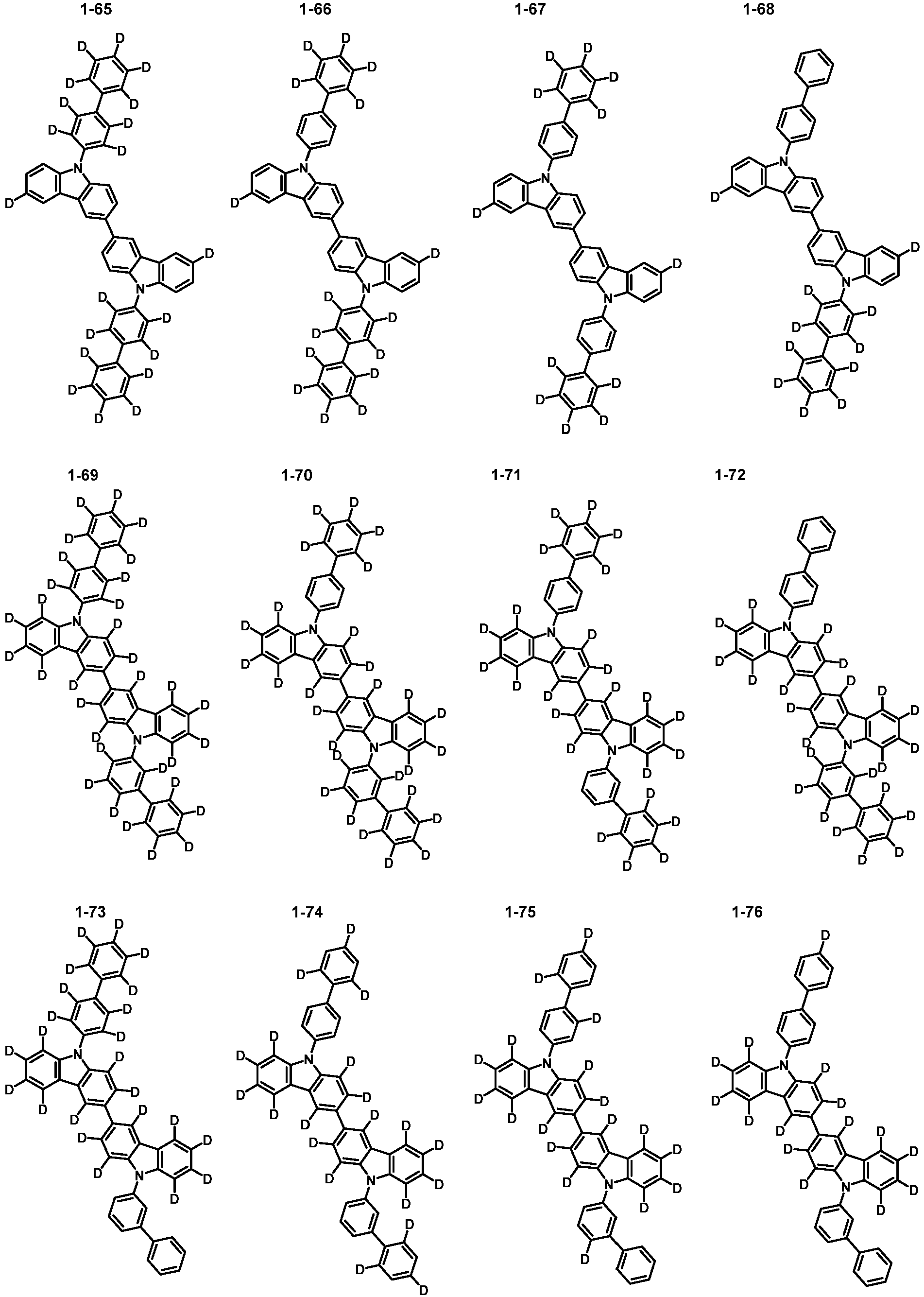

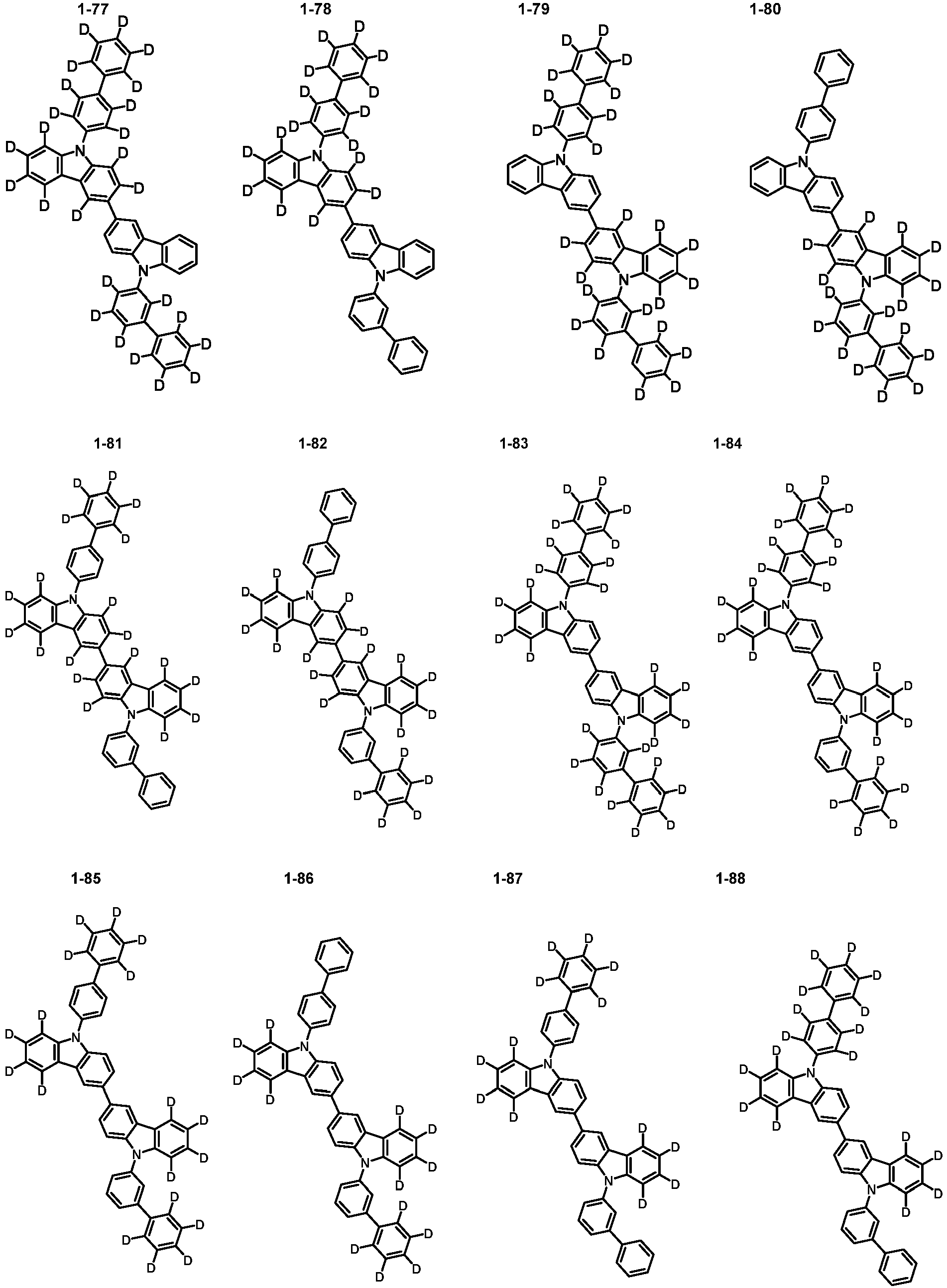

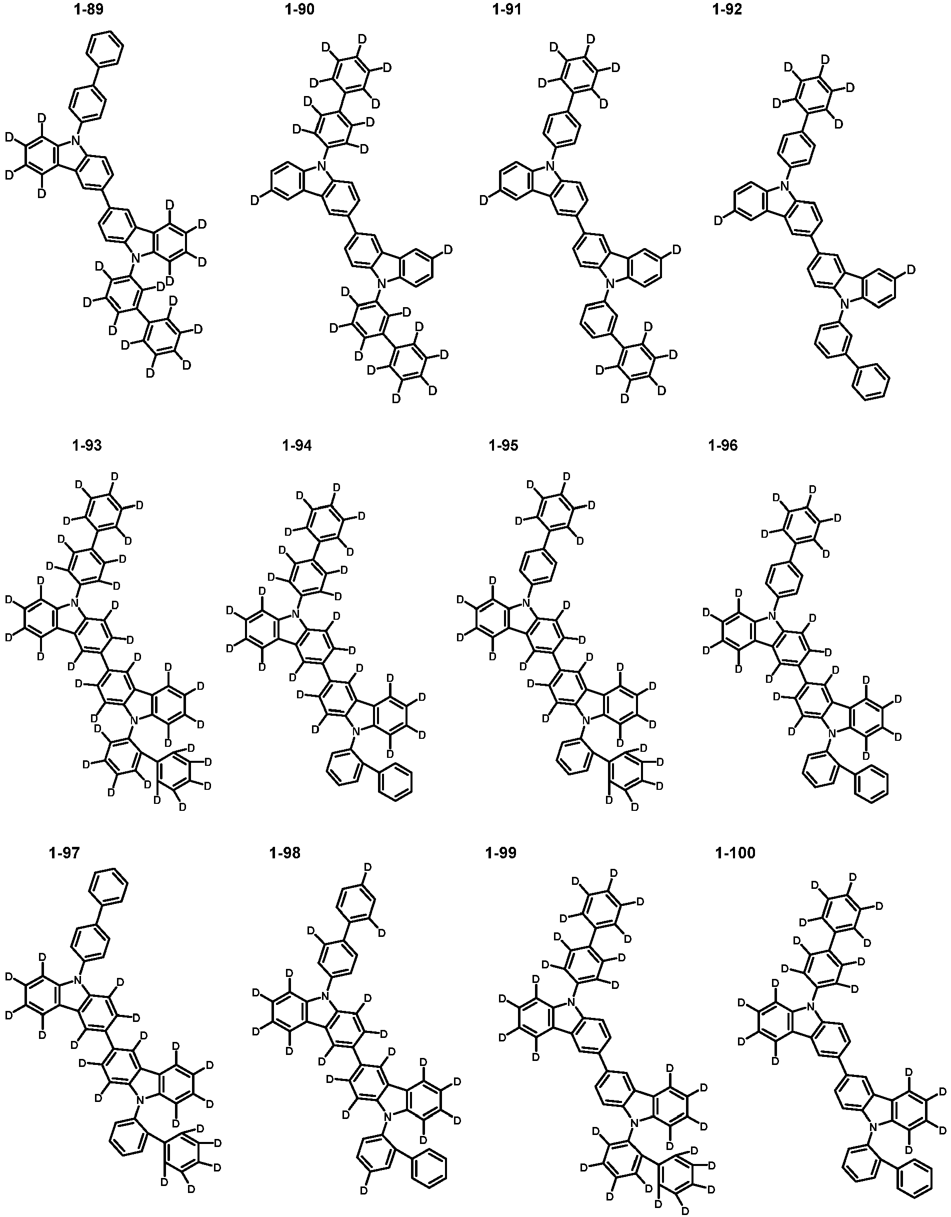

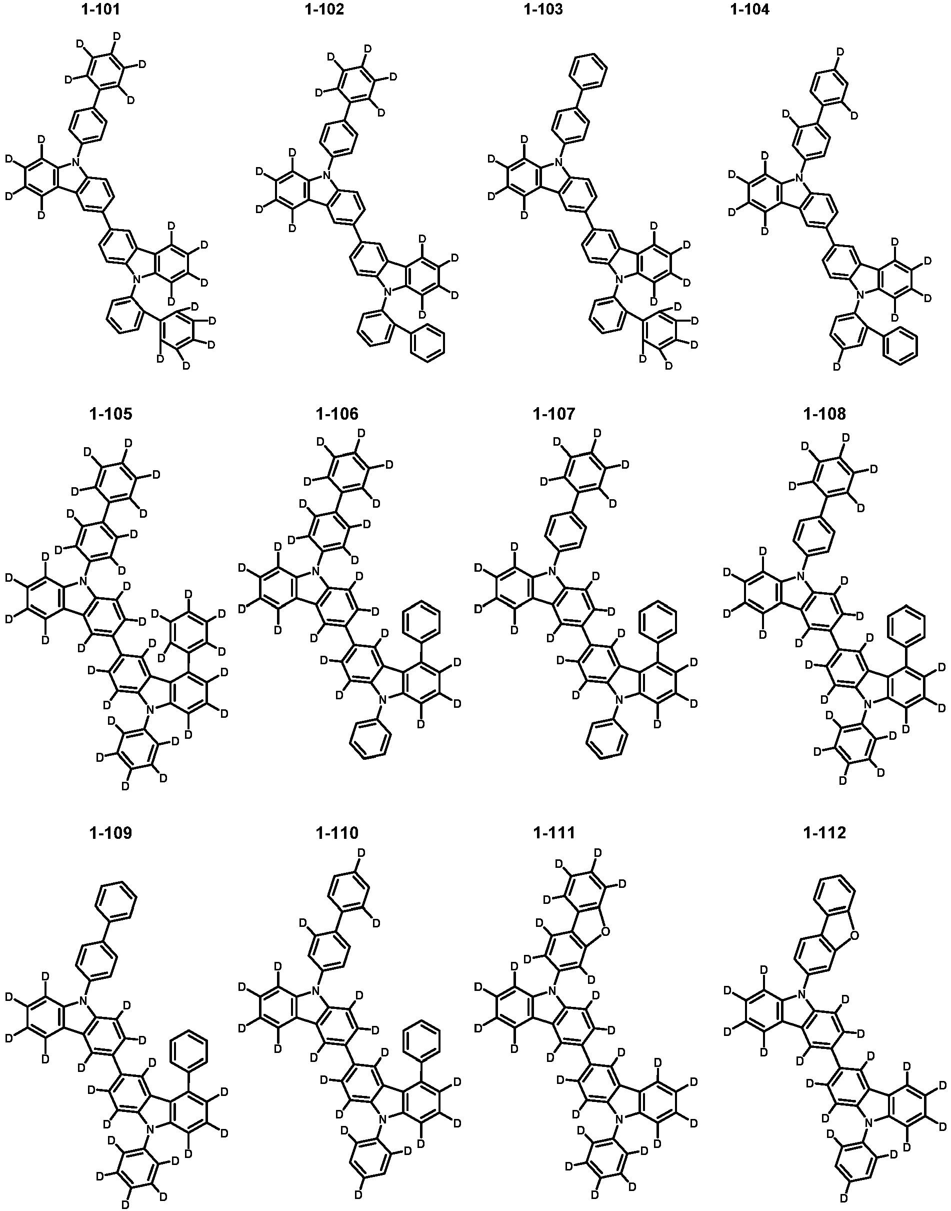

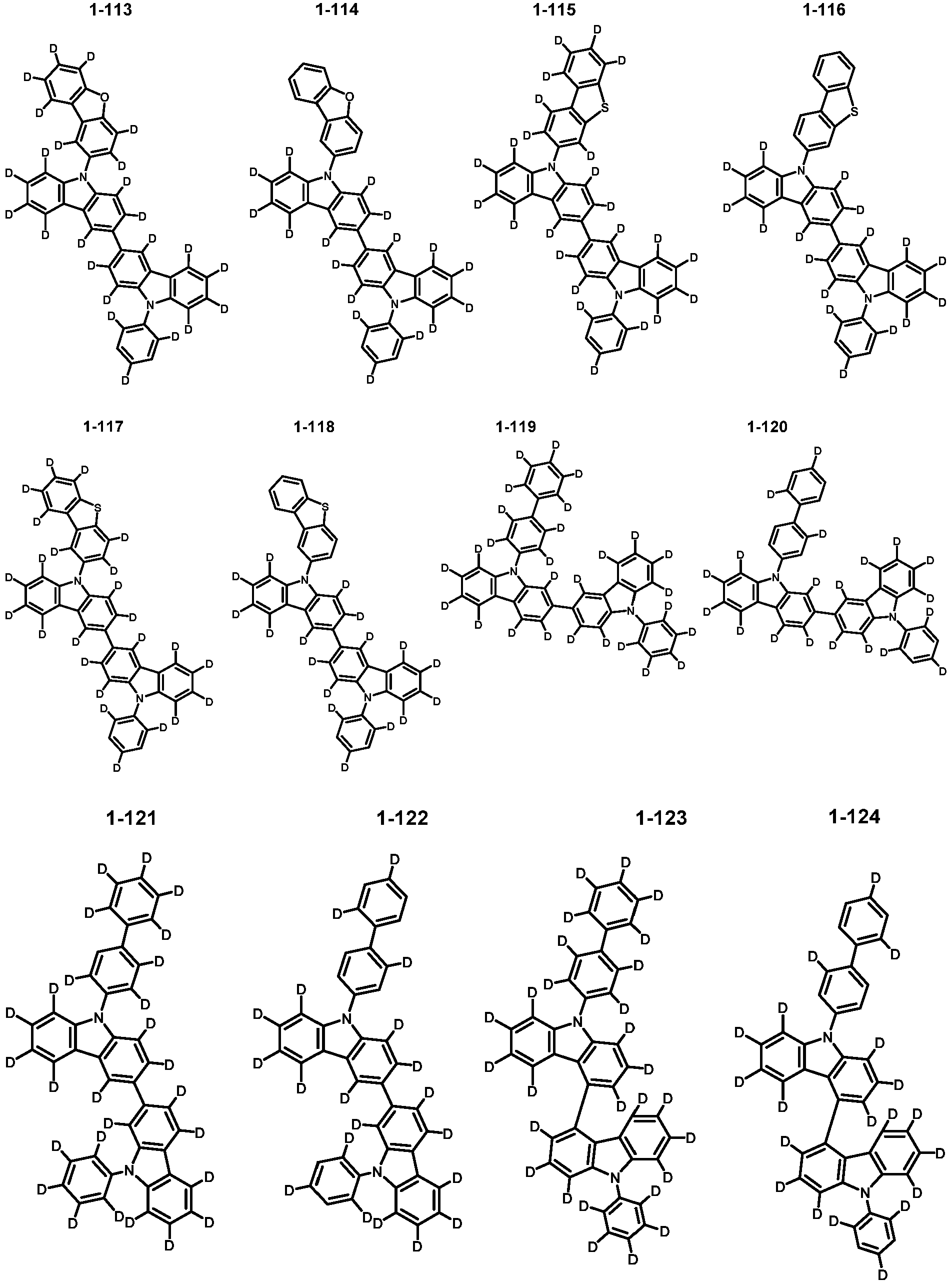

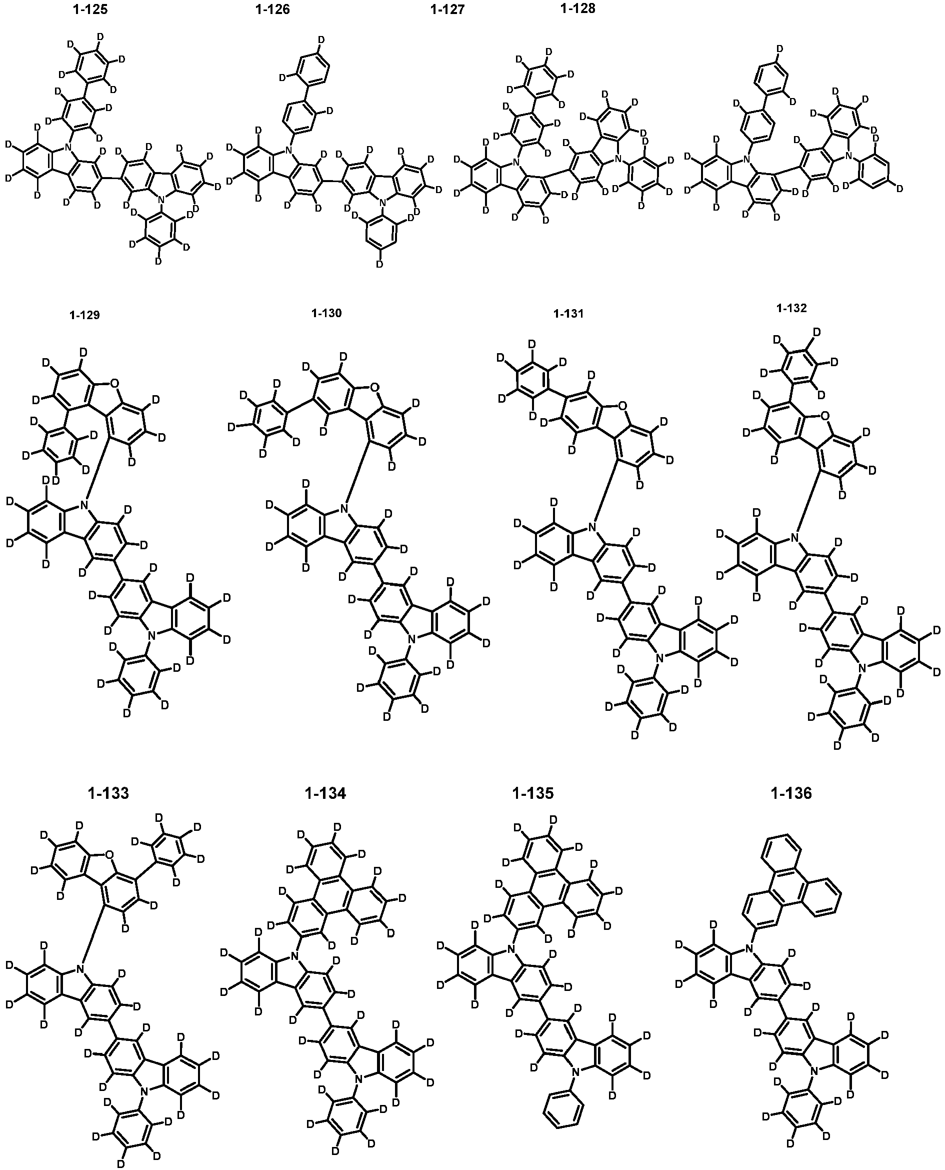

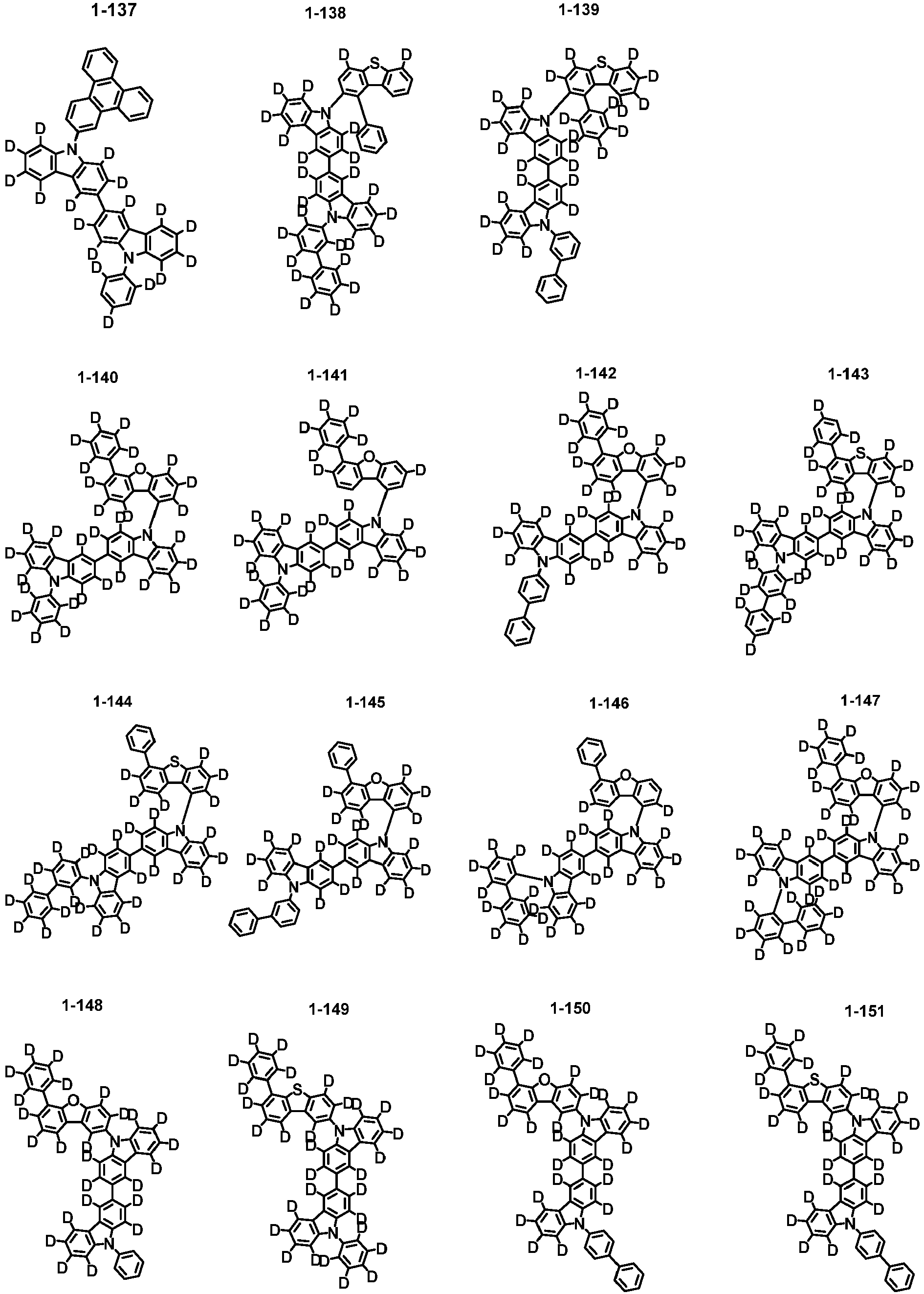

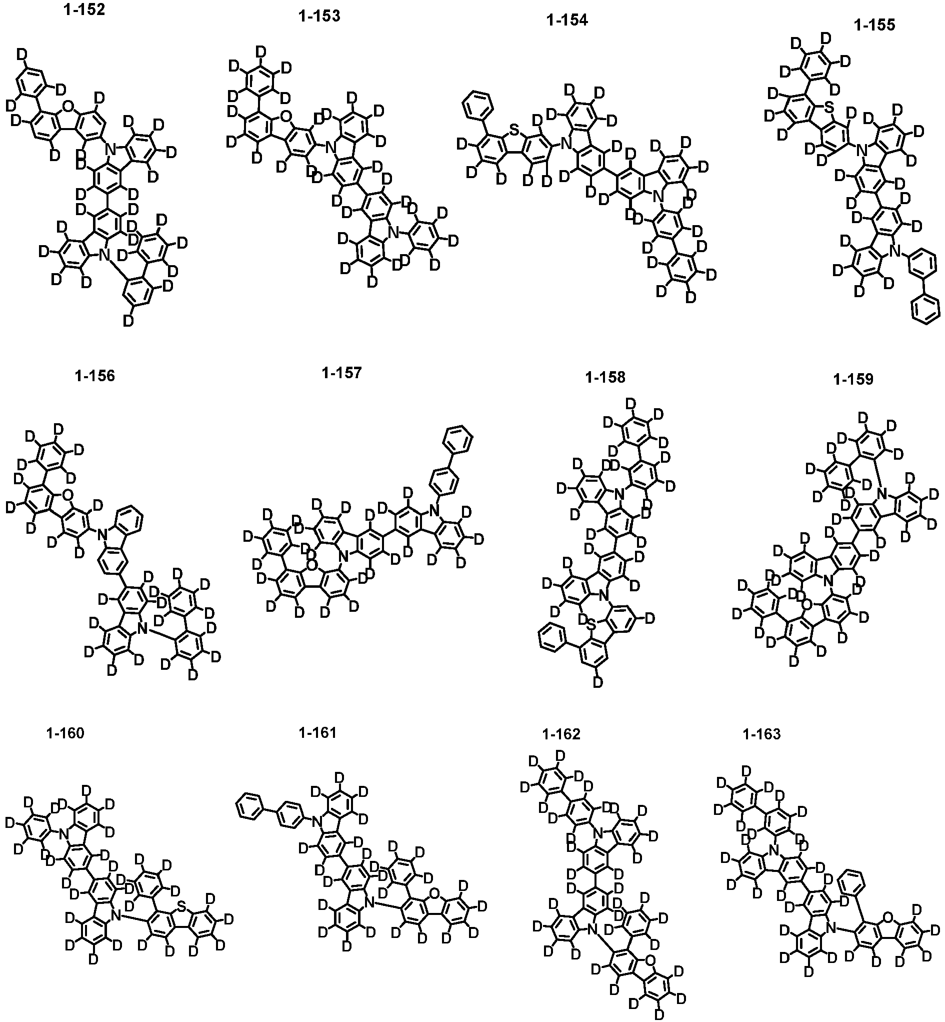

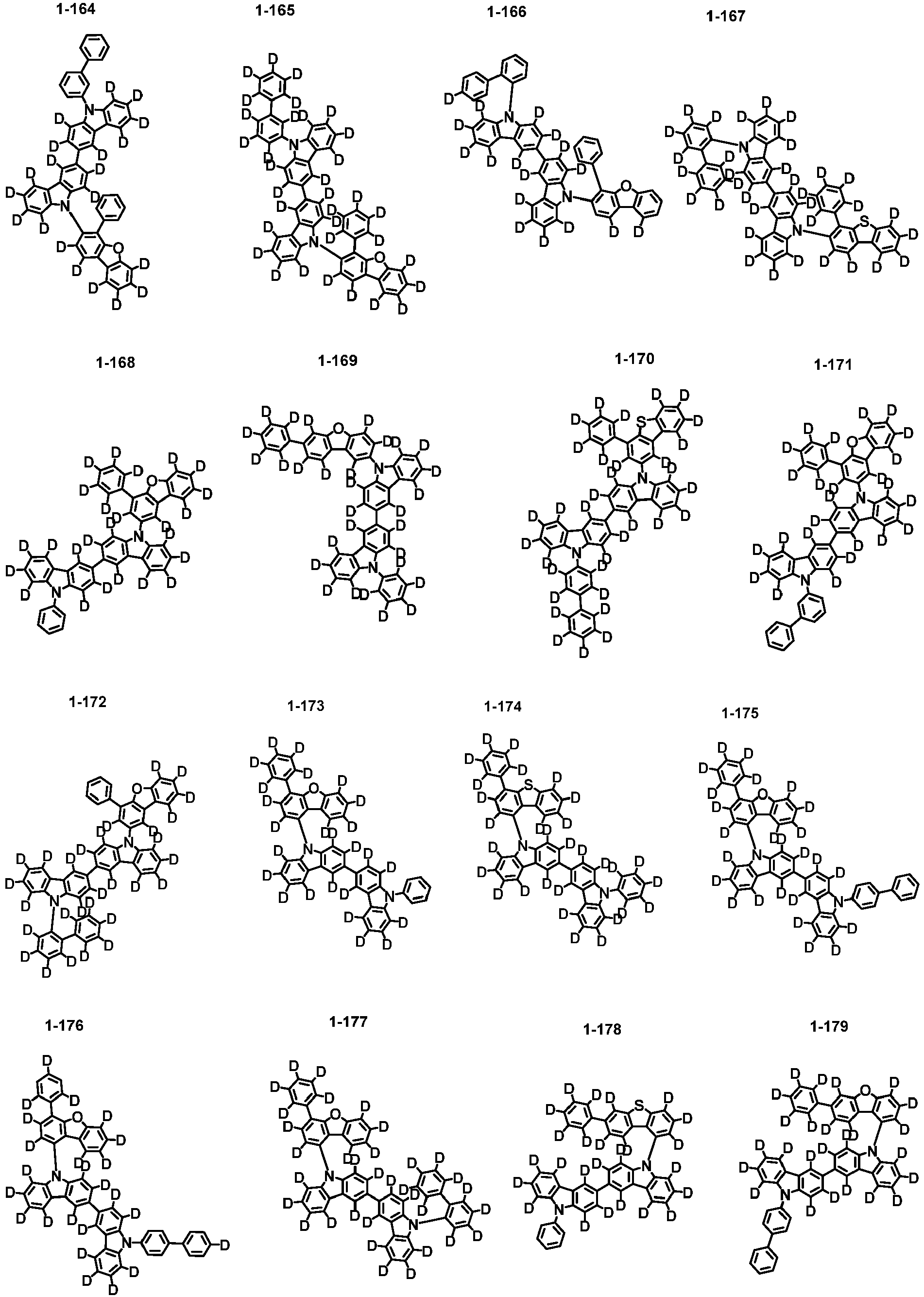

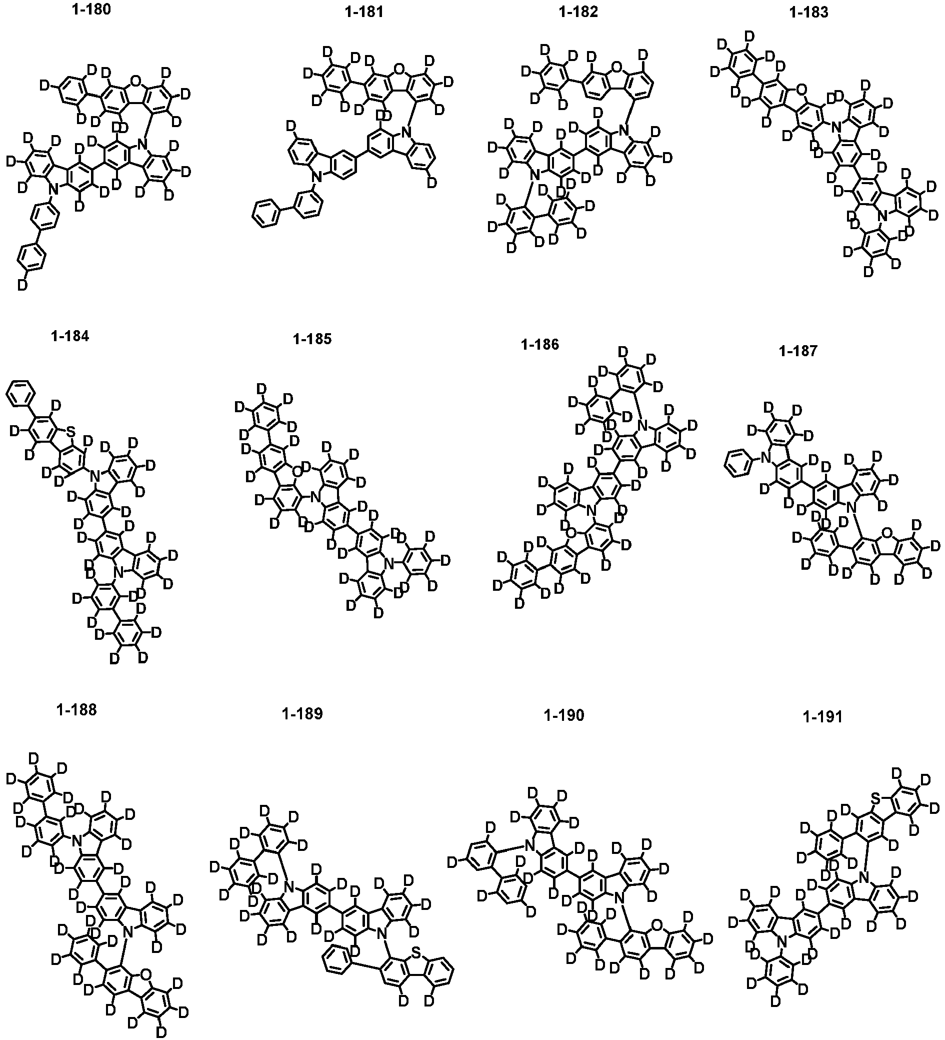

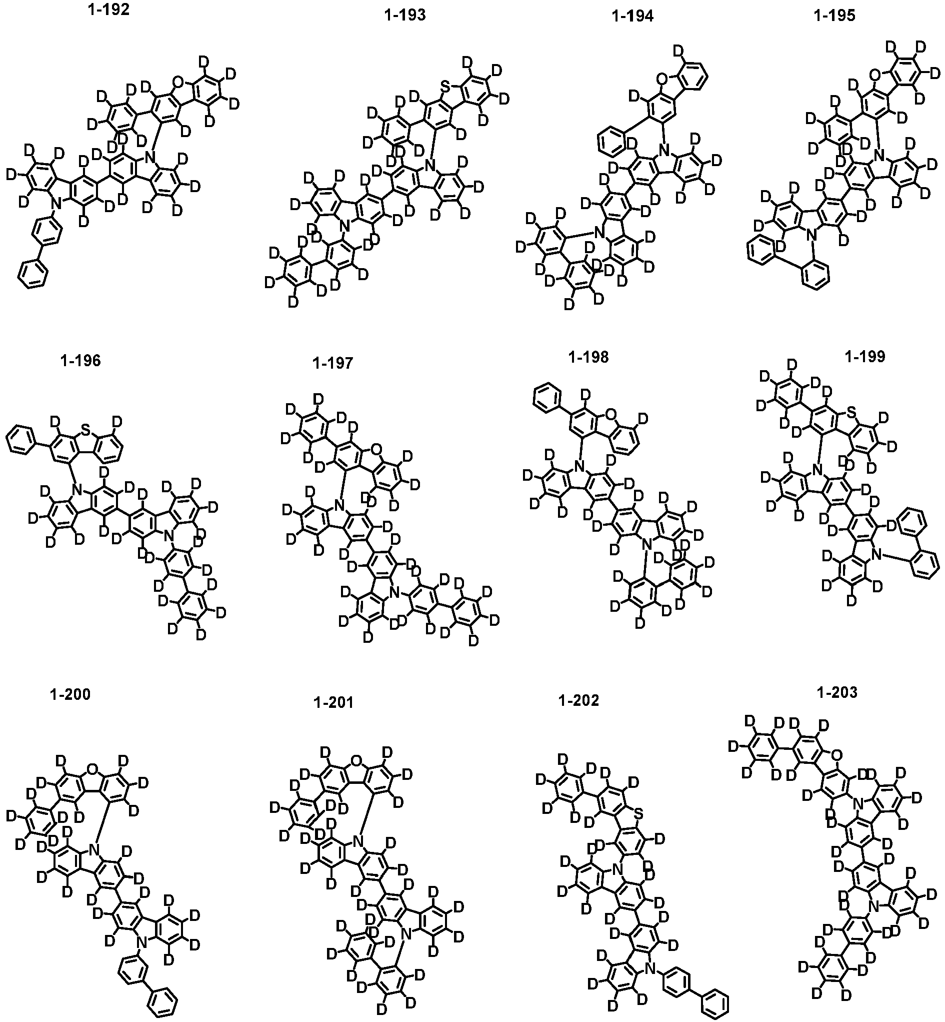

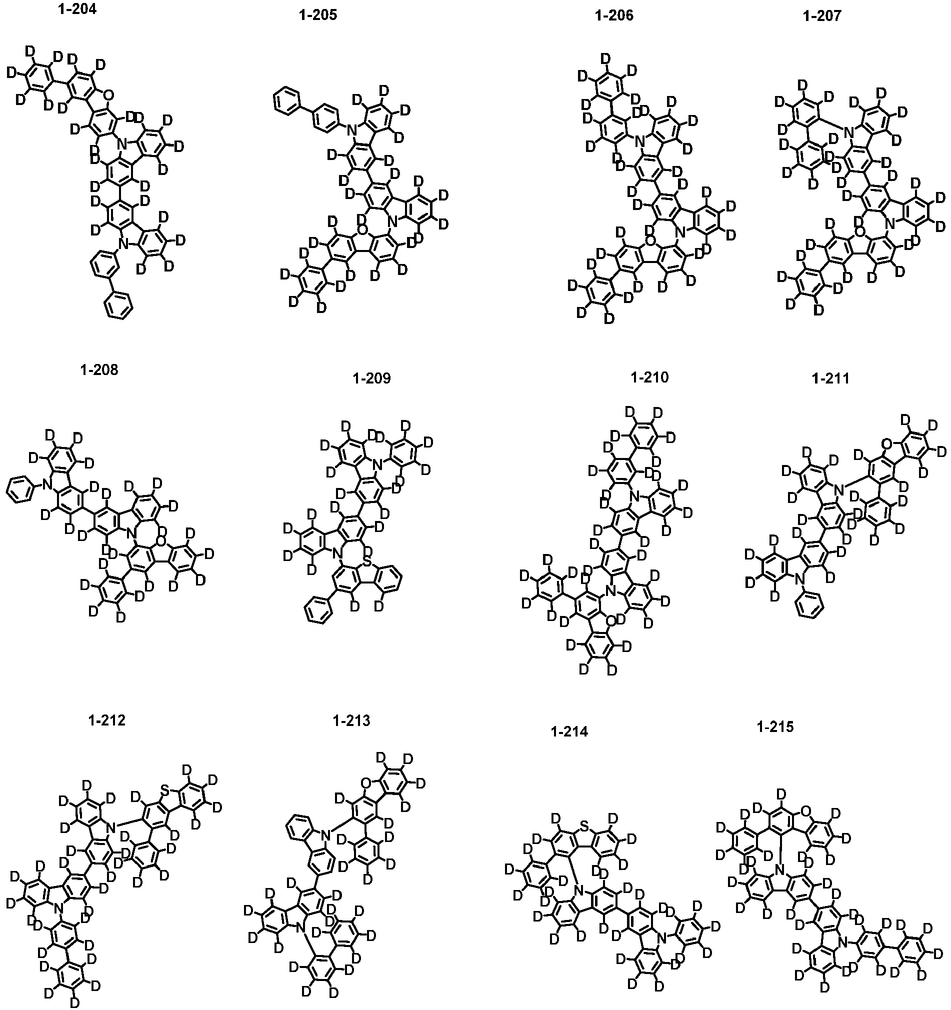

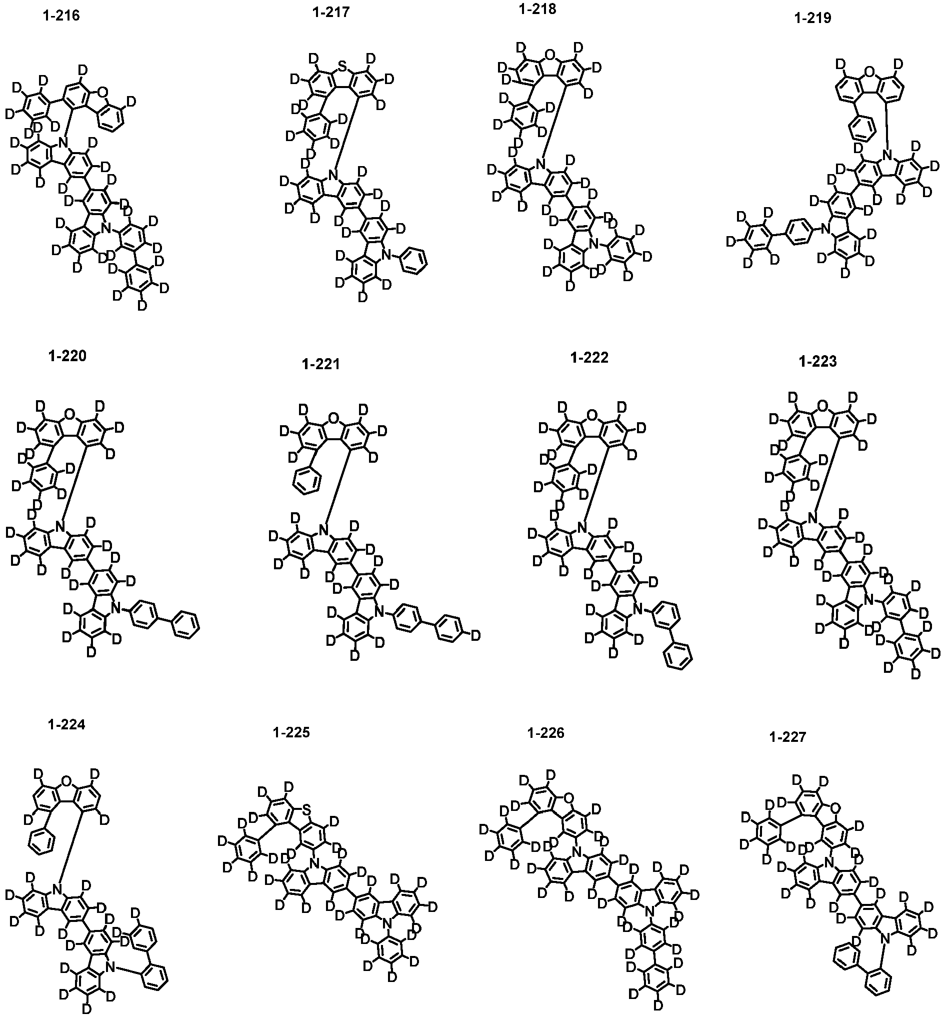

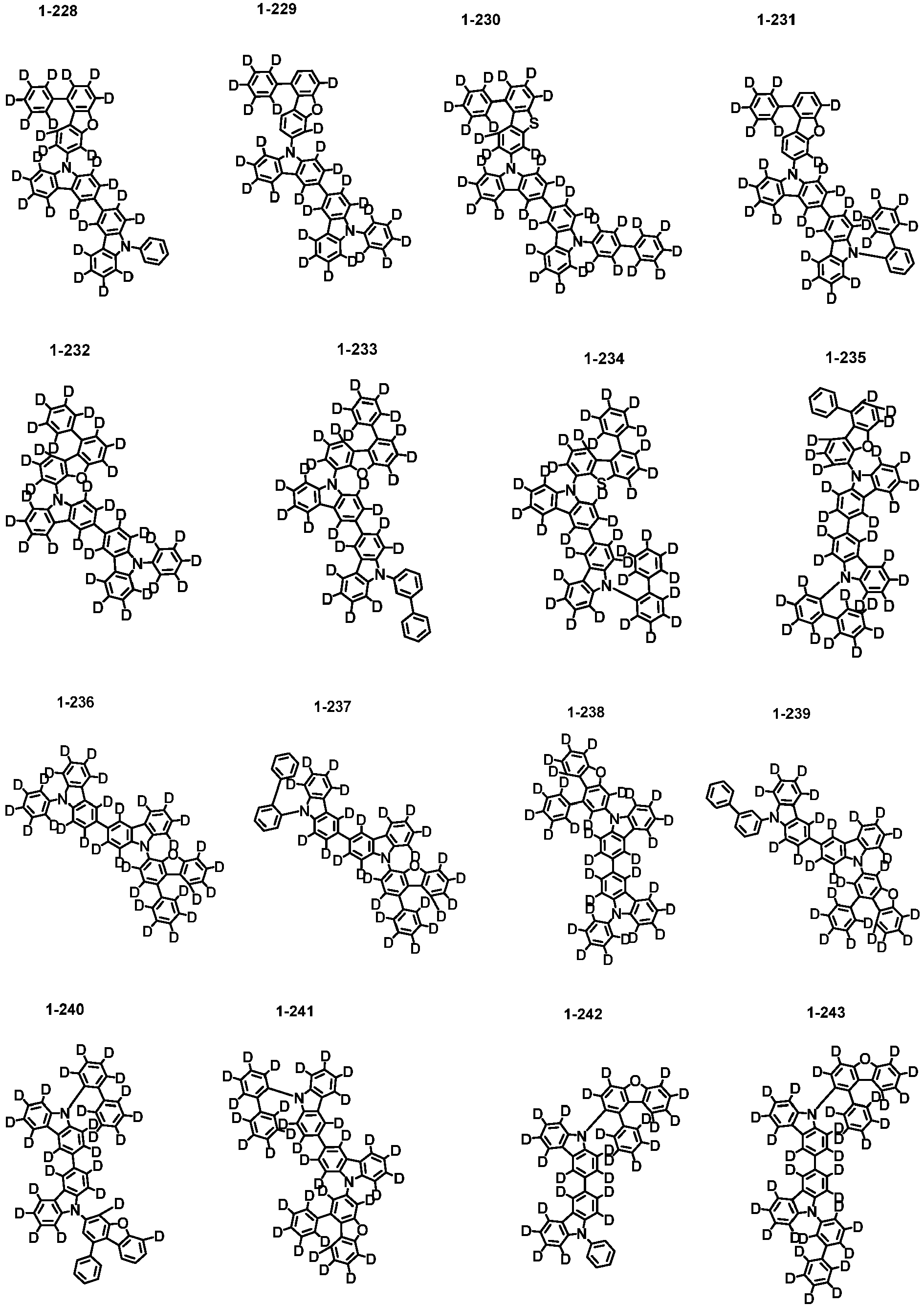

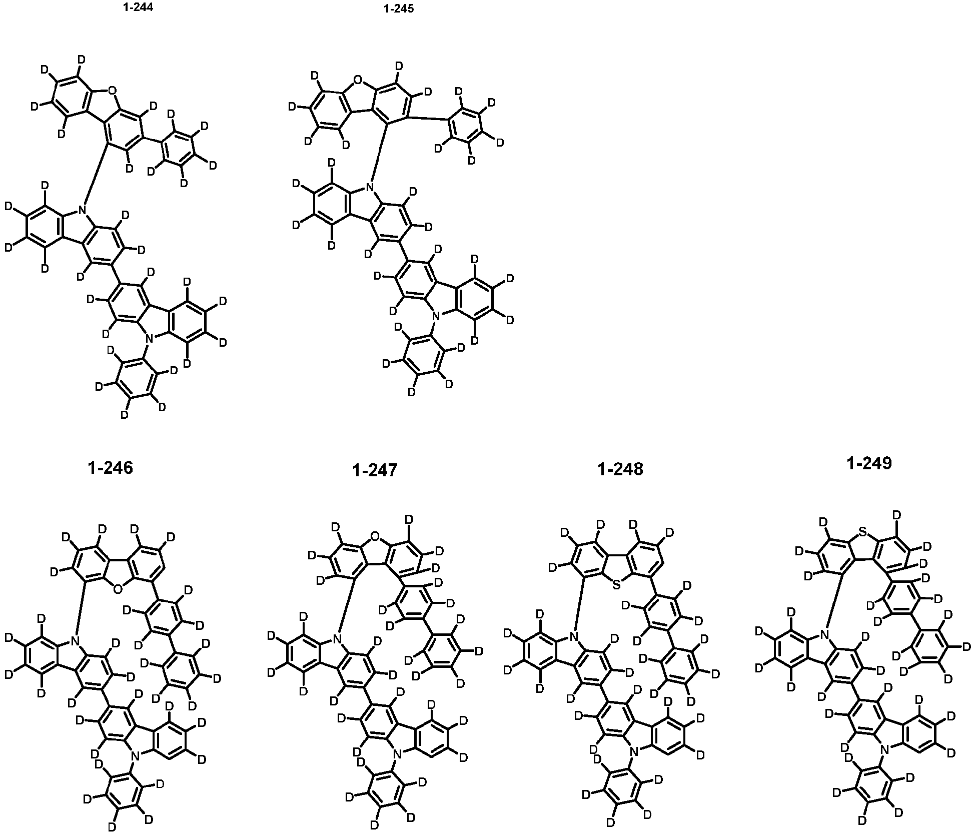

As a more specific example, the compound for an organic optoelectronic device according to the present invention is represented by Chemical Formula 1-8a, and
Ar¹ and Ar² may each be a phenyl group substituted with at least one deuterium, a biphenyl group substituted with at least one deuterium, a triphenylene group substituted with at least one deuterium, a dibenzofuranyl group substituted with at least one deuterium, or a dibenzothiophenyl group substituted with at least one deuterium.

In addition to the aforementioned compound for an organic optoelectronic device, one or more compounds may be further included.

For example, the aforementioned compound for an organic optoelectronic device may be applied in the form of a composition further including a known host material.

For example, the aforementioned compound for an organic optoelectronic device may further include a dopant.

The dopant may be, for example, a phosphorescent dopant, for example, a red, green or blue phosphorescent dopant, and may be, for example, a red phosphorescent dopant.

The dopant is a material mixed with the compound for an organic optoelectronic device in a small amount to cause light emission, and may be generally a material such as a metal complex that emits light by multiple excitation into a triplet or more. The dopant may be, for example, an inorganic, organic, or organic-inorganic compound, and one or more types thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example, a compound represented by Chemical Formula Z, but is not limited thereto.

[Chemical Formula Z] LMX

In Chemical Formula Z, M is a metal, and L and X are the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof and the L and X may be, for example a bidentate ligand.

Examples of ligands represented by L and X may be selected from the chemical formulas listed in Group A, but are not limited thereto.

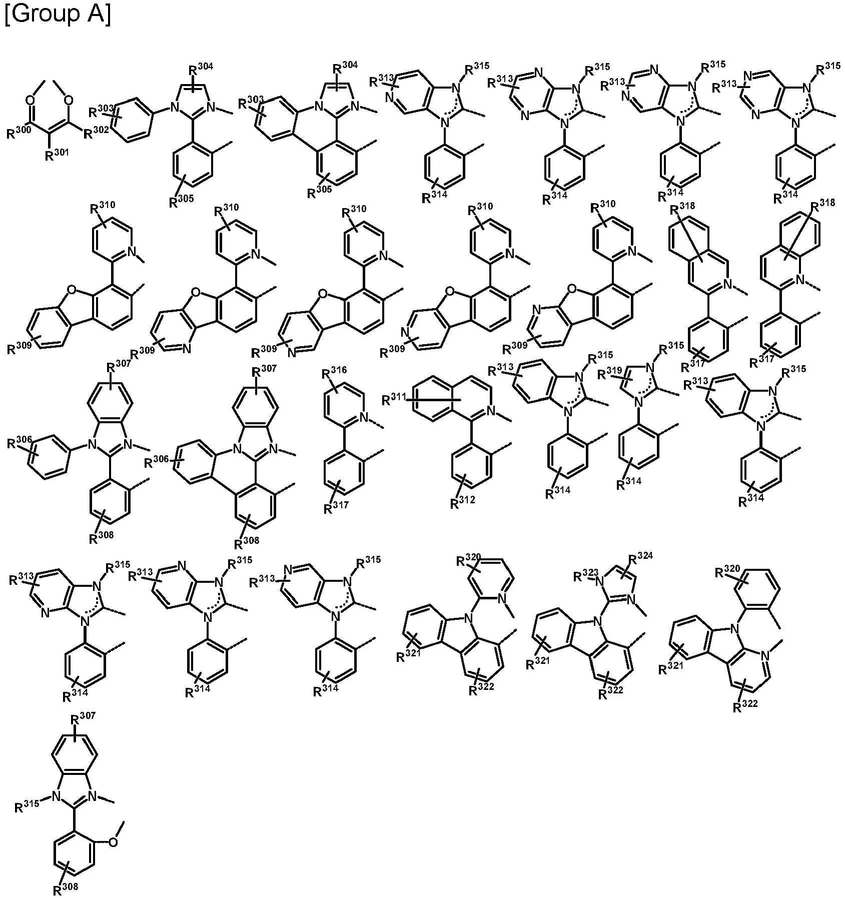

In Group A, R³⁰⁰ to R³⁰² are each independently hydrogen, deuterium, a C1 to C30 alkyl group that is substituted or unsubstituted with a halogen, a C6 to C30 aryl group that is substituted or unsubstituted with a C1 to C30 alkyl, or a halogen, and

R³⁰³ to R³²⁴ are each independently hydrogen, deuterium, halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SFs, a trialkylsilyl group having a substituted or unsubstituted C1 to C30 alkyl group, a dialkylarylsilyl group having a substituted or unsubstituted C1 to C30 alkyl group and a C6 to C30 aryl group, or a triarylsilyl group having a substituted or unsubstituted C6 to C30 aryl group.

As an example, it may include a dopant represented by Chemical Formula V.

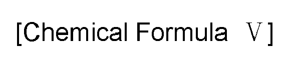

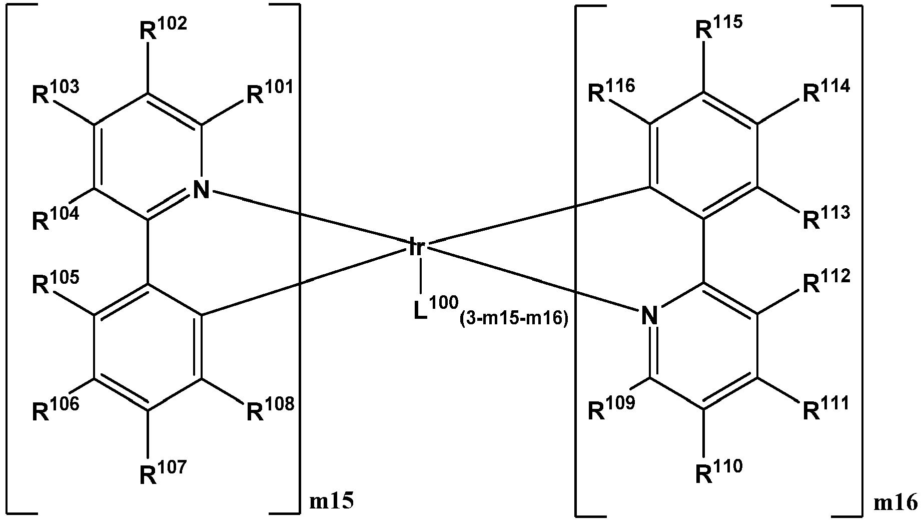

In Chemical Formula V,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group,
at least one of R¹⁰¹ to R¹¹⁶ is a functional group represented by Chemical Formula V-1,
L¹⁰⁰ is a bidentate ligand of a monovalent anion, and is a ligand that coordinates to iridium through a lone pair of carbons or heteroatoms, and
m15 and m16 are each independently any one of integers of 0 to 3, and m15 + m16 is any one of integers of 1 to 3,

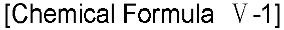

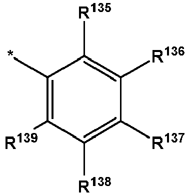

wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group, and
* means a portion linked to a carbon atom.

As an example, a dopant represented by Chemical Formula Z-1 may be included.

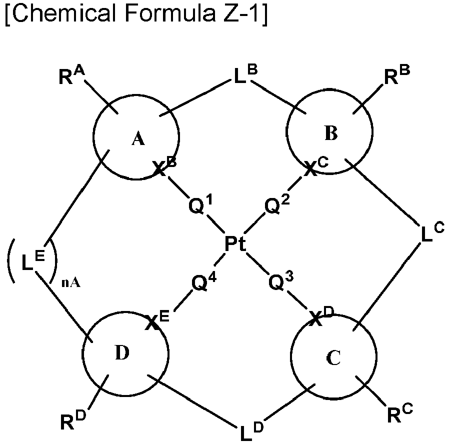

In Chemical Formula Z-1, rings A, B, C, and D each independently represent a 5-membered or 6-membered carbocyclic or heterocyclic ring;
R^{A}, R^{B}, R^{C}, and R^{D} each independently represent mono-, di-, tri-, or tetra-substitution, or unsubstitution;
L^{B}, L^{C}, and L^{D} are each independent selected from a direct bond, BR, NR, PR, O, S, Se, C=O, S=O, SO₂, CRR', SiRR', GeRR', and a combination thereof;
when nA is 1, L^{E} is selected from a direct bond, BR, NR, PR, O, S, Se, C=O, S=O, SO₂, CRR', SiRR', GeRR', and a combination thereof; when nA is 0, L^{E} does not exist; and
R^{A}, R^{B}, R^{C}, R^{D}, R, and R' are each independently selected from hydrogen, deuterium, a halogen, an alkyl group, a cycloalkyl group, a heteroalkyl group, an arylalkyl group, an alkoxy group, an aryloxy group, an amino group, a silyl group, an alkenyl group, a cycloalkenyl group, a heteroalkenyl group, an alkynyl group, an aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, and a combination thereof; any adjacent R^{A}, R^{B}, R^{C}, R^{D}, R, and R' are optionally linked to each other to provide a ring; X^{B}, X^{C}, X^{D}, and X^{E} are each independently selected from carbon and nitrogen; and Q¹, Q², Q³, and Q⁴ each represent oxygen or a direct bond.

The dopant according to an embodiment may be a platinum complex, and may be, for example, represented by Chemical Formula VI.

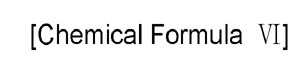

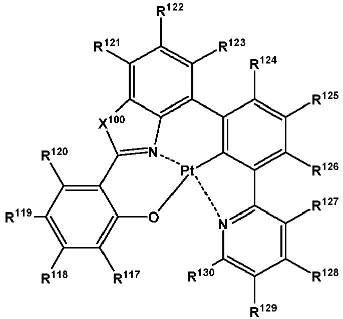

In Chemical Formula VI,
X¹⁰⁰ is selected from O, S, and NR¹³¹,
R¹¹⁷ to R¹³¹ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group, and
at least one of R¹¹⁷ to R¹³¹ is -SiR¹³²R¹³³R¹³⁴ or a tert-butyl group.

Hereinafter, an organic optoelectronic device including the aforementioned compound for an organic optoelectronic device is described.

The organic optoelectronic device may be any device to convert electrical energy into photoenergy and vice versa without particular limitation, and may be, for example an organic photoelectric device, an organic light emitting diode, an organic solar cell, and an organic photoconductor drum.

Herein, an organic light emitting diode as one example of an organic optoelectronic device is described referring to drawings.

FIG. 1 is a cross-sectional view illustrating an organic light emitting diode according to an embodiment.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a small work function to help electron injection, and may be for example a metal, a metal oxide, and/or a conductive polymer. The cathode 110 may be for example a metal such as magnesium, calcium, sodium, potassium, titanium, indium, yttrium, lithium, gadolinium, aluminum silver, tin, lead, cesium, barium, and the like, or an alloy thereof; a multi-layer structure material such as LiF/Al, LiO₂/Al, LiF/Ca, and BaF₂/Ca, but is not limited thereto.

The organic layer 105 may include the aforementioned compound for an organic optoelectronic device.

The organic layer 105 may include the light emitting layer 130 and the light emitting layer 130 may include the aforementioned compound for an organic optoelectronic device.

The composition for an organic optoelectronic device further including a dopant may be, for example, a red light emitting composition.

The light emitting layer 130 may include, for example, the aforementioned compound for an organic optoelectronic device as a phosphorescent host.

The organic layer may further include a charge transport region in addition to the light emitting layer.

The charge transport region may be, for example, a hole transport region 140.

The hole transport region 140 may further increase hole injection and/or hole mobility and block electrons between the anode 120 and the light emitting layer 130.

Specifically, the hole transport region 140 may include a hole transport layer between the anode 120 and the light emitting layer 130, and a hole transport auxiliary layer between the light emitting layer 130 and the hole transport layer, and at least one of the compounds of Group B may be included in at least one of the hole transport layer and the hole transport auxiliary layer.

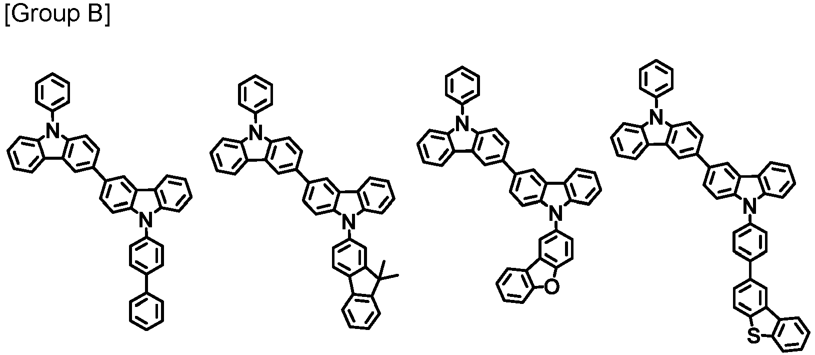

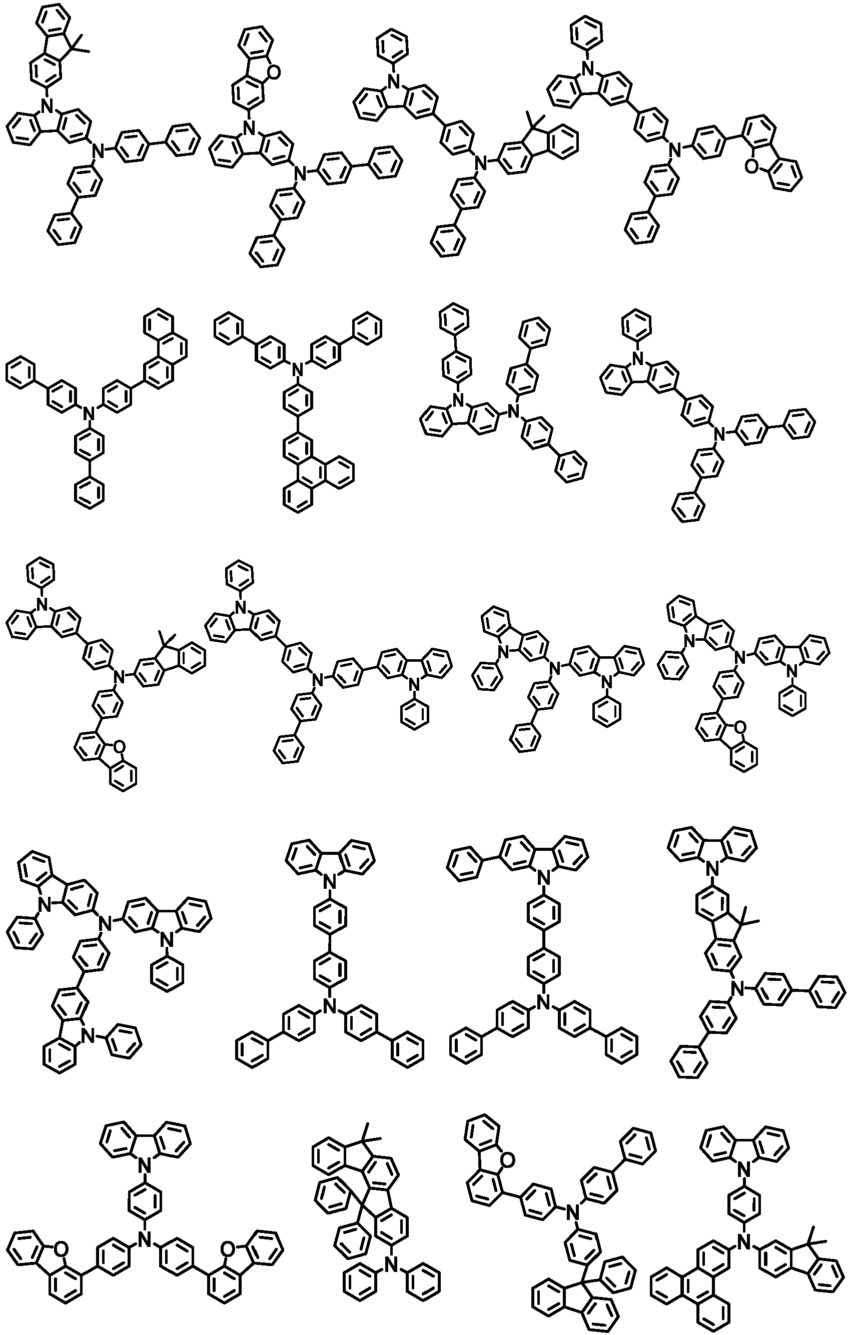

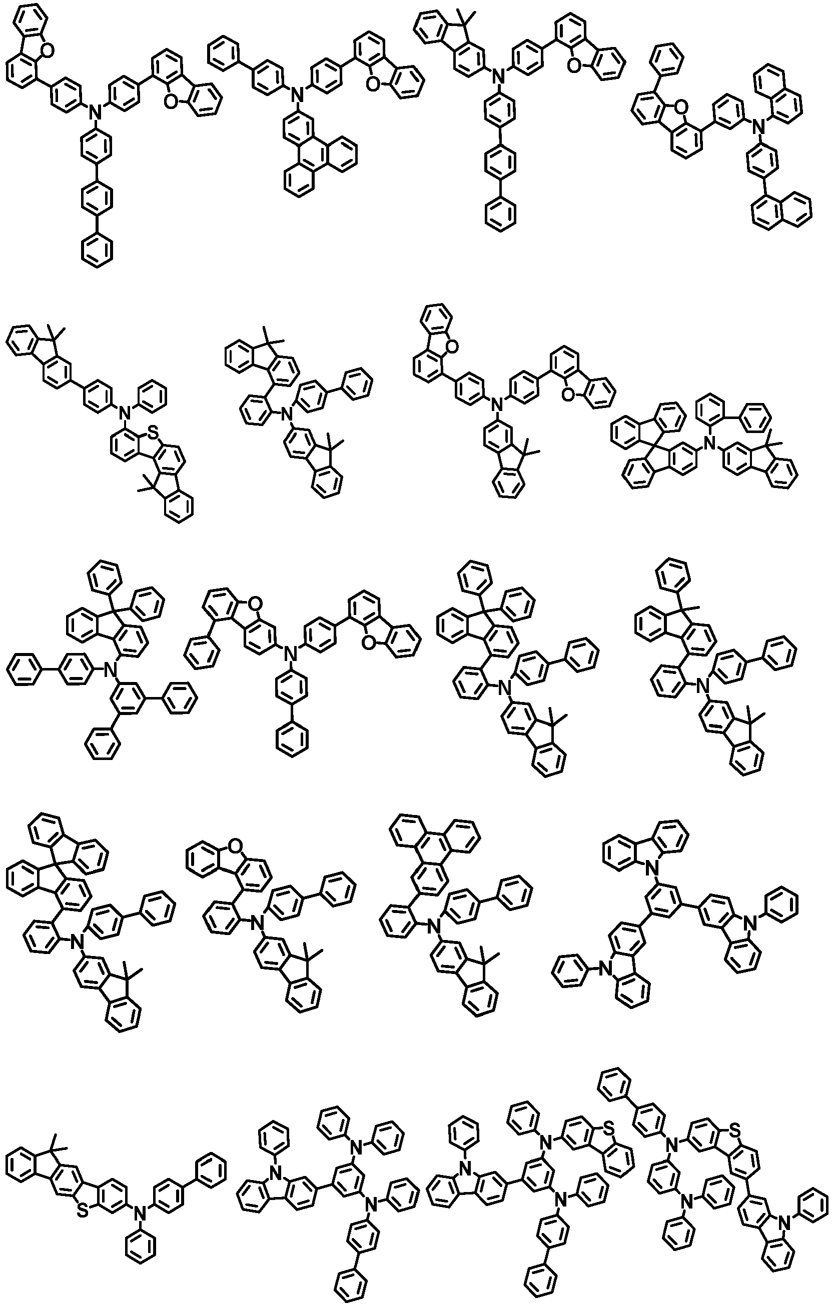

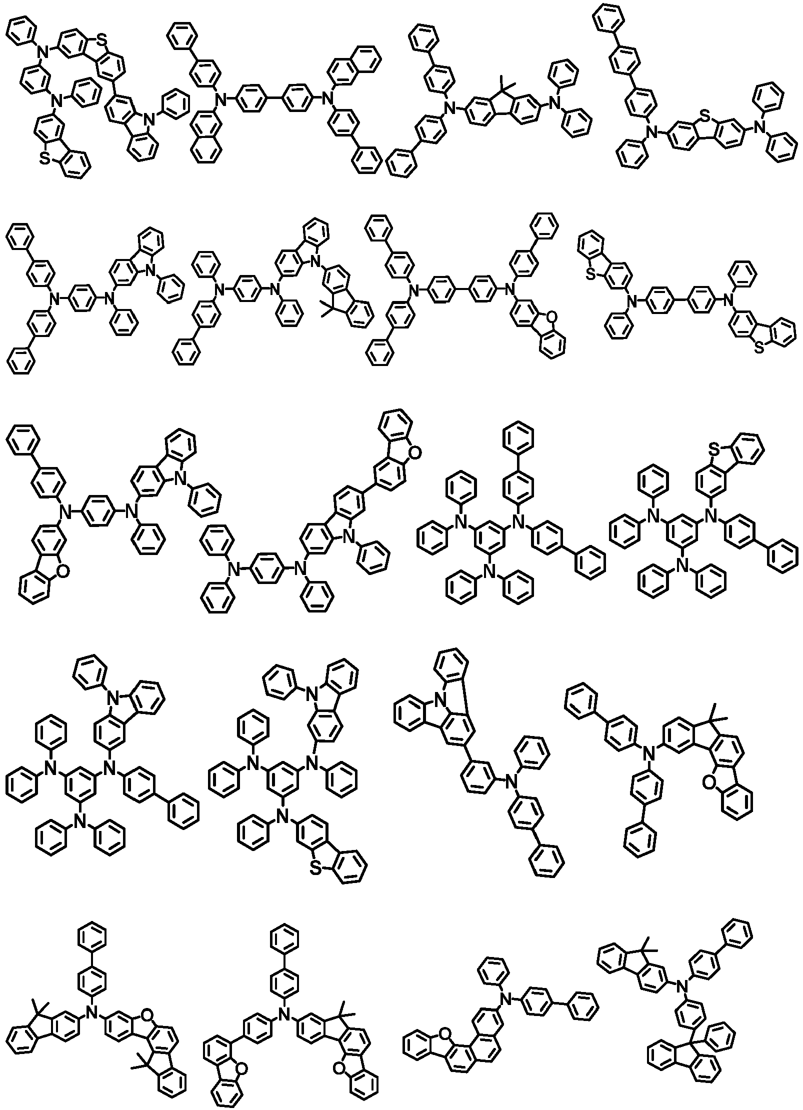

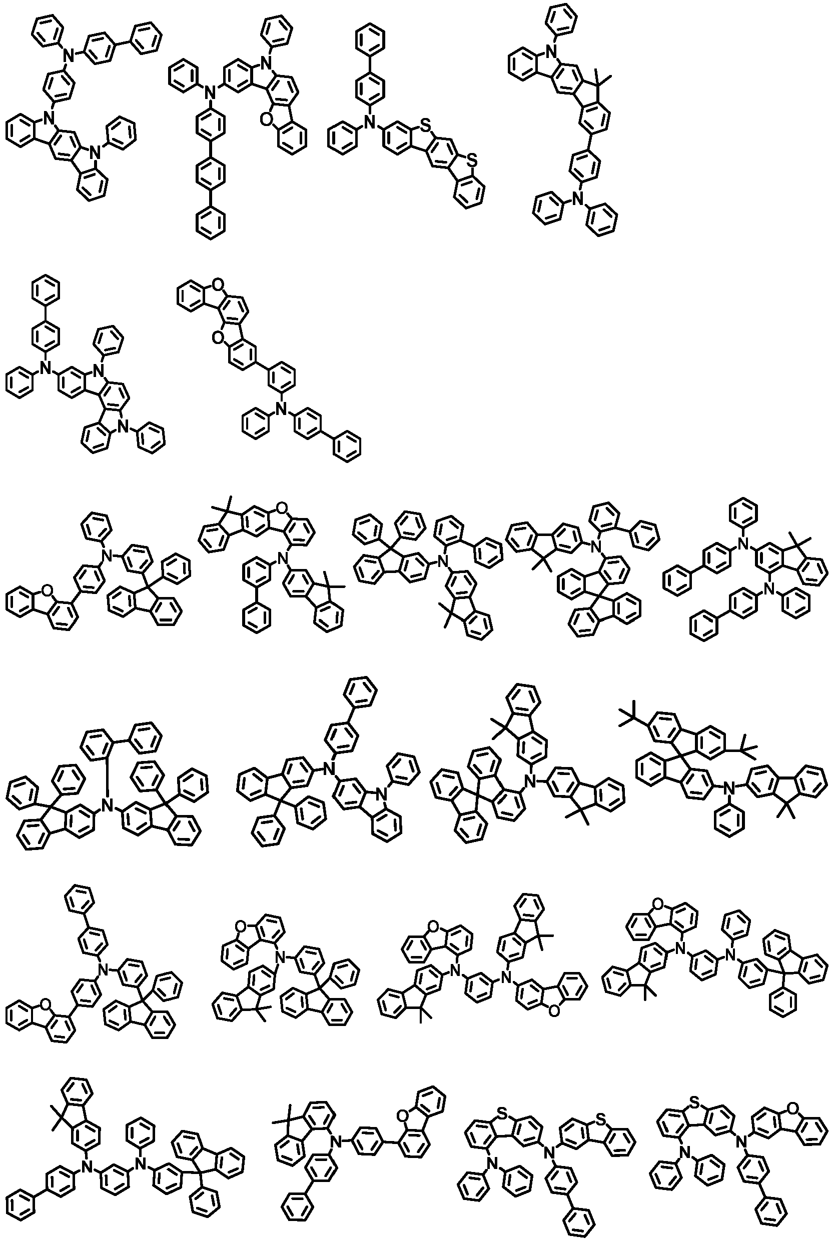

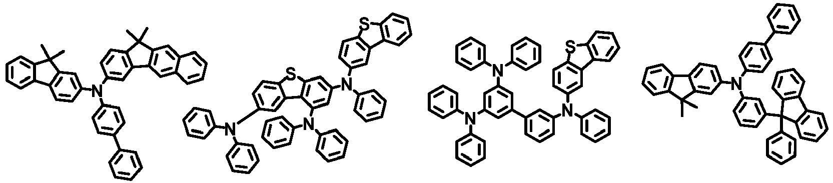

In the hole transport region 140, known compounds disclosed in US5061569A, JP1993-009471A, WO1995-009147A1, JP1995-126615A, JP1998-095973A, and the like and compounds similar thereto may be used in addition to the aforementioned compound.

Also, the charge transport region may be, for example, an electron transport region 150.

The electron transport region 150 may further increase electron injection and/or electron mobility and block holes between the cathode 110 and the light emitting layer 130.

Specifically, the electron transport region 150 may include an electron transport layer between the cathode 110 and the light emitting layer 130, and an electron transport auxiliary layer between the light emitting layer 130 and the electron transport layer, and at least one of the compounds of Group C may be included in at least one of the electron transport layer and the electron transport auxiliary layer.

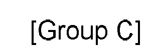

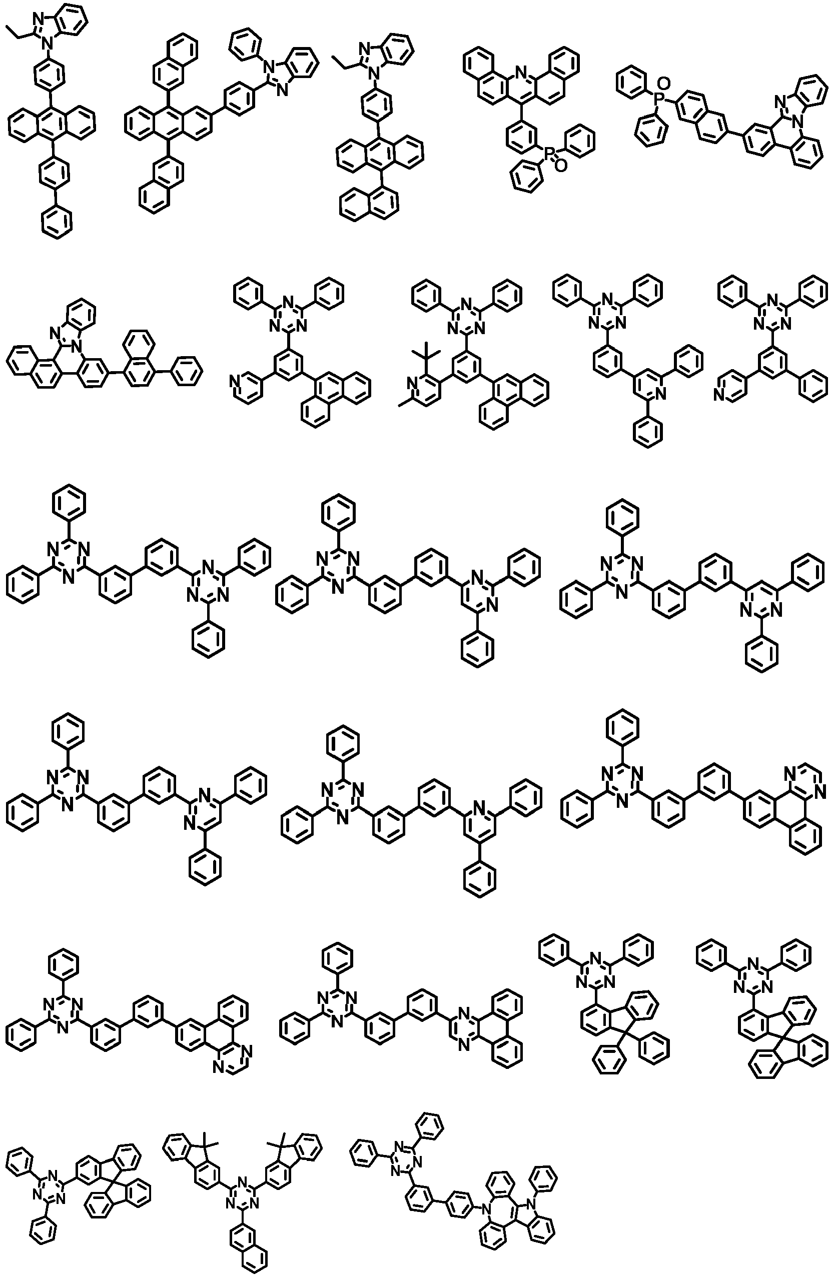

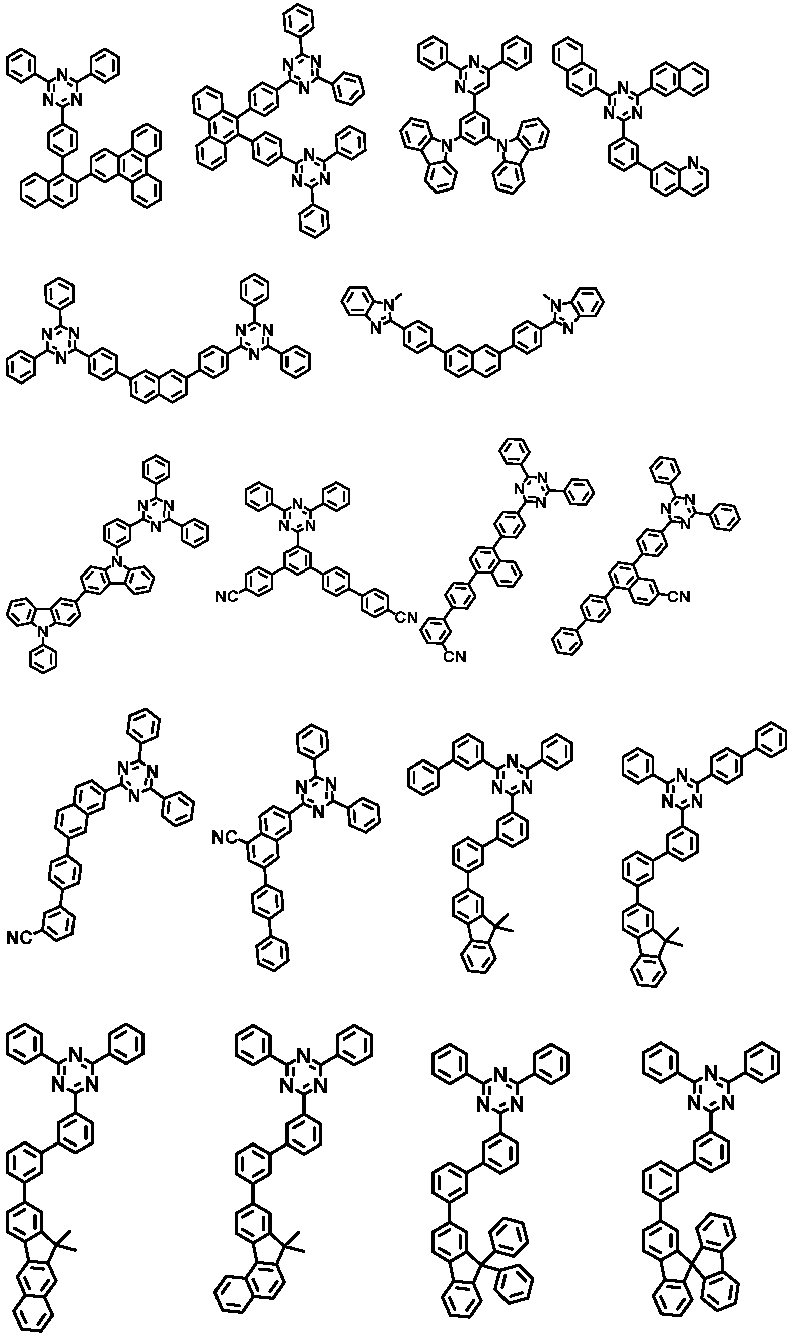

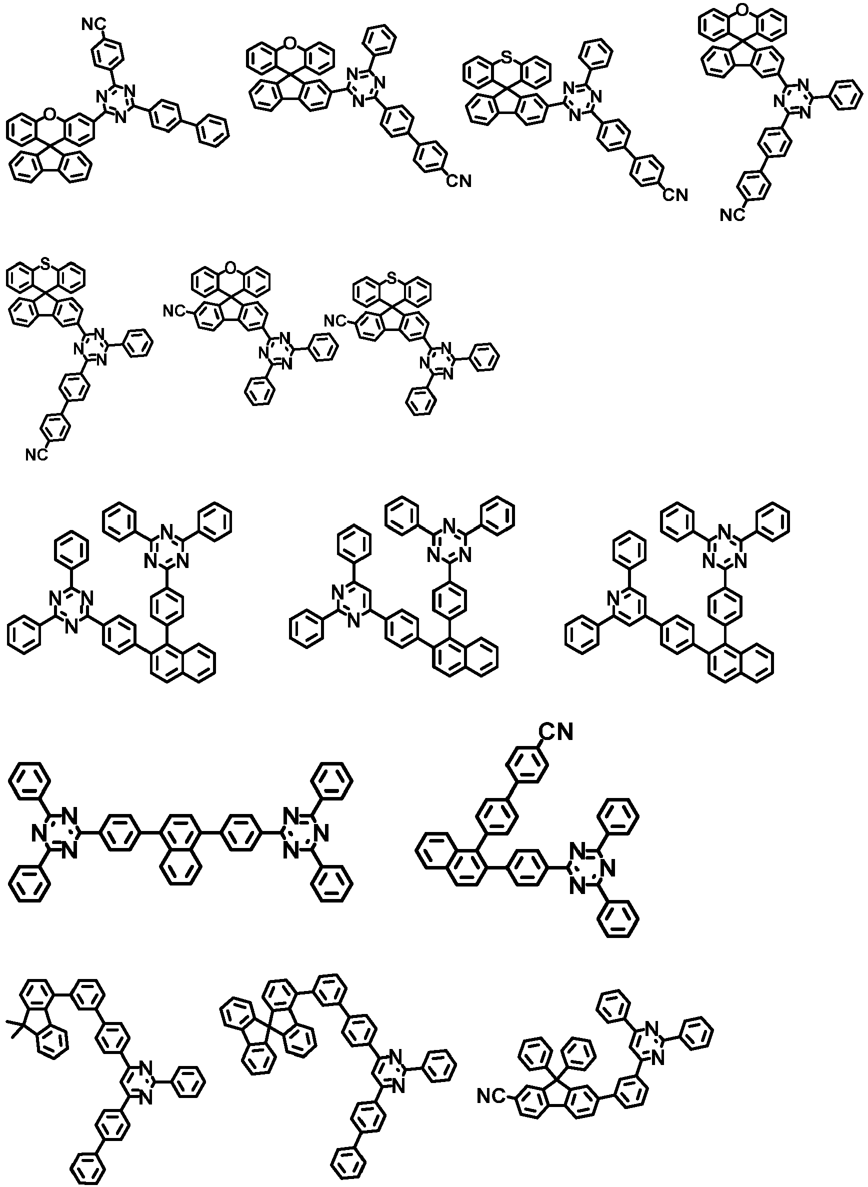

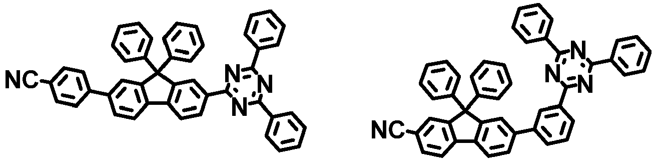

An embodiment may provide an organic light emitting diode including a light emitting layer as an organic layer.

Another embodiment may provide an organic light emitting diode including a light emitting layer and a hole transport region as an organic layer.

Another embodiment may provide an organic light emitting diode including a light emitting layer and an electron transport region as an organic layer.

As shown in FIG. 1, the organic light emitting diode according to an embodiment of the present invention may include a hole transport region 140 and an electron transport region 150 in addition to the light emitting layer 130 as the organic layer 105.

On the other hand, the organic light emitting diode may further include an electron injection layer (not shown), a hole injection layer (not shown), etc. in addition to the light emitting layer as the aforementioned organic layer.

The organic light emitting diode 100 may be produced by forming an anode or a cathode on a substrate, forming an organic layer using a dry film formation method such as a vacuum deposition method (evaporation), sputtering, plasma plating, and ion plating, and forming a cathode or an anode thereon.

The organic light emitting diode may be applied to an organic light emitting display device.

### DETAILED DESCRIPTION OF THE EMBODIMENTS

Hereinafter, the embodiments are illustrated in more detail with reference to examples. However, these examples are exemplary, and the scope of claims is not limited thereto.

Hereinafter, starting materials and reactants used in Examples and Synthesis Examples were purchased from Sigma-Aldrich Co. Ltd., TCI Inc., Tokyo chemical industry, or P&H tech as far as there is no particular comment or were synthesized by known methods.

### (Preparation of Compound for Organic Optoelectronic Device)

A compound presented as a more specific example of the compound of the present invention was synthesized through the following steps.

### Synthesis Example 1: Synthesis of Compound 1-38

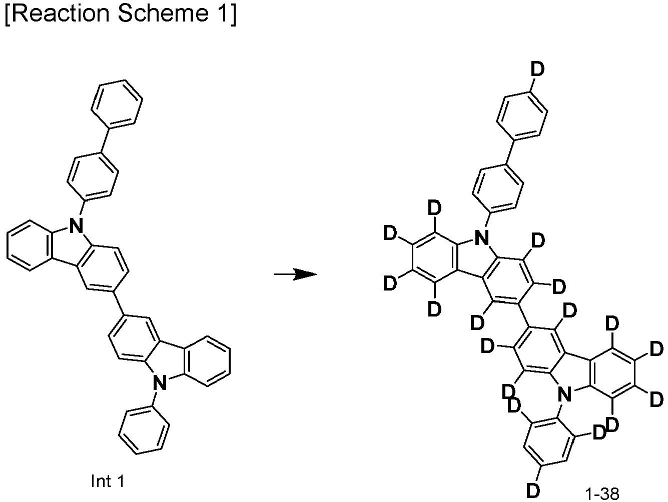

### 1st step: Synthesis of Compound Int 1

Compound Int 1 was synthesized by referring to the method disclosed in Korean Publication No. 2016-0049842.

### 2nd step: Synthesis of Compound 1-38

30 g (0.0535 mol) of Compound Int 1, 40 g (0.267 mol) of trifluoromethanesulfonic acid, and 282 g (3.35 mol) of D₆-benzene were stirred at 10 °C for 24 hours. Subsequently, purified water was added thereto and then, neutralized with a saturated K₃PO₄ solution. An organic layer therefrom was concentrated and column-purified, obtaining 18 g of Compound 1-38 (white solid, LC-Mass Mz 578.79, C₄₂H₁₀D₁₈N₂).

### Synthesis Example 2: Synthesis of Compound 1-98

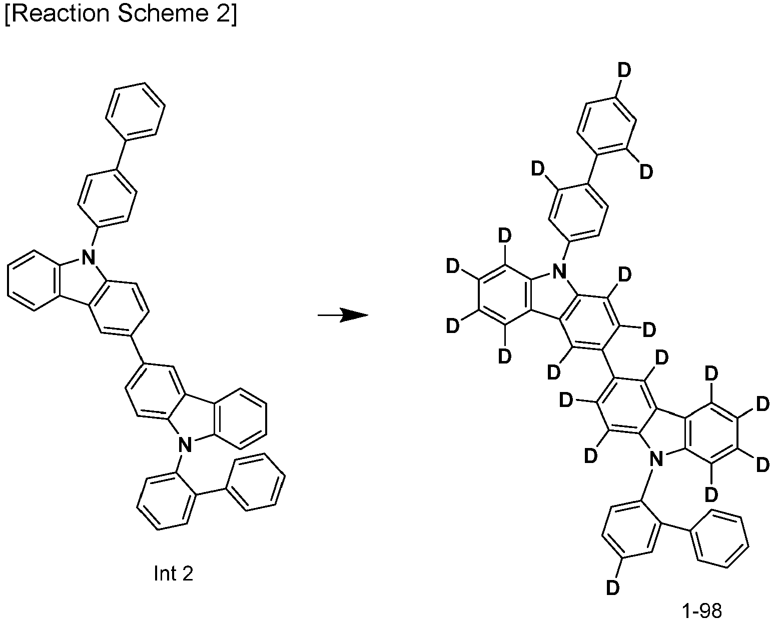

19 g of Compound 1-98 (white solid, LC-mass Mz 655.29, C₄₈H₁₄D₁₈N₂) was obtained in the same synthesis manner as in Synthesis Example 1 except that 30 g (0.047 mol) of Int 2 was used instead of Int 1.

### Synthesis Example 3: Synthesis of Compound 1-110

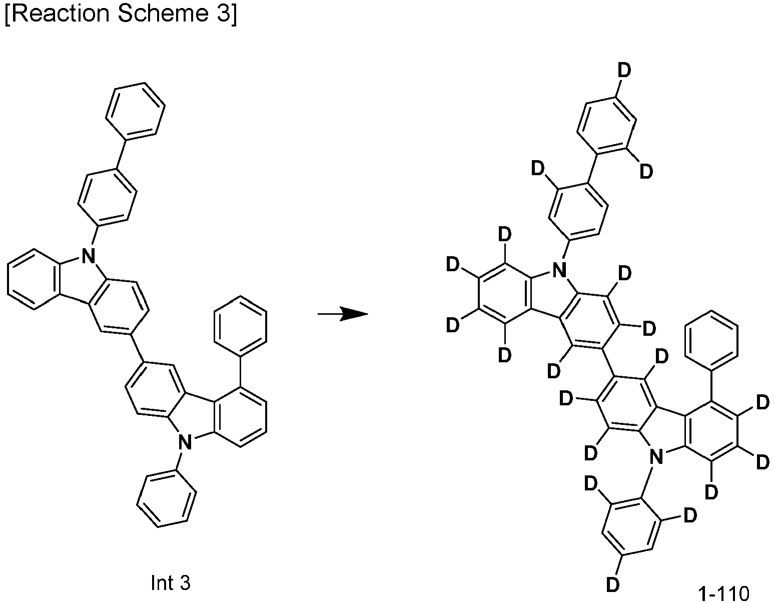

15 g of Compound 1-110 (white solid, LC-mass Mz 657.31, C₄₈H₁₃D₁₉N₂) was obtained in the same synthesis manner as in Synthesis Example 1 except that 20 g (0.036 mol) of Int 3 was used instead of Int 1

### Comparative Synthesis Example 1: Synthesis of Compound Y1

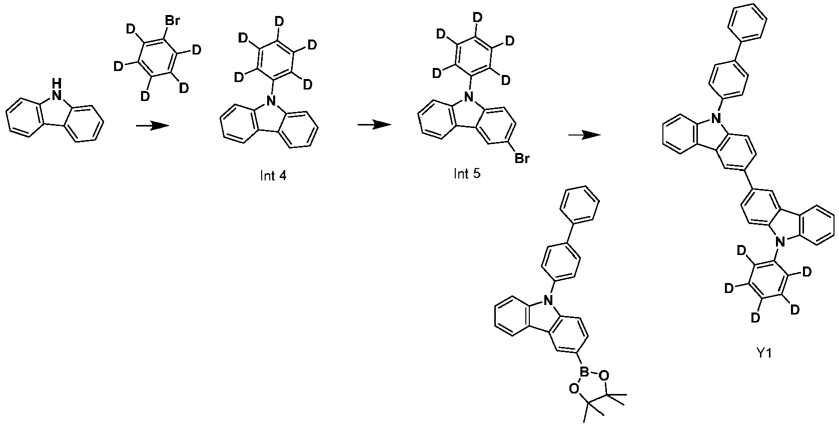

47 g (0.281 mol) of carbazole, 50 g (0.310 mol) of bromobenzene-Ds, 53 g (0.028 mol) of Cul, 58 g (0.42 mol) of K₂CO₃, 5 g (0.028 mol) of 1,10-phenanthroline, and 560 ml of DMF were put and stirred under reflux. When a reaction was completed, the resultant was cooled to room temperature, and purified water was added thereto to form a solid. The solid was column-purified, obtaining 62 g of Int 4 (molecular weight: 248.33).

62 g (0.25 mol) of Int 4 was added to DMF and dissolved therein. 45 g (0.25 mol) of NBS was slowly added thereto at 0 °C and then, stirred at room temperature, completing a reaction. Subsequently, purified water was added to the reaction solution to produce a crystal, and the solid was column-purified, obtaining 80 g of Int 5 (molecular weight: 327.23).

80 g (0.245 mol) of Int 5, 120 g (0.27 mol) of 4-biphenyl-carbazole-3-boronic ester, 68 g (0.49 mol) of K₂CO₃, 14g (0.0122mol) of Pd(PPh₃)₄, 320 ml of purified water, and 490 ml of THF were put and stirred under reflux. When a reaction was completed, purified water was added thereto for extraction, and an organic layer therefrom was concentrated. The mixture was column-purified, obtaining 90 g of Compound Y1 (molecular weight: 565.72).

### Comparative Synthesis Example 2: Synthesis of Compound Y2

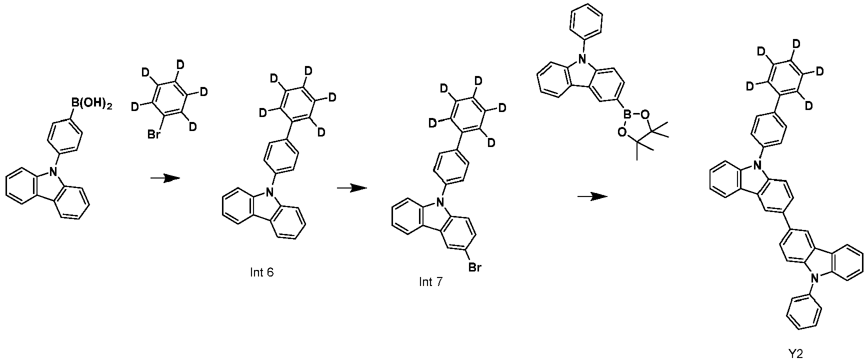

35 g (0.095 mol) of (phenyl-4-boronic acid)-9H-carbazole, 17 g (0.105 mol) of bromobenzene-Ds, 3.3 g (0.0028 mol) of Pd(PPh₃)₄, 32.7 g (0.237 mol) of K₂CO₃, 120 ml of purified water, and 320 ml of THF were put and stirred under reflux. When a reaction was completed, the resultant was cooled, purified water was added thereto, and an organic layer separated therefrom was concentrated. The concentrate was column-purified, obtaining 25 g of Int 6 (molecular weight: 324.43).

20 g (0.062 mol) of Int 6 was dissolved in 200 ml of DMF, and 11.5 g (0.065 mol) of NBS was slowly added thereto at 0 °C. The obtained mixture was stirred at room temperature to complete a reaction, and purified water was added thereto to produce a solid. The solid was column-purified, obtaining 23 g of Int 7 (molecular weight 403.33).

20 g (0.0496 mol) of Int 7, 22 g (0.06 mol) of phenyl-9H-carbazole-3-boronic ester, 1.72 g (0.0015 mol) of Pd(PPh₃)₄, 13.7 g (0.099 mol) of K₂CO₃, 50 ml of purified water, and 165 ml of THF were put and stirred under reflux. When a reaction was completed, the resultant was cooled, purified water was added thereto, and an organic layer was separated therefrom and concentrated. The concentrate was column-purified, obtaining 11.5 g of Compound Y2 (molecular weight: 565.72).

### Comparative Synthesis Example 3: Synthesis of Compound Y3

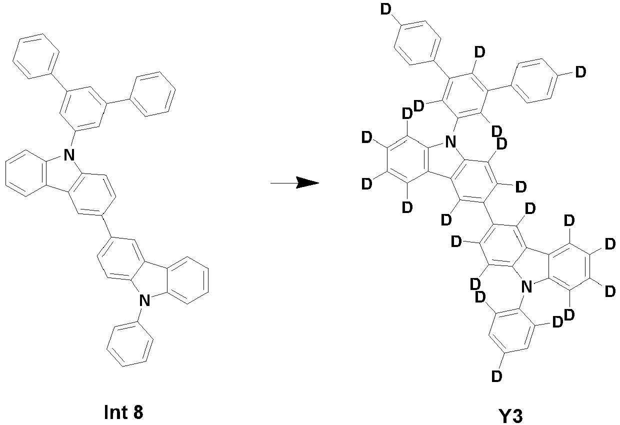

5 g of Compound Y3 (white solid, LC-mass Mz 659.78, C₄₈H₁₀D₂₂N₂) was obtained in the same synthesis manner as in Synthesis Example 1 except that 20 g (0.036 mol) of Int 8 was used instead of Int 1.

### (Manufacture of Organic Light Emitting Diode)

### Example 1

A glass substrate coated with ITO (Indium tin oxide) was washed with distilled water and ultrasonic waves. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This obtained ITO transparent electrode was used as an anode, Compound A doped with 1% NDP-9 (available from Novaled) was vacuum-deposited on the ITO substrate to form a 100 Å-thick hole injection layer, and Compound A was deposited to on the hole injection layer to form a 1350 Å-thick hole transport layer. On the hole transport layer, 350 Å-thick hole transport auxiliary layer was formed by depositing Compound B. On the hole transport auxiliary layer, 400 Å-thick light emitting layer was formed by vacuum-depositing Compound 1-38 obtained in Synthesis Example 1 as a host and doping 7 wt% of PhGD as a dopant. Subsequently, Compound C was deposited on the light emitting layer to form a 50 Å-thick electron transport auxiliary layer, and Compound D and Liq were simultaneously vacuum-deposited at a weight ratio of 1:1 to form a 300 Å-thick electron transport layer. LiQ (15 Å) and Al (1200 Å) were sequentially vacuum-deposited on the electron transport layer to form a cathode, thereby manufacturing an organic light emitting diode.

ITO / Compound A (1% NDP-9 doping,100 Å) / Compound A (1350 Å) / Compound B (350 Å) / EML [93 wt% of host (Compound 1-38) : 7 wt% of PhGD] (400 Å) / Compound C (50 Å) / Compound D:LiQ (300 Å) / LiQ (15 Å) / Al (1200 Å).
Compound A: N-(biphenyl-4-yl)-9,9-dimethyl-N-(4-(9-phenyl-9H-carbazol-3-yl)phenyl)-9H-fluoren-2-amine
Compound B: N,N-bis(9,9-dimethyl-9H-fluoren-4-yl)-9,9-spirobi(fluorene)-2-amine
Compound C: 2-[3'-(9,9-dimethyl-9H-fluoren-2-yl)[1,1'-biphenyl]-3-yl]-4,6-diphenyl-1,3,5-triazine
Compound D: 2-[4-[4-(4'-cyano-1,1'-biphenyl-4-yl)-1-naphthyl]phenyl]-4,6-diphenyl-1,3,5-triazine

### [PhGD]

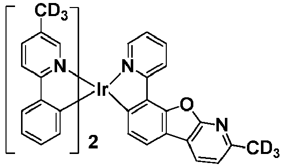

### Comparative Examples 1 to 3

Diodes according to Comparative Examples1 to 3 were manufactured in the same manner as in Example 1, except that the hosts were changed as shown in Tables 1amd 2.

### Evaluations

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

Luminous efficiency (cd/A) of the same current density (10 mA/cm²) was calculated using the luminance and current density measured from (1) and (2) above.

Relative values based on the luminous efficiency of Comparative Example 3 were shown in Table 2.

### (4) Measurement of Life-span

While luminance (cd/m²) was maintained at 6,000 cd/m², time taken until luminous efficiency (cd/A) was reduced to 97% was measured.

Relative values based on the T95 life-span of Comparative Example 1 were shown in Table 1.

### (5) Measurement of Driving Voltage

Driving voltages of the organic light emitting diodes at 15 mA/cm² were measured by using a current-voltage meter (Keithley 2400).

Relative values based on the driving voltage of Comparative Example 3 are shown in Table 2.

**(Table 1)**

| | Host | Life-span T97 |
|---|---|---|
| | | (%) |
| Example 1 | Compound 1-38 | 117.6 |
| Comparative Example 1 | Compound Y1 | 100 |
| Comparative Example 2 | Compound Y2 | 92.6 |

**(Table 2)**

| | Host | Driving voltage | Luminous efficiency |
|---|---|---|---|
| | | (%) | (%) |
| Example 1 | Compound 1-38 | 97.2 | 109.8 |
| Comparative Example 3 | Compound Y3 | 100 | 100 |

Referring to Tables 1 and 2, the driving voltage, efficiency, and life-span characteristics of the organic light emitting diodes according to examples of the present invention are significantly improved compared to the organic light emitting diodes according to the comparative examples.

While this invention has been described in connection with what is presently considered to be practical example embodiments, it is to be understood that the invention is not limited to the disclosed embodiments. On the contrary, it is intended to cover various modifications and equivalent arrangements included within the spirit and scope of the appended claims.

## Claims

1. A compound for an organic optoelectronic device represented by Chemical Formula 1: 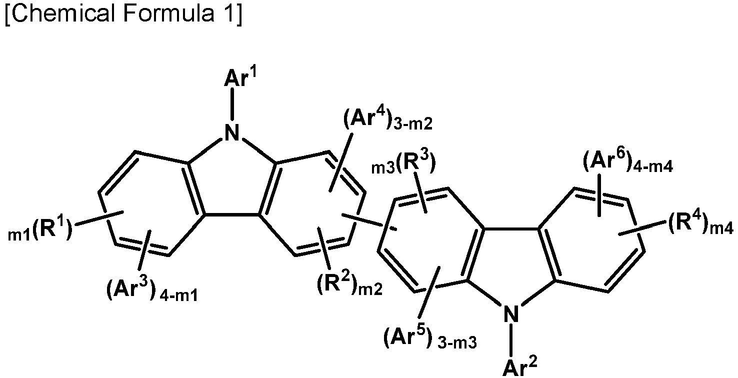
wherein, in Chemical Formula 1,
R¹ to R⁴ are each independently hydrogen, deuterium, or a substituted or unsubstituted C6 to C30 aryl group,
Ar³ to Ar⁶ are each independently hydrogen, or a substituted or unsubstituted C6 to C30 aryl group,
m1 and m4 are each independently one of integers of 1 to 4,
m2 and m3 are each independently one of integers of 1 to 3,
at least one of R¹ to R⁴ is deuterium,
Ar¹ and Ar² are each independently one selected from the substituents listed in Group I and Group II, and
at least one of Ar¹ and Ar² is one selected from the substituents listed in Group II ; 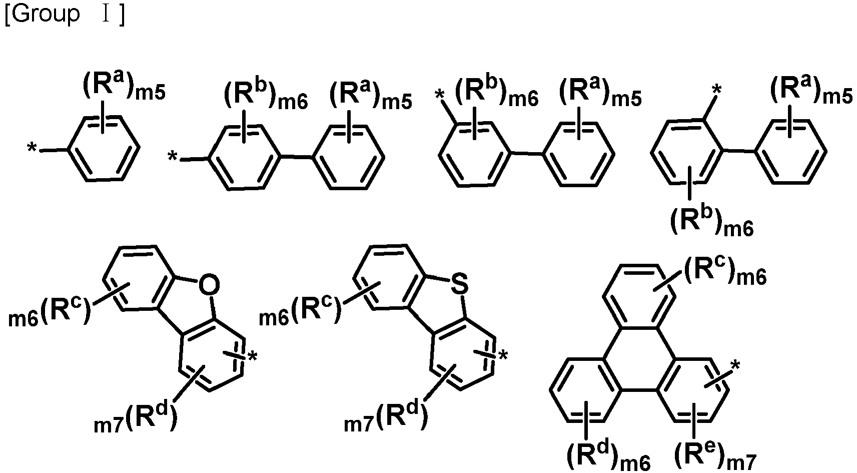 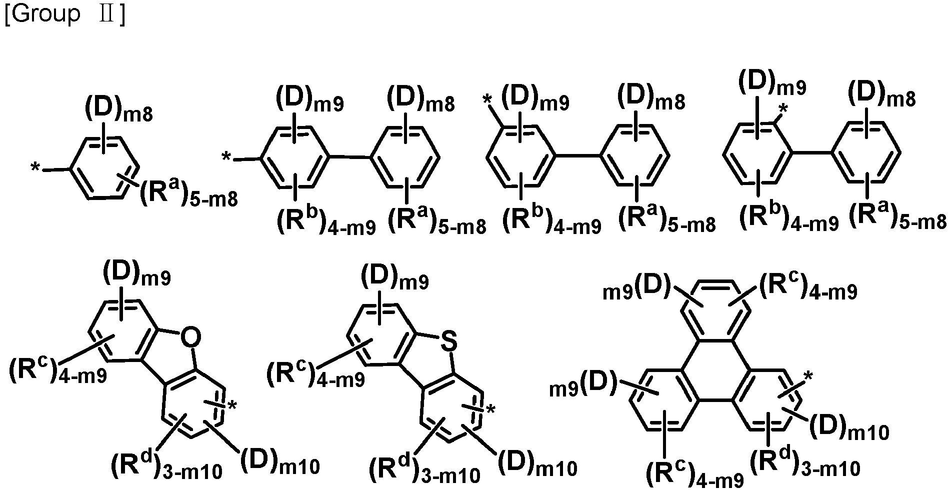
wherein, in Group I and Group II,
R^{a} and R^{b} are each independently hydrogen, deuterium, a cyano group, or a substituted or unsubstituted C1 to C10 alkyl group,
R^{c} to R^{e} are each independently hydrogen, deuterium, a cyano group, a substituted or unsubstituted C1 to C10 alkyl group, or a substituted or unsubstituted C6 to C20 aryl group,
D is deuterium,
m5 and m8 are each independently one of integers of 1 to 5,
m6 and m9 are each independently one of integers of 1 to 4, and
m7 and m10 are each independently one of integers of 1 to 3.

2. The compound for an organic optoelectronic device of claim 1, wherein
Chemical Formula 1 is represented by any one of Chemical Formula 1-1 to Chemical Formula 1-10: 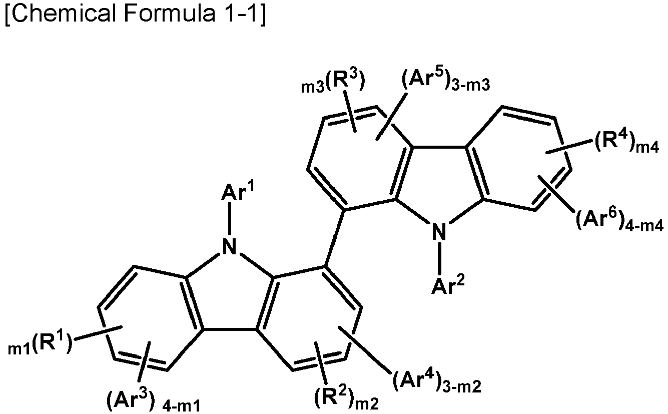 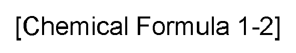 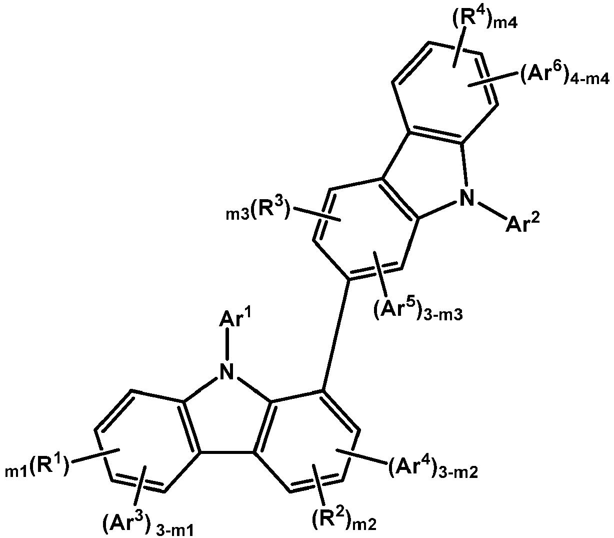 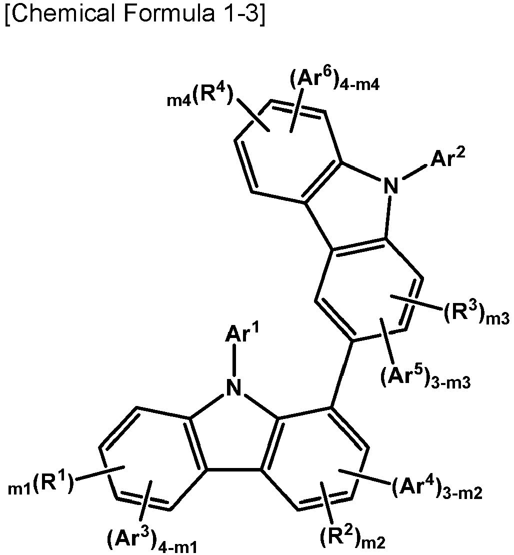 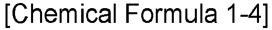 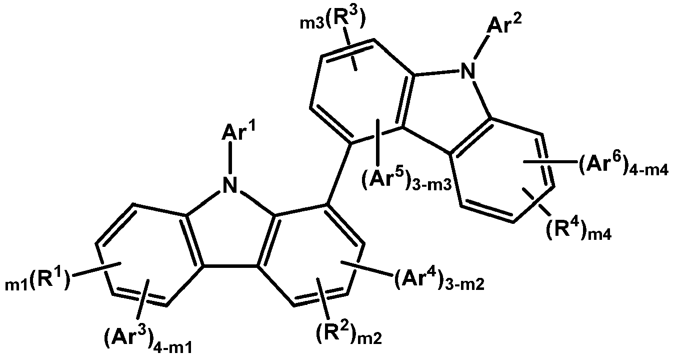 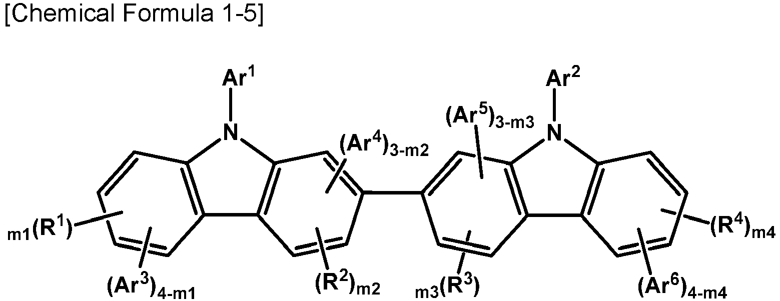 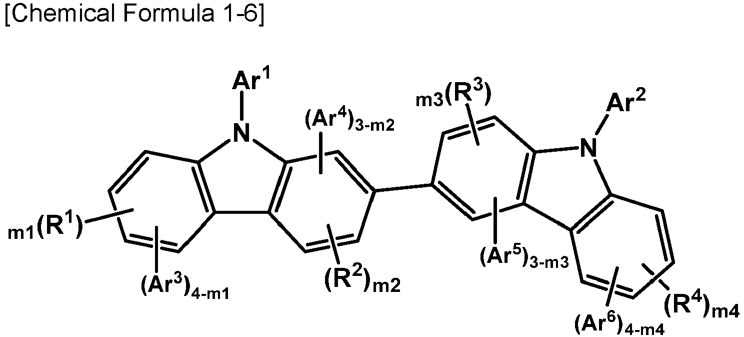 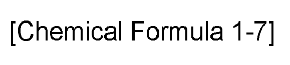 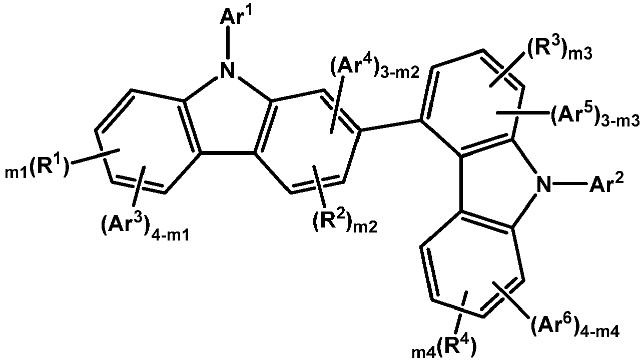 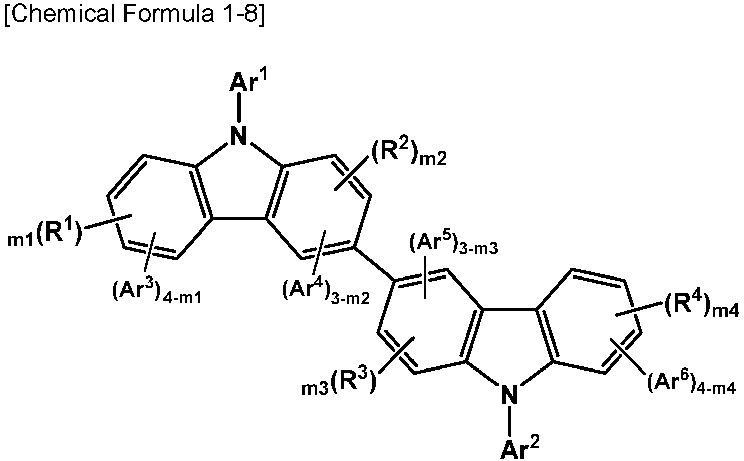 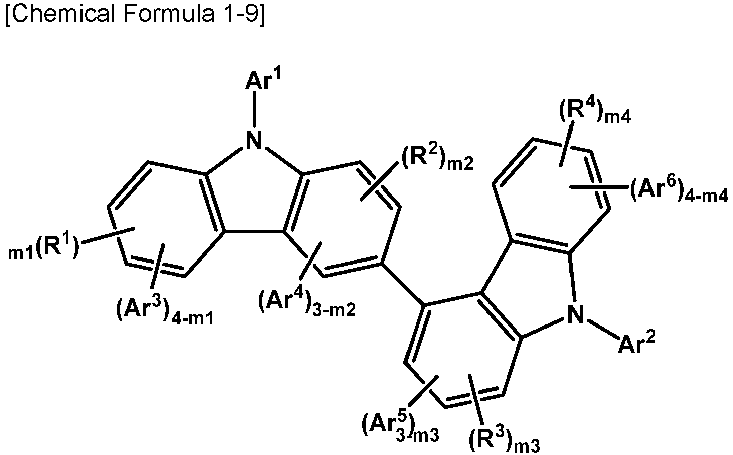 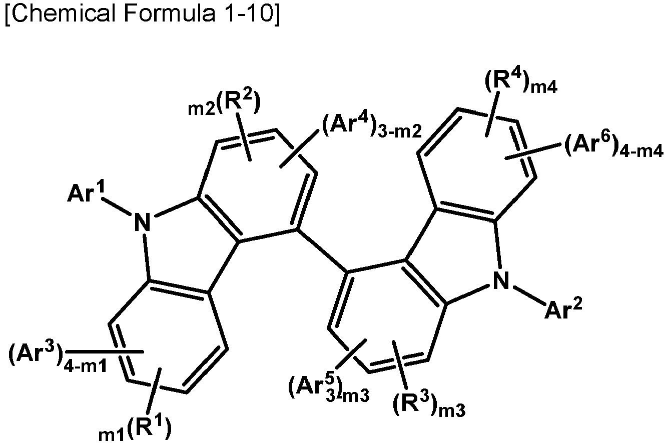
wherein, in Chemical Formula 1-1 to Chemical Formula 1-10,
definitions of Ar¹ to Ar⁶, R¹ to R⁴ and m¹ to m⁴ are the same as defined in claim 1.

3. The compound for an organic optoelectronic device of claim 1, wherein
Ar¹ and Ar² in Chemical Formula 1 are each independently one selected from the substituents listed in Group I -1 and Group II-1, and
at least one of Ar¹ and Ar² is one selected from the substituents listed in Group II-1. [Group I -1] 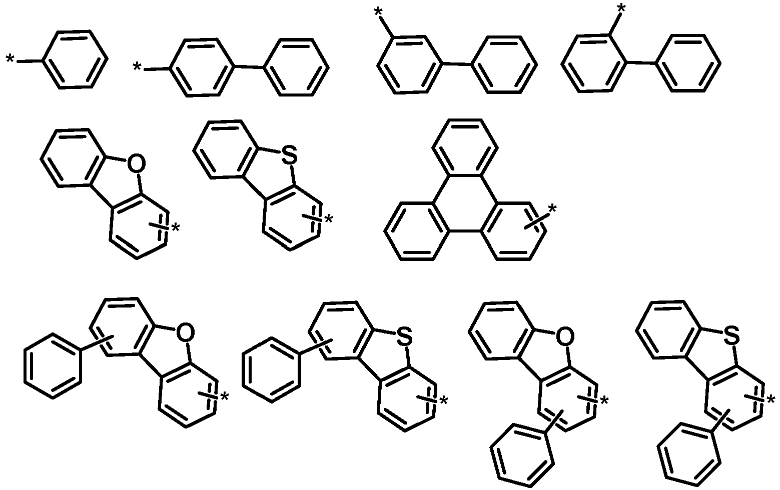 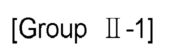 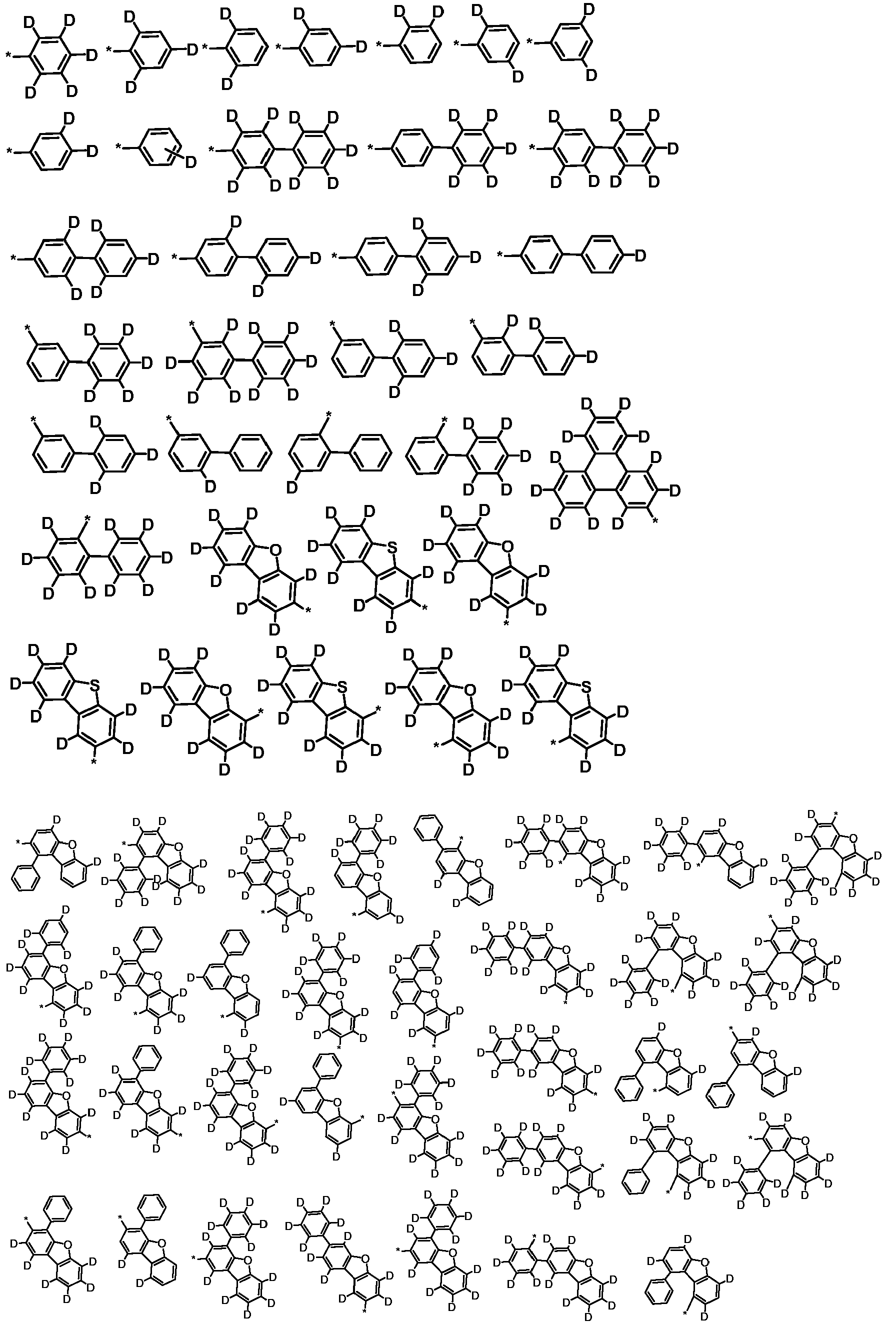 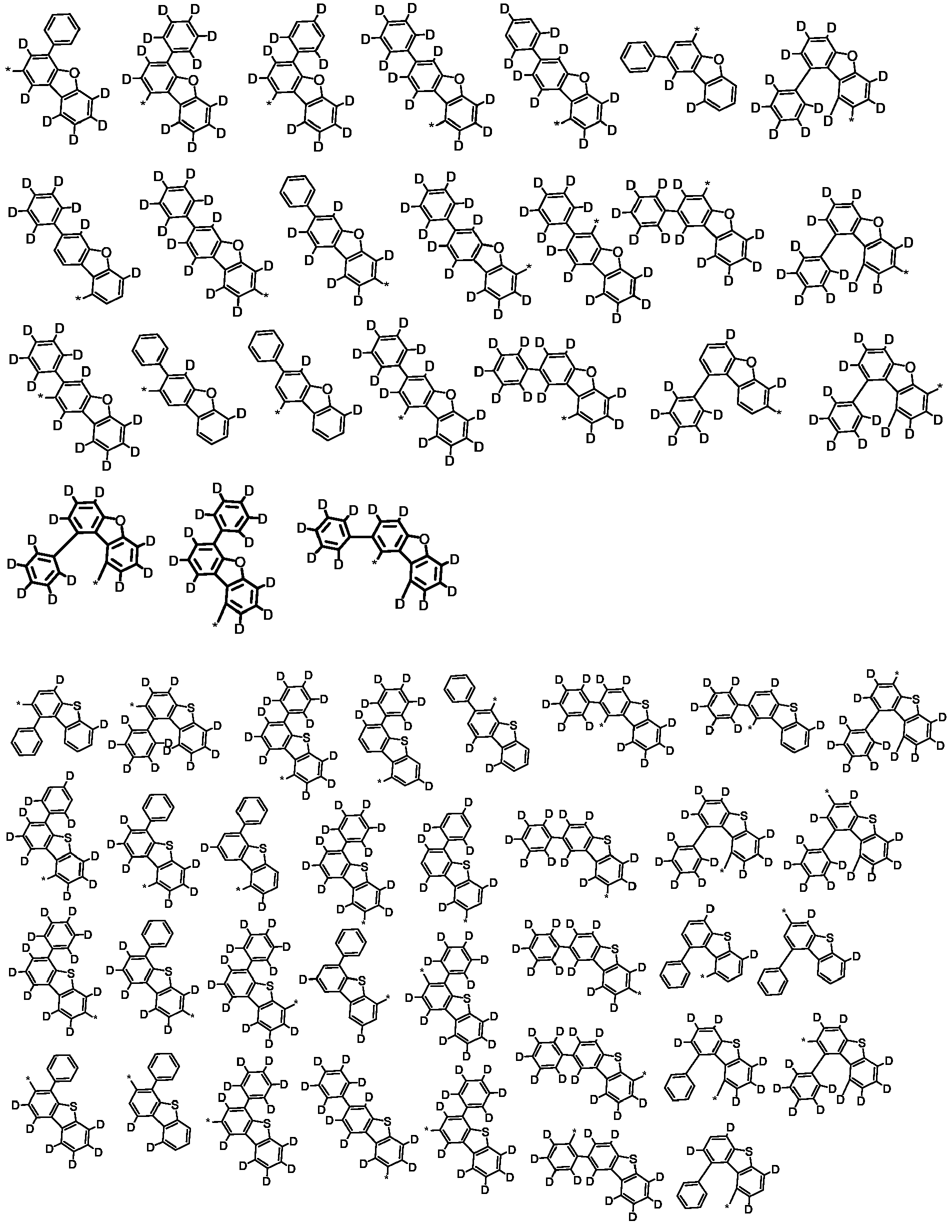 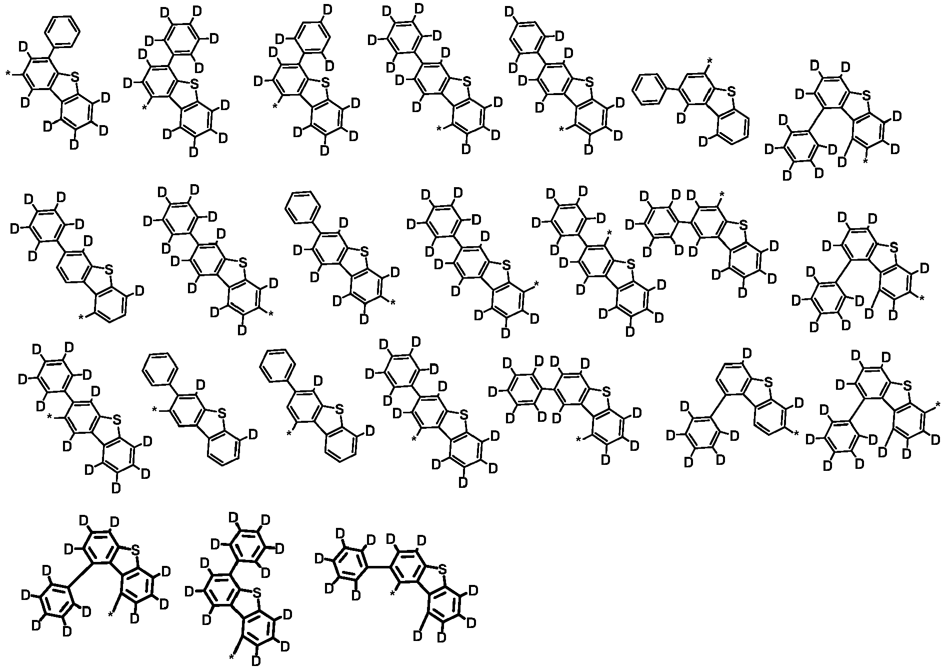
wherein, in Group I -1 and Group II -1, * is a linking point.

4. The compound for an organic optoelectronic device of claim 1, wherein
Chemical Formula 1 is represented by Chemical Formula 1-8a or Chemical Formula 1-8e: 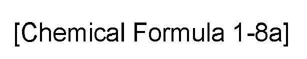 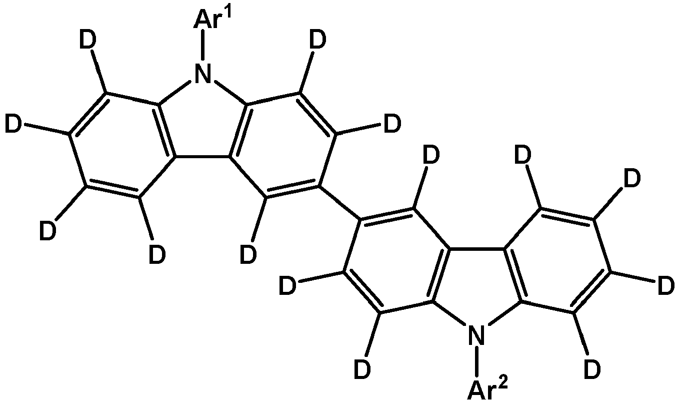 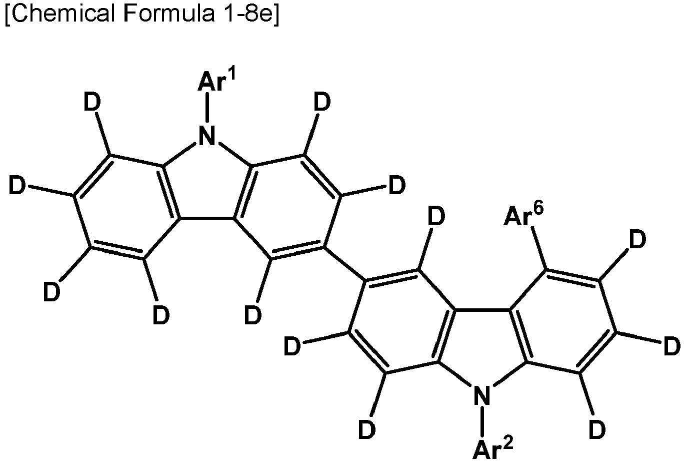
wherein, in Chemical Formula 1-8a and Chemical Formula 1-8e,
Ar¹ and Ar² are the same as defined in claim 1, and
Ar⁶ is a C6 to C30 aryl group that is substituted or unsubstituted with deuterium.

5. The compound for an organic optoelectronic device of claim 1, wherein the compound is one selected form compounds listed in Group 1:

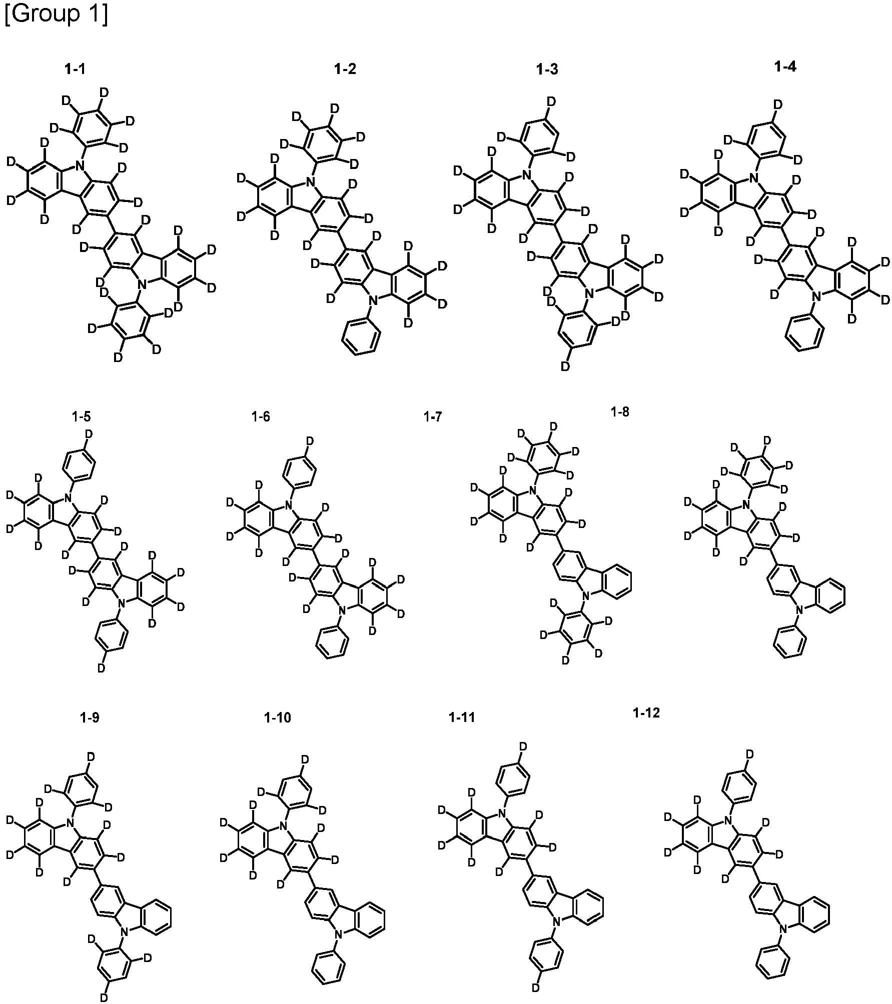

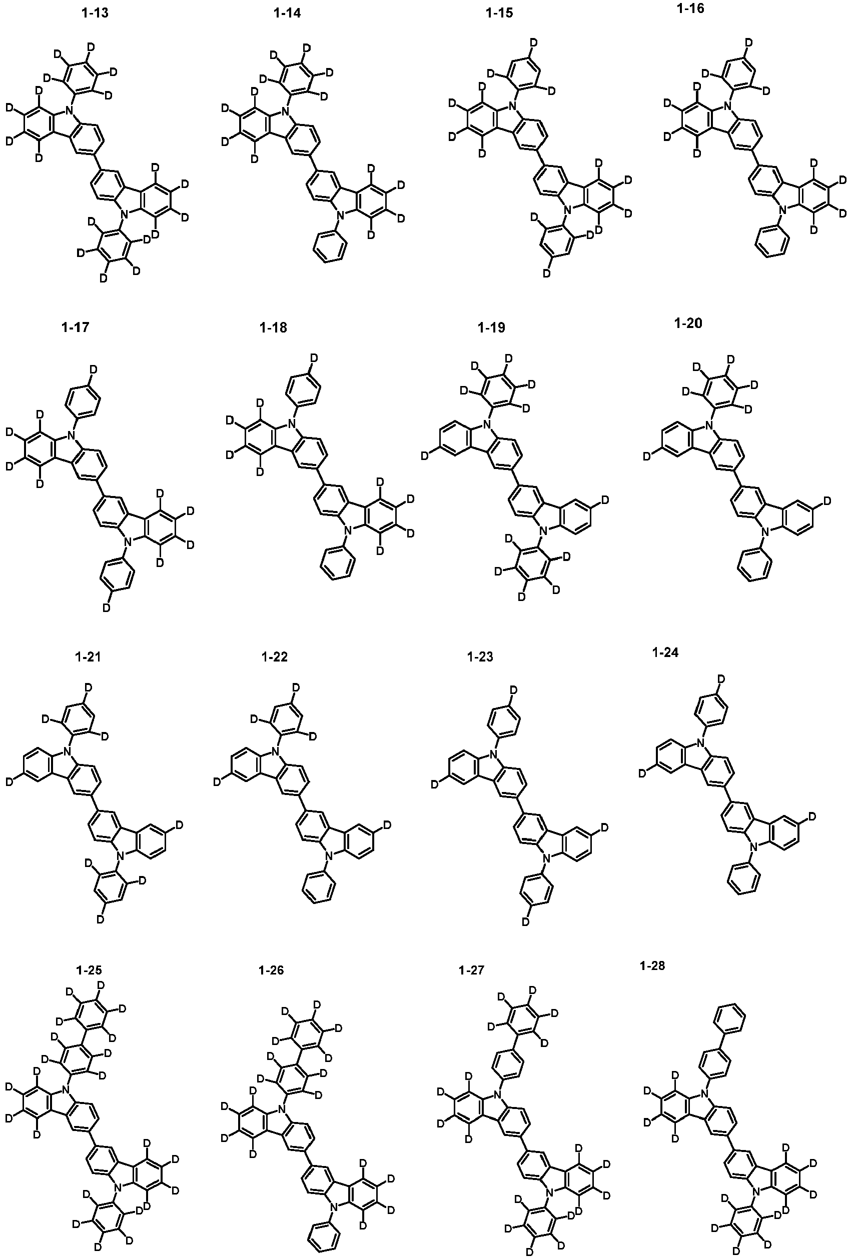

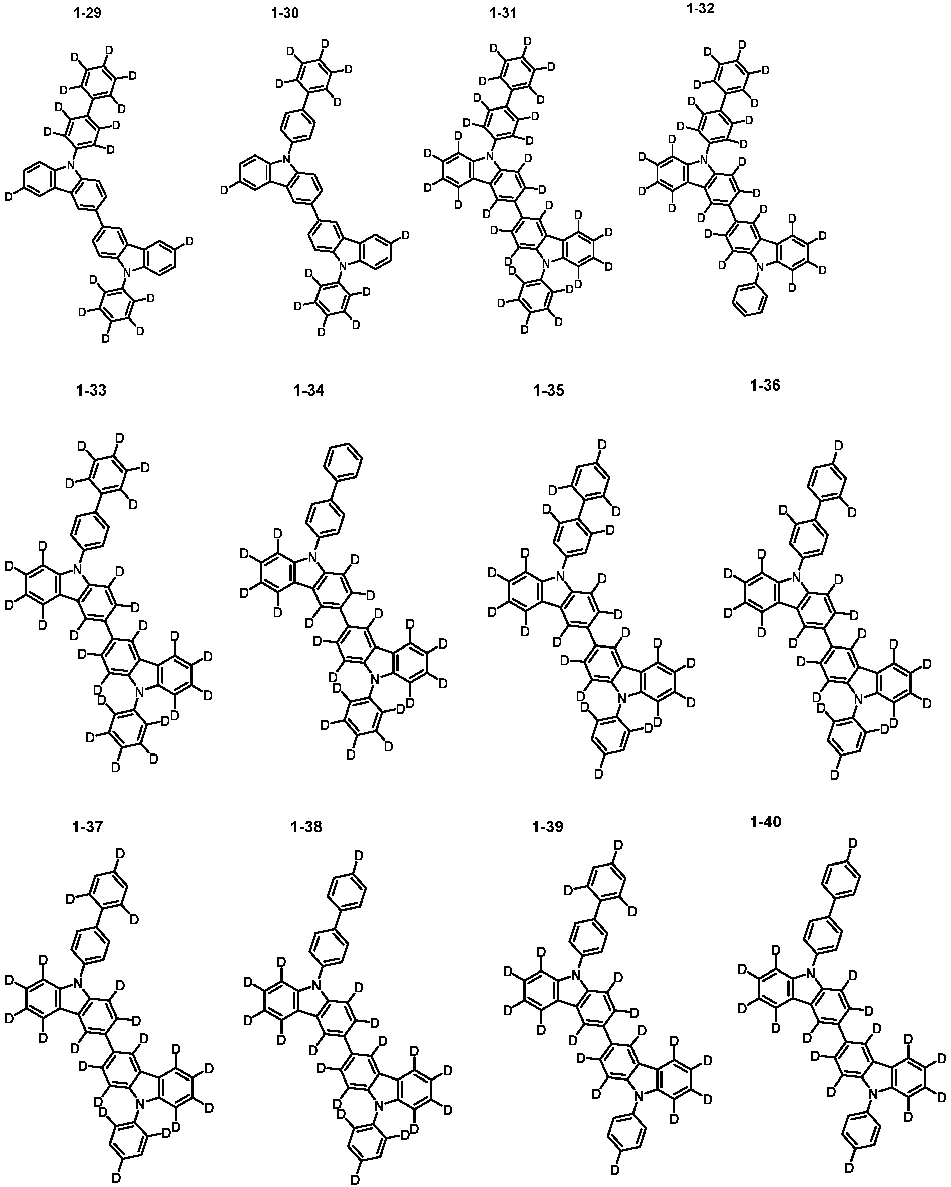

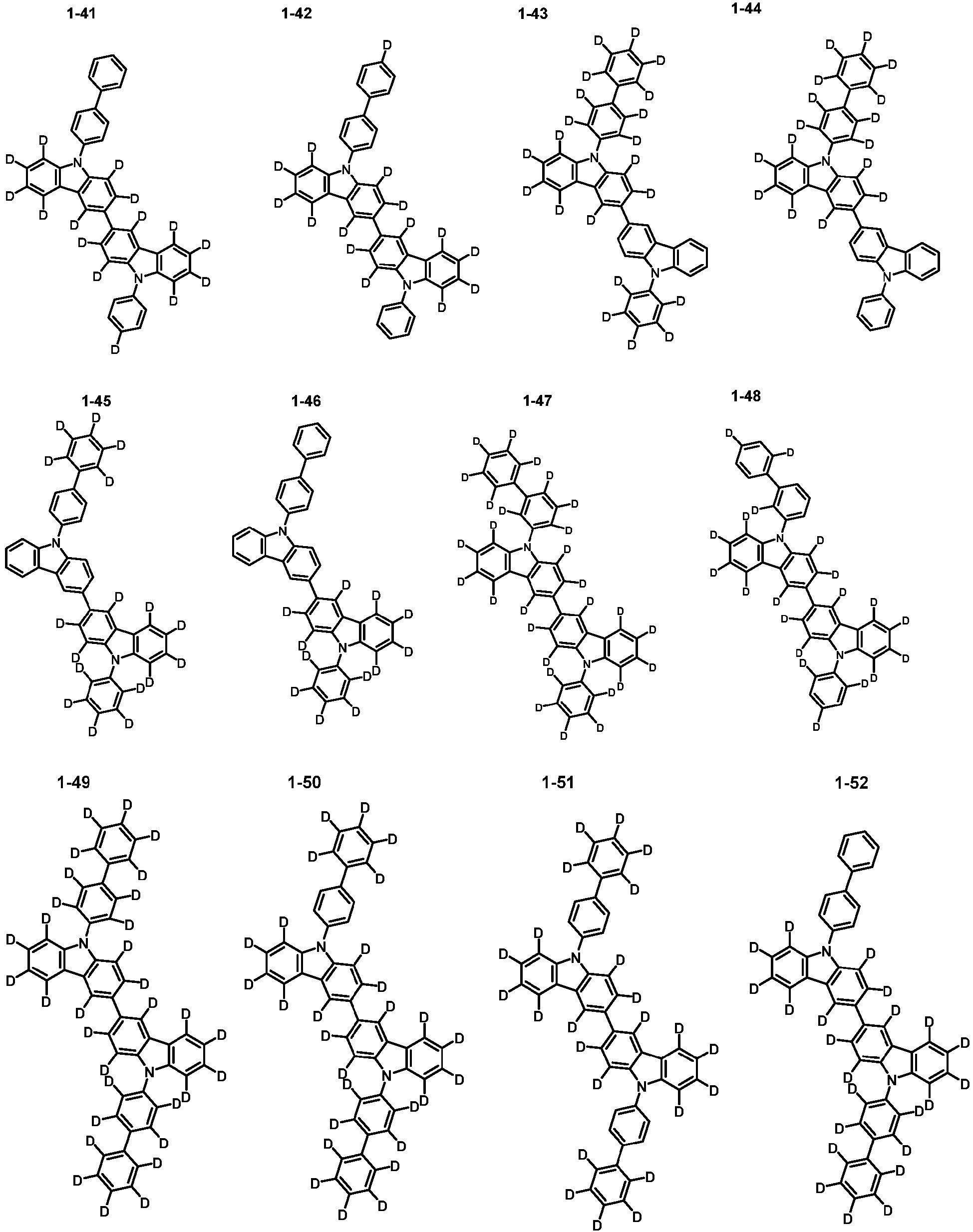

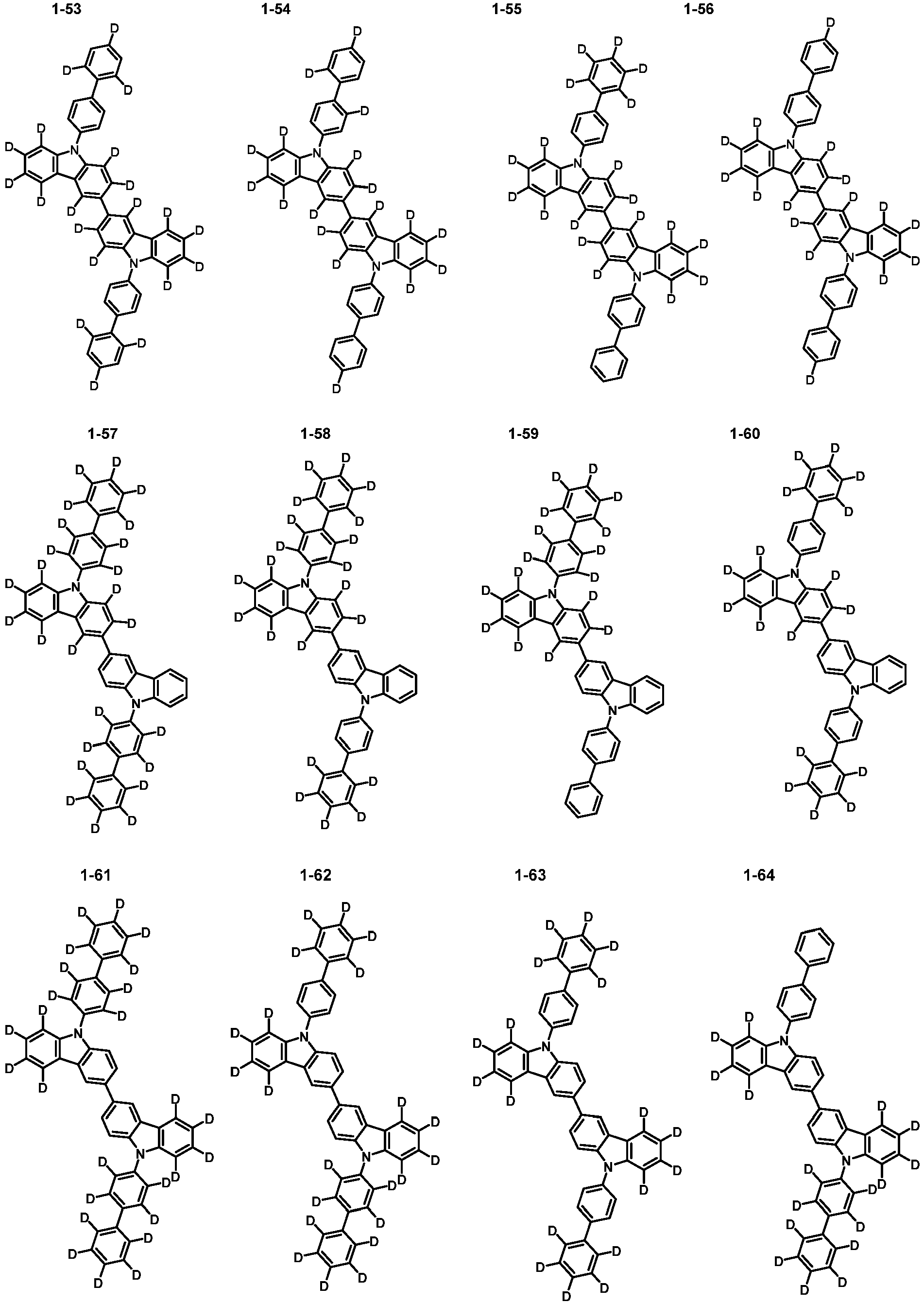

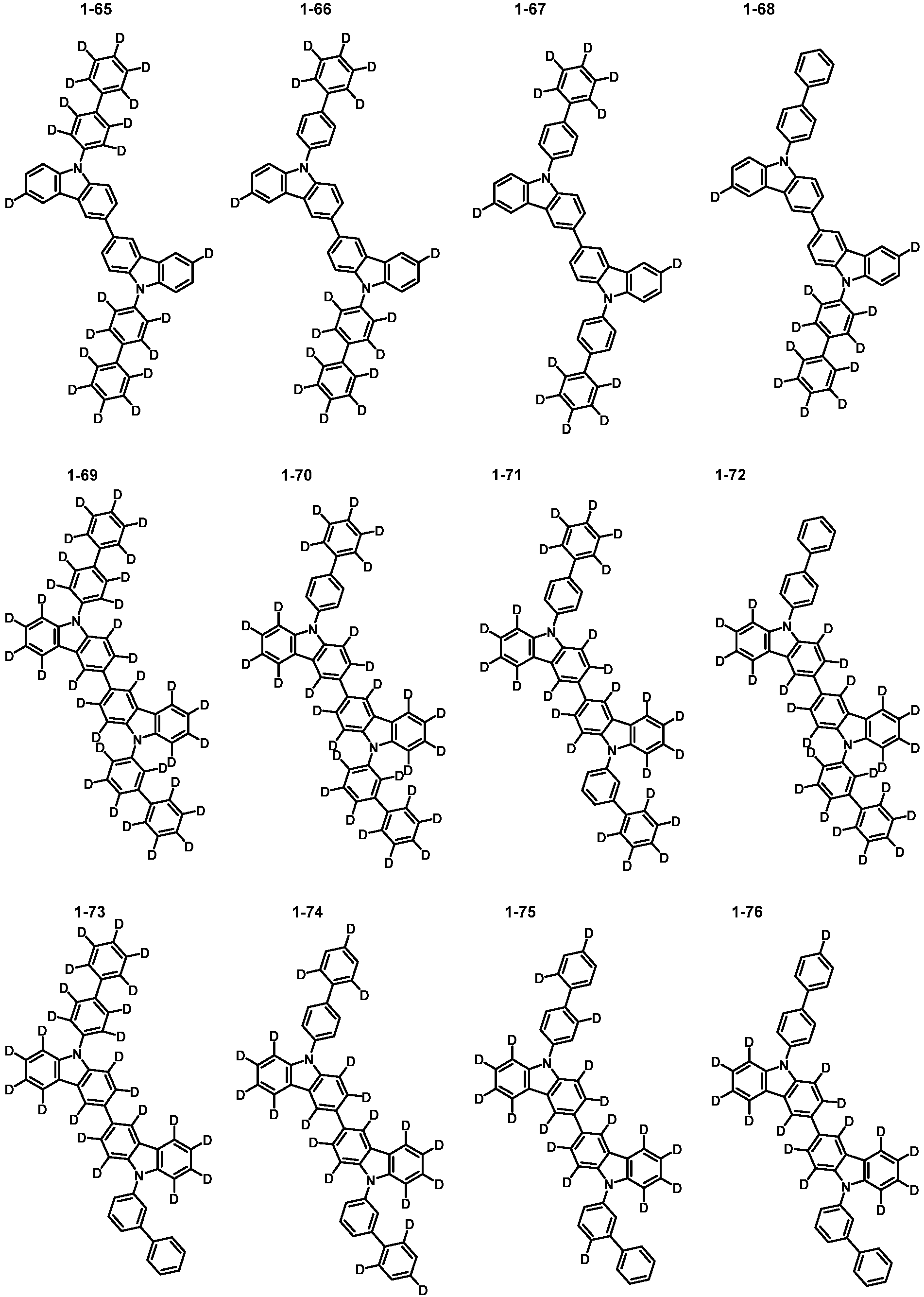

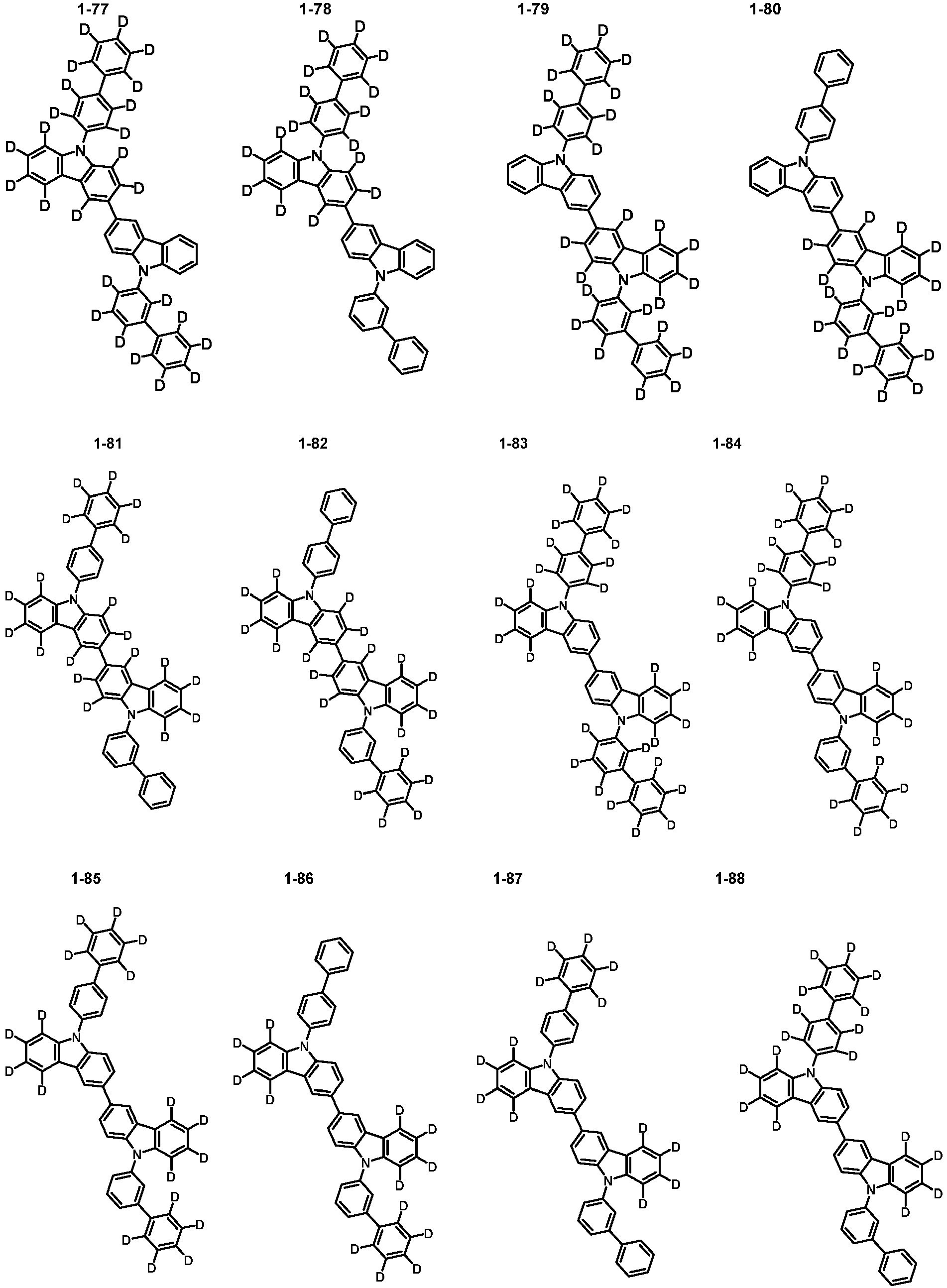

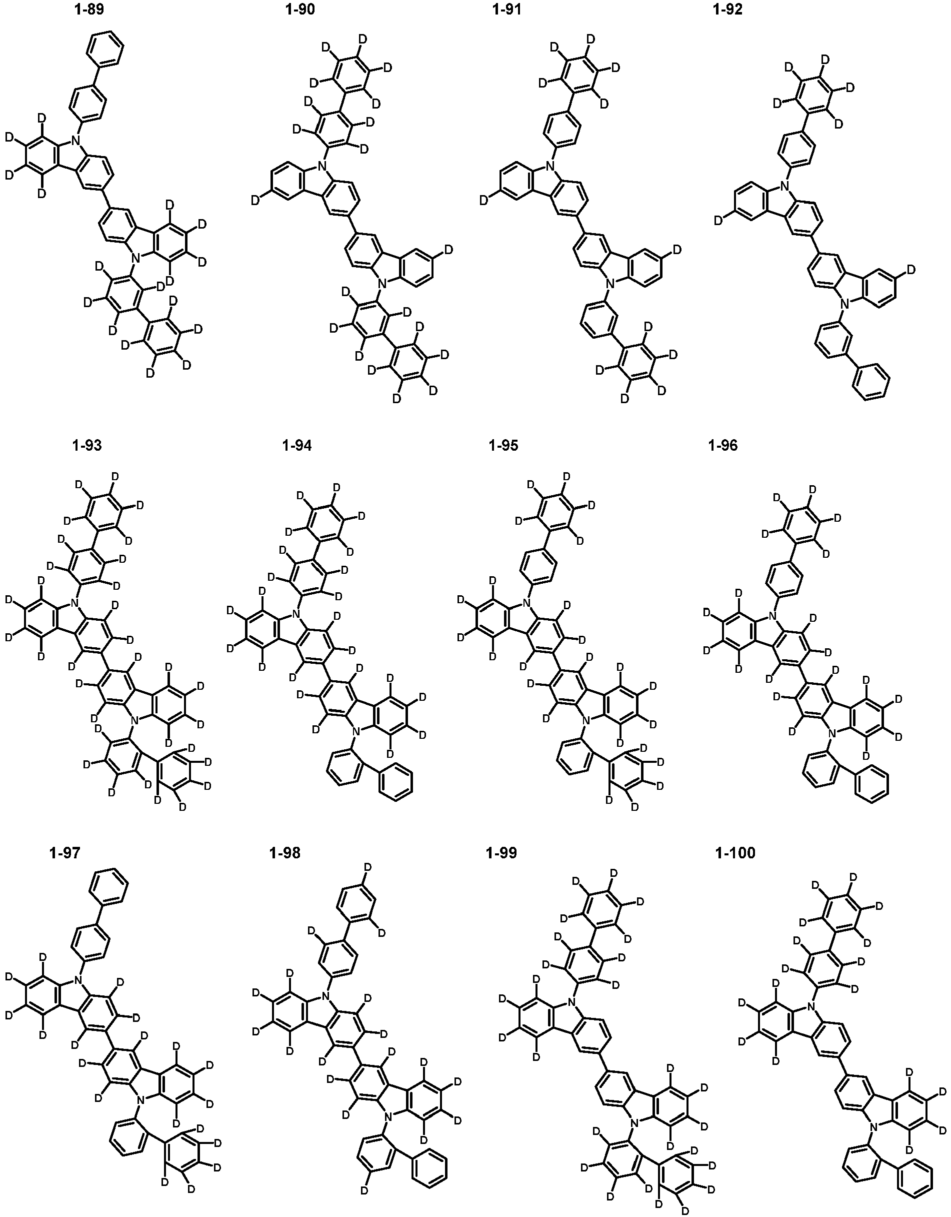

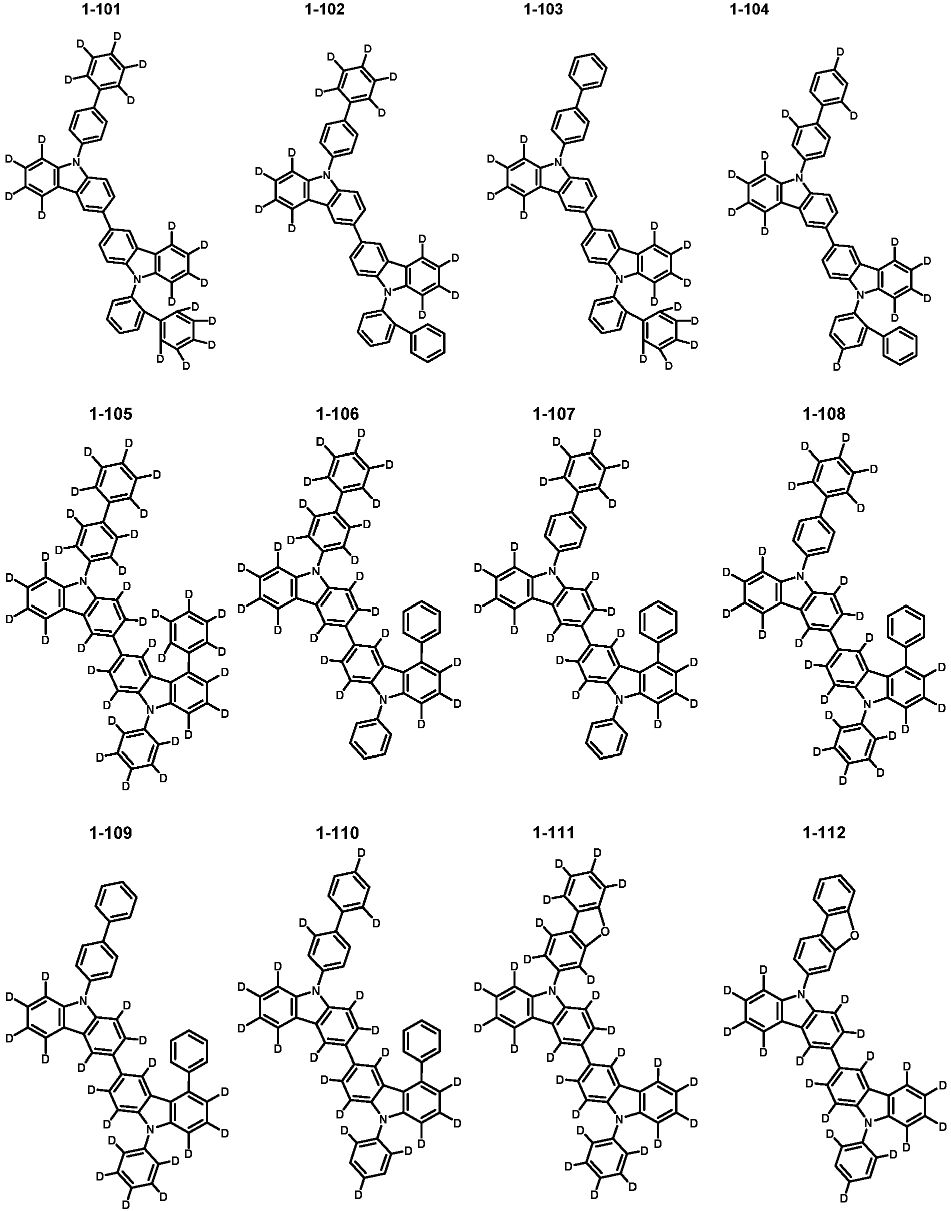

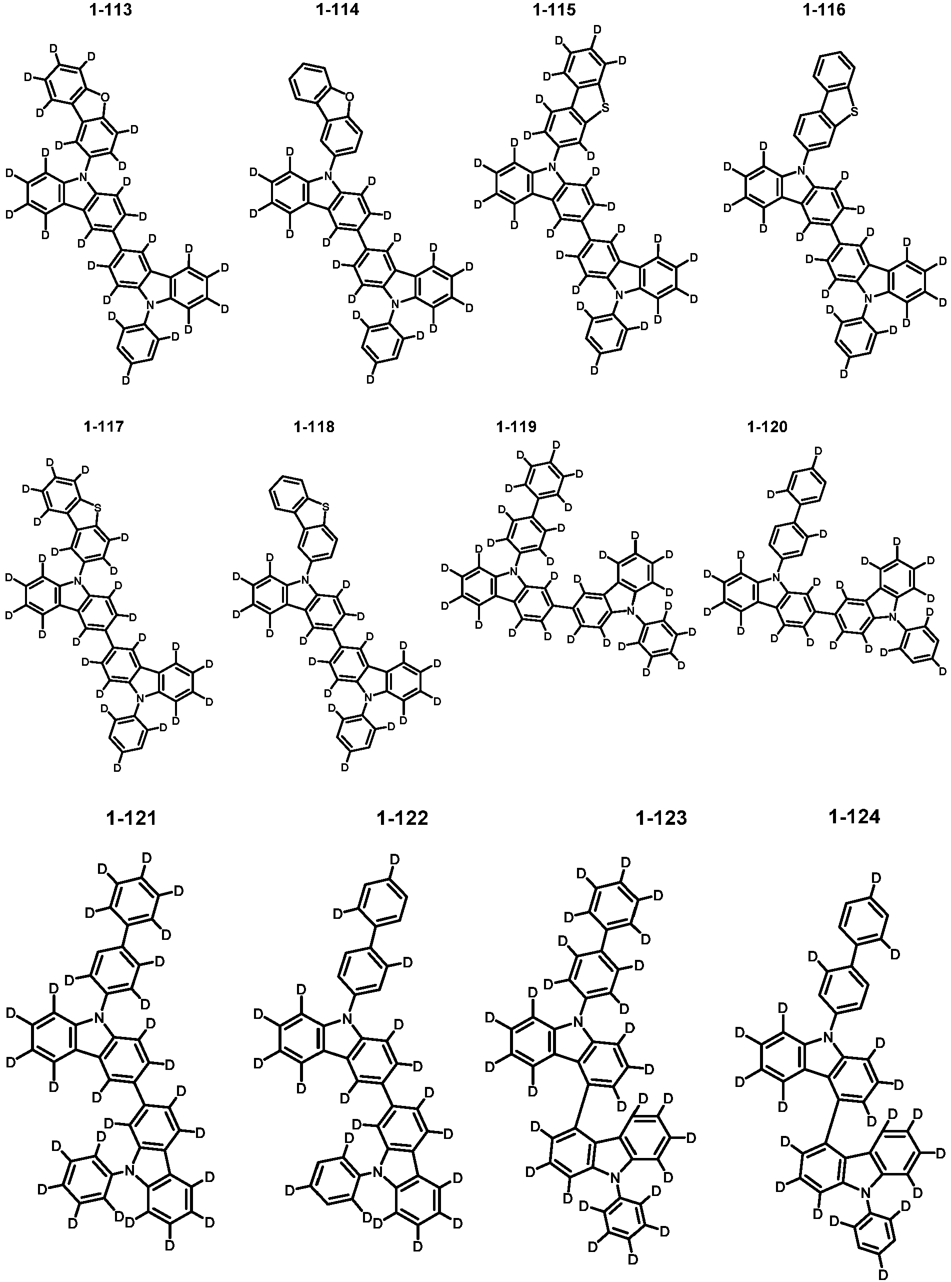

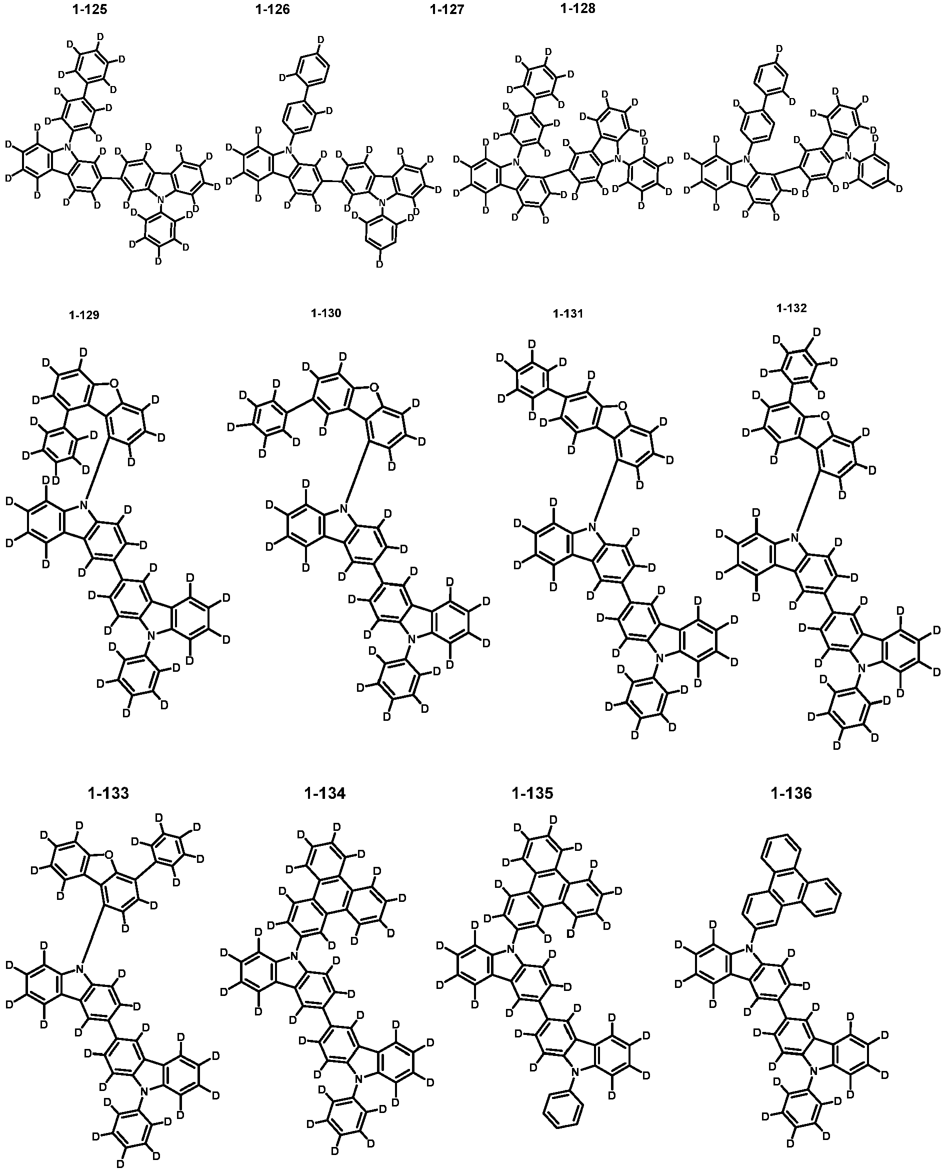

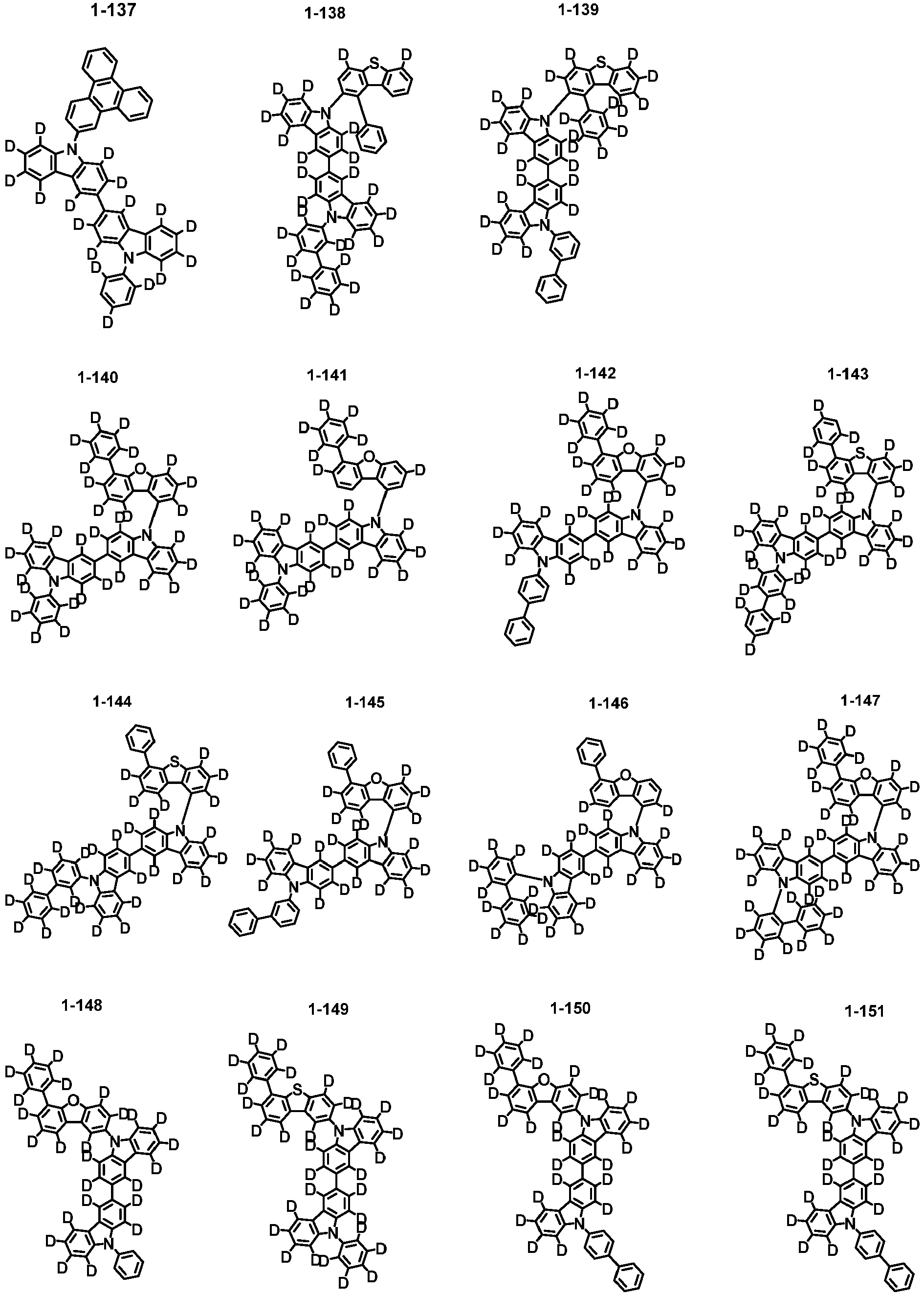

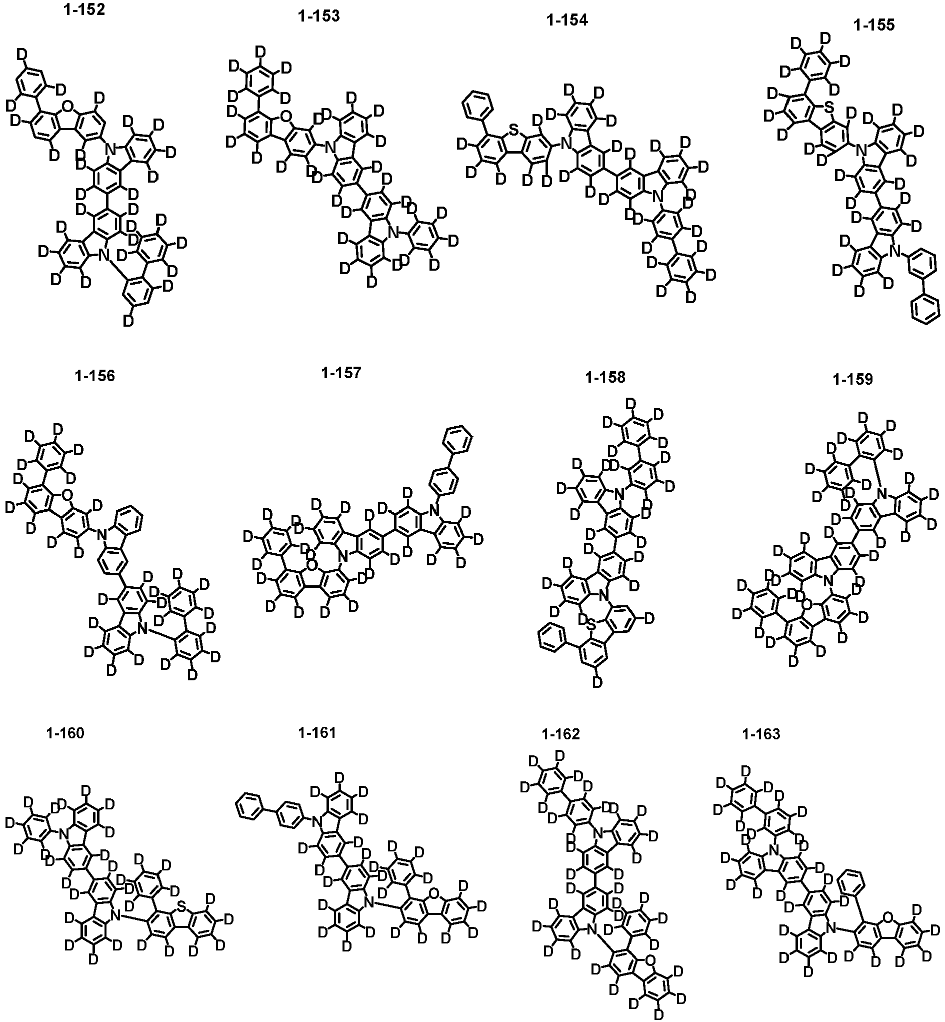

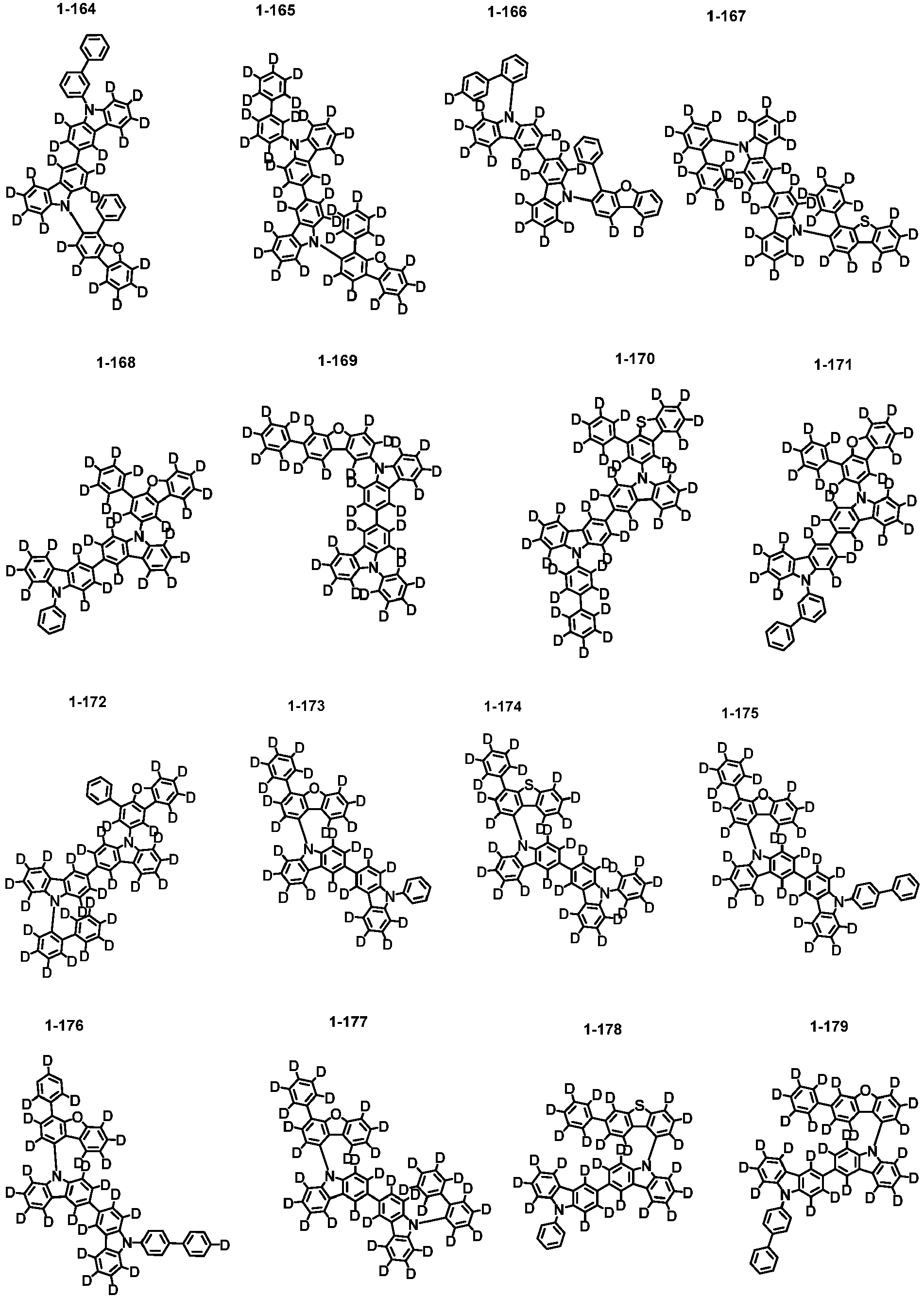

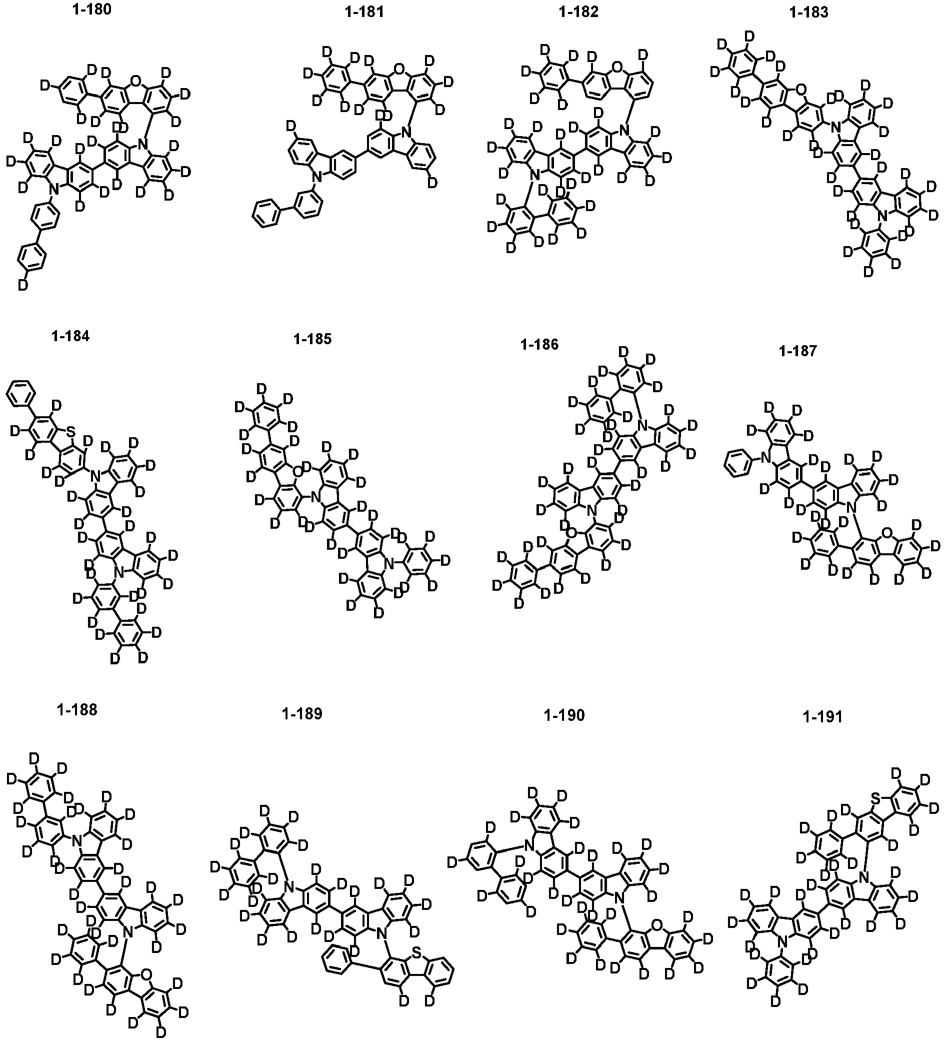

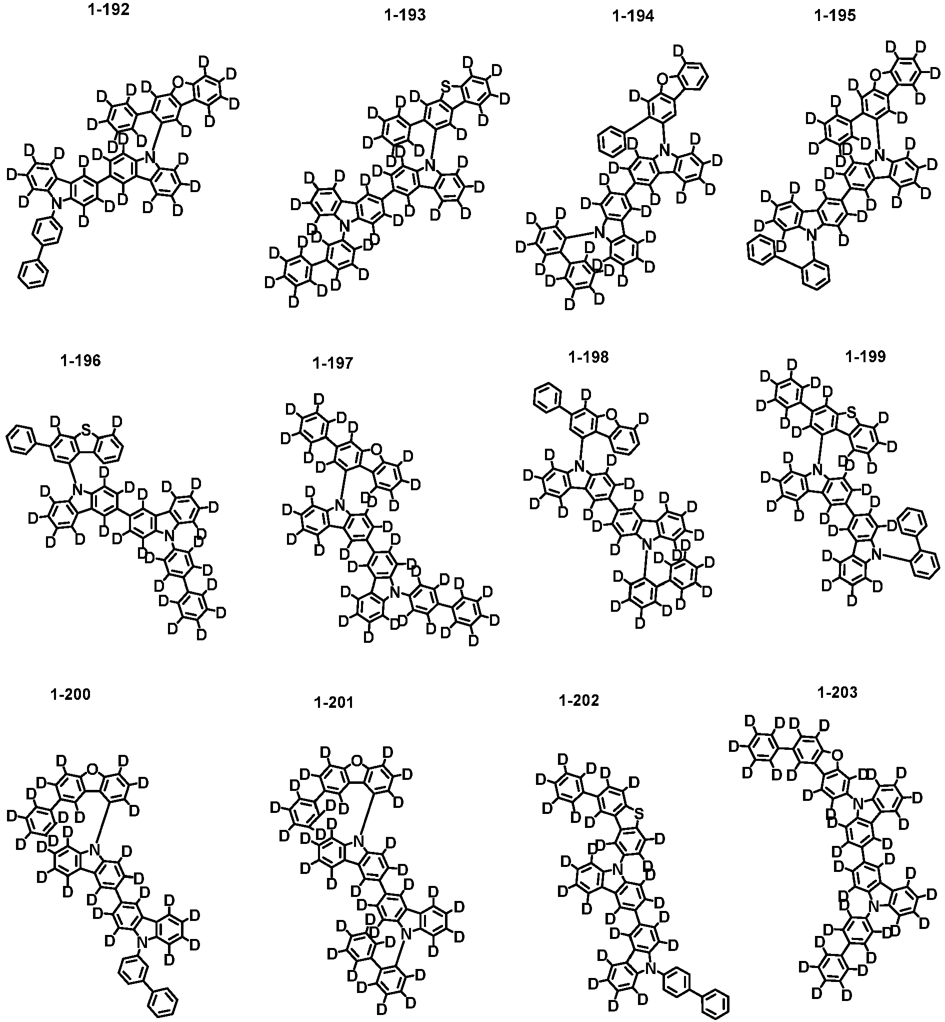

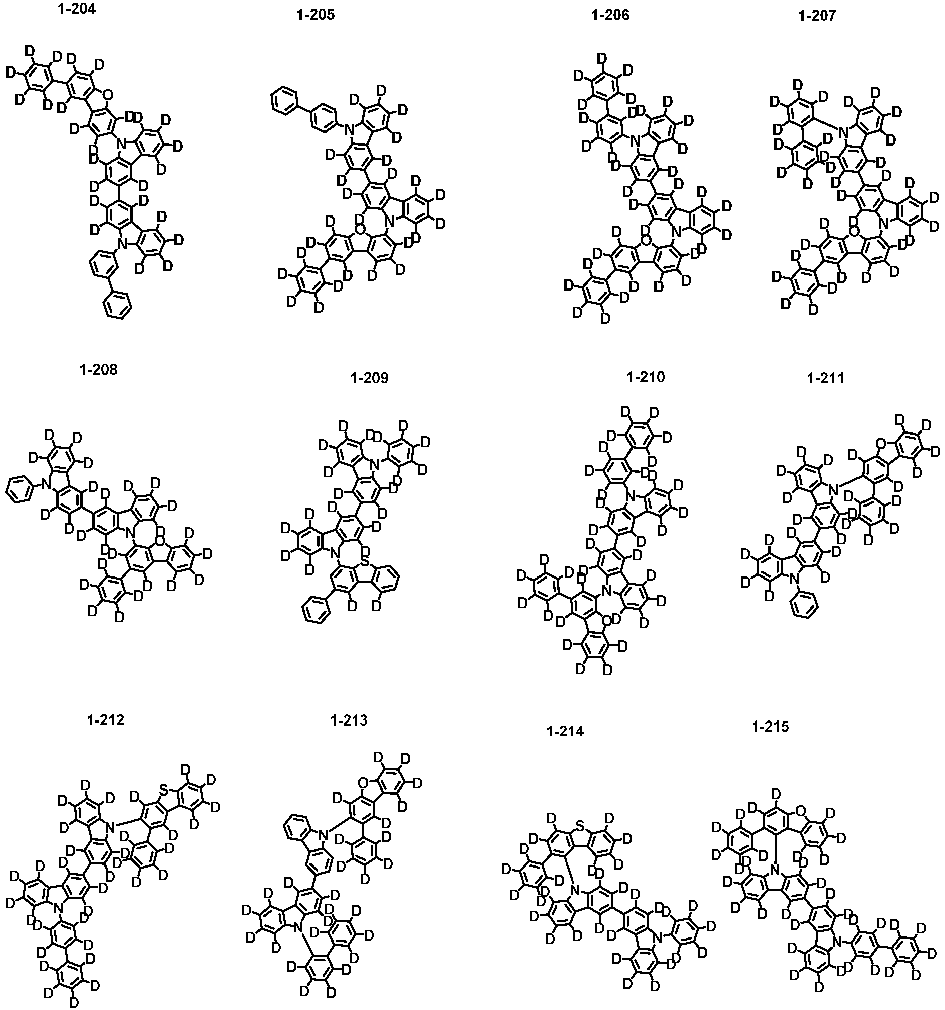

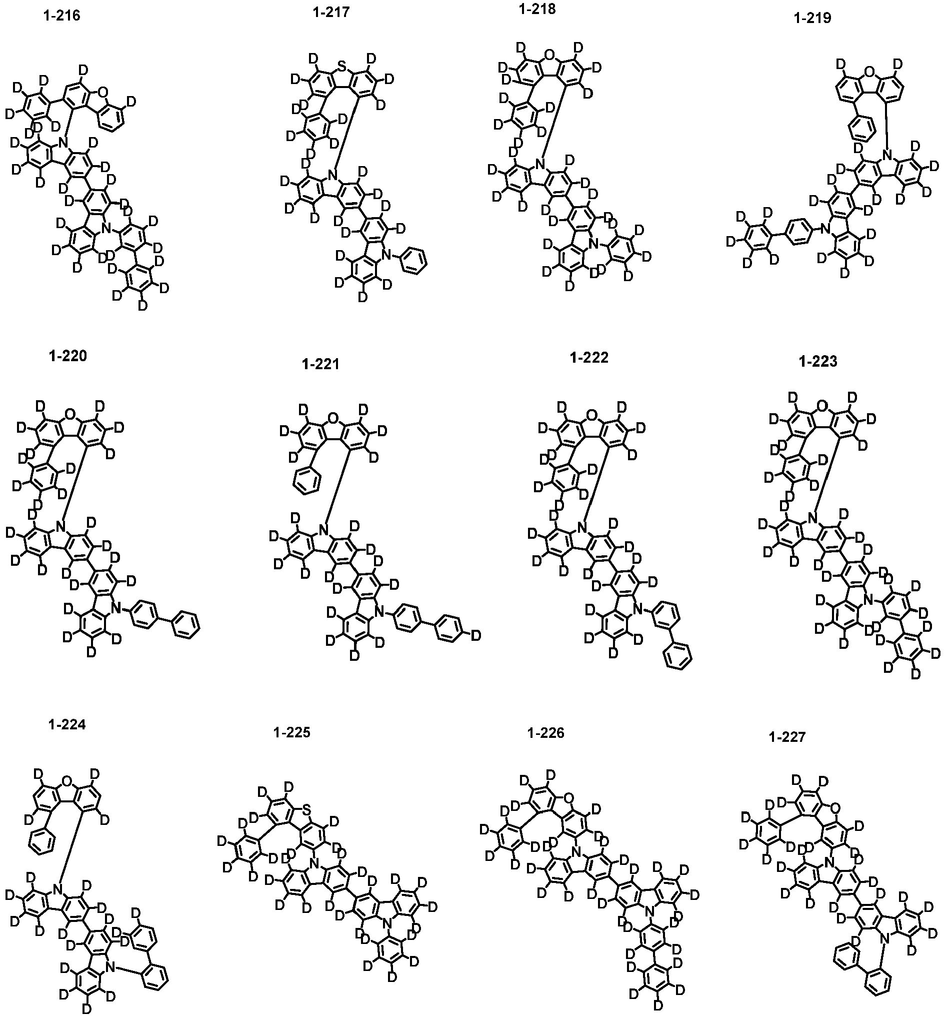

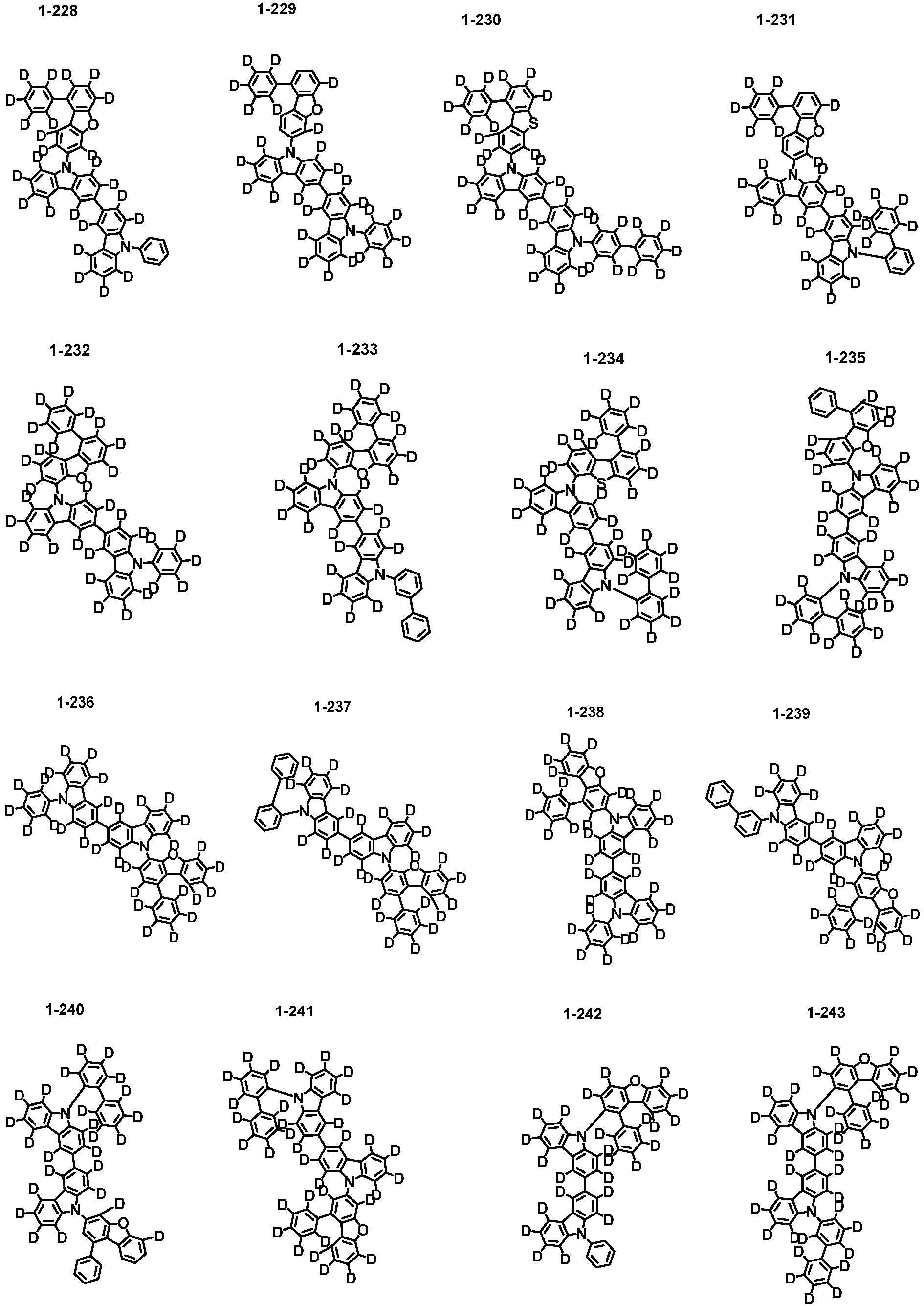

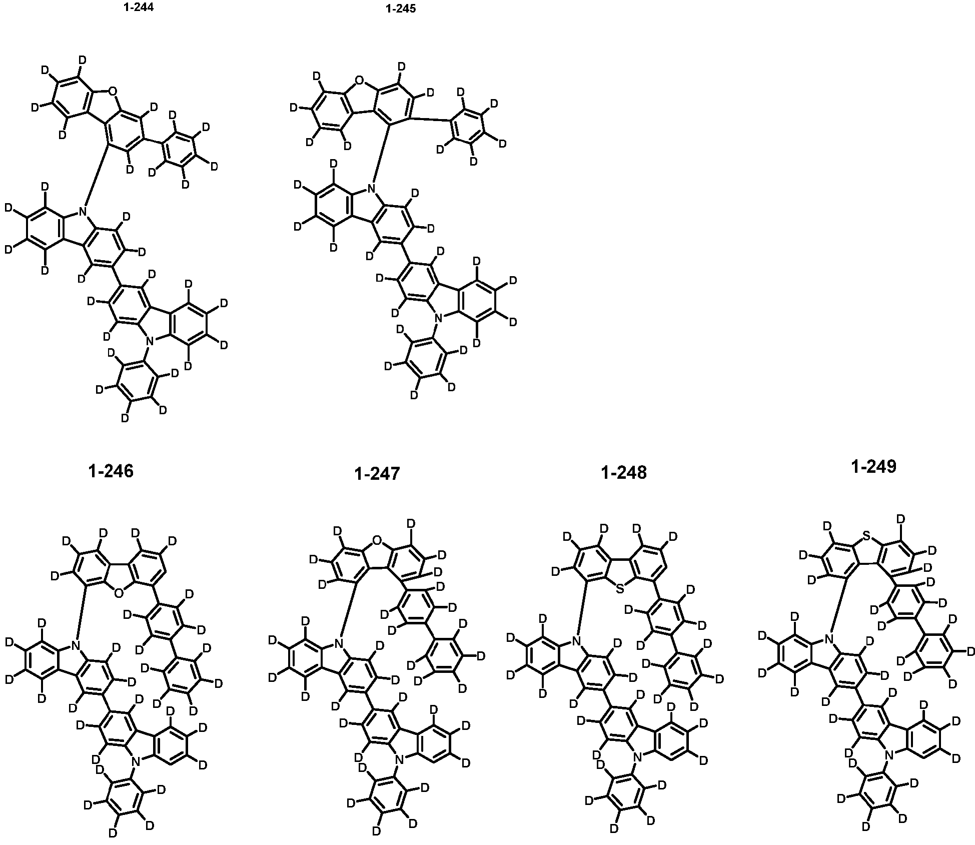

6. An organic optoelectronic device, comprising
an anode and a cathode facing each other, and
at least one organic layer between the anode and the cathode,
wherein the organic layer includes the compound for an organic optoelectronic device of any one of claim 1 to claim 5.

7. The organic optoelectronic device of claim 6, wherein
the organic layer includes a light emitting layer, and
the light emitting layer includes the compound for an organic optoelectronic device.

8. A display device comprising the organic optoelectronic device of claim 6.
